# EUROPEAN PATENT APPLICATION

(11) **EP 0 801 071 A1**
(43) Date of publication of application: **15.10.1997**
(21) Application number: 95941894.8
(22) Date of filing: 26.12.1995
(51) Int. Cl.: C07H 15/04, C07H 15/18, A61K 31/70

(54) **LEWIS X DERIVATIVE AND PROCESS FOR PRODUCING THE SAME**

(30) Priority: 28.12.1994 JP 340346/94; 17.02.1995 JP 87769/95; 26.05.1995 JP 152303/95
(71) Applicant: SUMITOMO PHARMACEUTICALS COMPANY, LIMITED, Osaka-shi, Osaka-fu 541 (JP)
(72) Inventor: HAYASHI, Masaji, Hyogo 651-13 (JP); MIYAUCHI, Hiroshi, Osaka 597 (JP); TANAKA, Masashi, Osaka 593 (JP); ITOH, Masanori, Kanagawa 228 (JP)
(74) Representative: VOSSIUS & PARTNER
(86) International application number: JP9502690
(87) International publication number: WO9620204

(57) **Abstract**

A novel oligosaccharide derivative having a cell adhesion inhibitory activity and represented by structural formula (I).

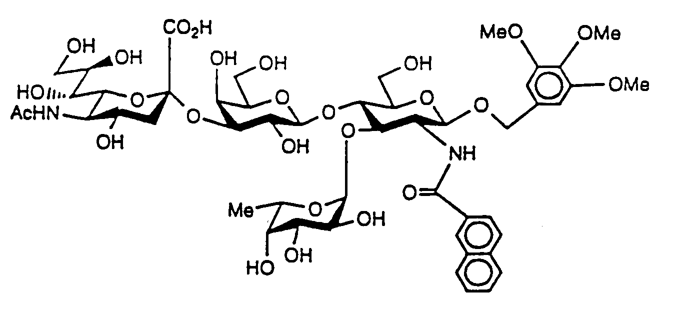

## Description

### FIELD OF THIS INVENTION

This invention relates to a derivative of Lewis X and sialyl Lewis X oligosaccharies known as a causative agent of inflammation, ischemia reperfusion injury, autoimmune disease and cancer metastasis, and processes for preparing the same. Such derivative is useful as a therapeutic agent for treating and improving the state of these diseases.

### BACKGROUND OF THIS INVENTION

It has been reported that E-selectin, a neutrophil adhesion molecule expressed in vascular endothelial cells, P-selectin, a neutrophil adhesion molecule expressed in vascular endothelial cells and platelets, and L-selectin, a homing receptor of lymphocyte, can recognize the Lewis X and sialyl Lewis X oligosaccharide structure as a ligand thereof(Shigeaki Morooka, Igaku no Ayumi, 169: 108 (1994)). It is expected that a material which inhibits the adhesion may be useful as anti-inflammatory drugs, because the onset of various inflammatory diseases starts from interactions mediated by binding of selectins with their ligands (M. P. Bevilacqua, et al., Thrombosis Haemostasis, 70: 152 (1993)). Accordingly, the oligosaccharide derivatives are prospectively useful for treating selectin-mediated diseases, and their therapeutic approaches are on trial. It is reported that in in vivo pathological models lung injuries caused by IgG immunocomplex (M. S. Mulligan, et al., J. Exp. Med., 178: 623 (1993)) or cobra venom factor (M. S. Mulligan, et. al., Nature, 364: 149 (1993)) and reperfusion injury after cardiac ischemia (D. Lefer, et al., J. Clin. Invest., 93: 1140 (1994)) are improved with sialyl Lewis X derivatives. Therefore, it is important to study the structure-activity relationships by efficiently synthesizing the derivatives and to find a derivative having superior activities for the purpose of providing medicals for various diseases.

### PROBLEMS TO BE SOLVED BY THIS INVENTION

Because of numerous steps necessary for synthesizing such derivatives, almost none of extensive study on the structure-activity relationship has been reported. It is desirous to have achievements of such extensive studies to find superior derivatives. The object of this invention is to effectively synthesize Lewis X and sialyl Lewis X derivatives having various O-glycoside and N-substituent, to extensively study their structure-activity relationships, and to provide derivatives possessing superior activities.

### MEANS FOR SOLVING THE PROBLEMS

The present inventors studied the structure-activity relationship to solve the problem, and found that the compounds of this invention strongly inhibit the adhesion of E-selectin to neutrophil. The present invention is based on this finding.

Thus, this invention firstly relates to Lewis X derivatives of the formula: 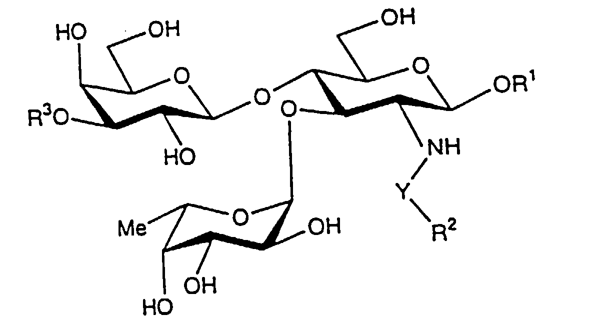 [wherein R¹ is C₁-C₁₈ alkyl, aryl, or aryl C₁-C₁₂ alkyl having at least one of substituents X defined below.
When R¹ has two or more substituents X, the substituents X may be the same or different each other.
Substituent X is a substituent selected from the group consisting of halogen, trifluoromethyl, hydroxy, C₁-C₁₈ alkoxy, aryloxy, aryl C₁-C₆ alkyloxy, amino, aryl C₁-C₆ alkylamino, mono(C₁-C₁₈ alkyl)amino, di(C₁-C₁₈ alkyl)amino, (C₁-C₁₈ alkyl)(aryl C₁-C₆ alkyl)amino, C₁-C₁₈ alkanoylamino, aroylamino, mono(C₁-C₁₈ alkyl)carbamoyl, di(C₁-C₁₈ alkyl)carbamoyl, aryl C₁-C₆ alkylcarbamoyl, (C₁-C₁₈ alkyl)(aryl C₁-C₆ alkyl)carbamoyl, arylcarbamoyl, C₁-C₁₈ alkanoyl, aroyl, C₁-C₁₈ alkylthio, arylthio, C₁-C₁₈ alkylsulfonyl, arylsulfonyl, cyano, and nitro.
The substituent X as defined above may be further substituted once or twice by the substituent as defined above on its alkyl chain or aryl ring;
Y is C(O), SO₂, C(O)NH, C(O)O or C(O)S;
R² is aryl, substituted aryl, or aryl C₁-C₆ alkyl; and
R³ is hydrogen or a group of the formula: 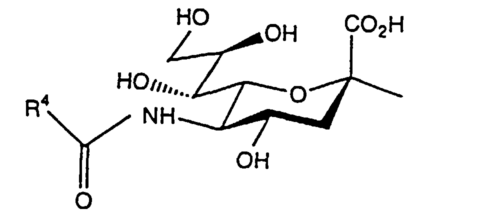 [wherein R⁴ is methyl or hydroxymethyl]], or a salt thereof.

Secondly, the present invention relates to a Lewis X derivative of the formula: 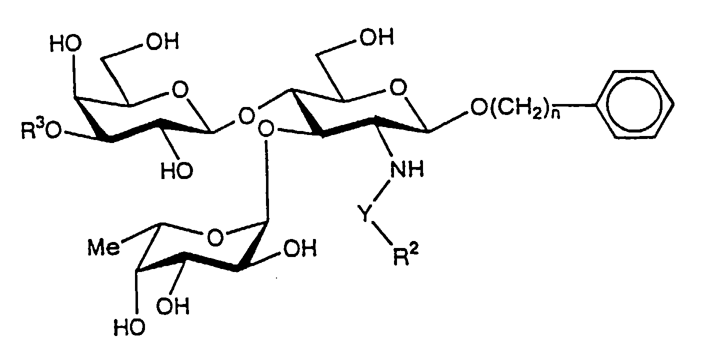 [wherein Y, R² and R³ are as defined above, and n is an integer of 2 to 6] or a salt thereof.

Thirdly, it relates to a compound of the formula: 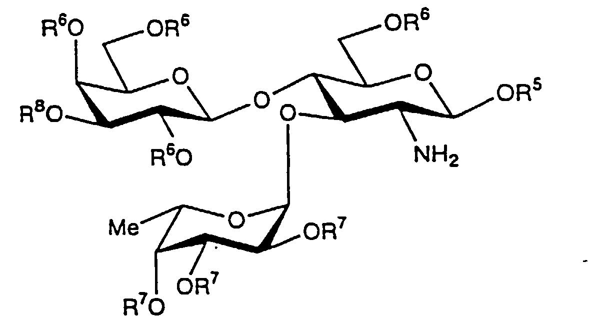 [wherein R⁵ is 2-tri(C₁-C₄ alkyl/phenyl)silylethyl;
R⁶ and R⁷ are each hydrogen, C₁-C₆ alkanoyl or aroyl; and
R⁸ is hydrogen, C₁-C₆ alkanoyl, aroyl, or a group of the formula: 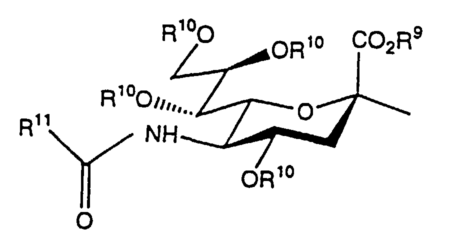 [wherein R⁹ is hydrogen or C₁-C₆ alkyl;
R¹⁰ is hydrogen, C₁-C₆ alkanoyl or aroyl; and
R¹¹ is methyl, hydroxymethyl, C₁-C₆ alkanoyloxymethyl, or aroyloxymethyl]] or salts thereof useful as an intermediate for synthesizing Lewis X derivatives.

Fourthly, it relates to a compound of the formula: 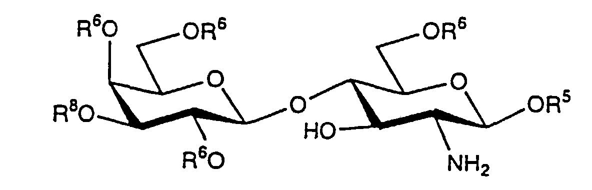 [wherein R⁵, R⁶ and R⁸ are as defined above]
or salts thereof useful as an intermediate for synthesizing Lewis X derivatives.

Fifthly, it relates to a process for preparing a Lewis X derivative which comprises appropriately modifying the amino group of the compound of the formula: 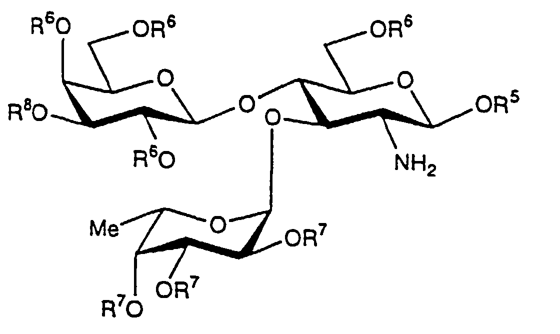 [wherein R⁵, R⁶, R⁷ and R⁸ are as defined above] to give a compound of the formula: 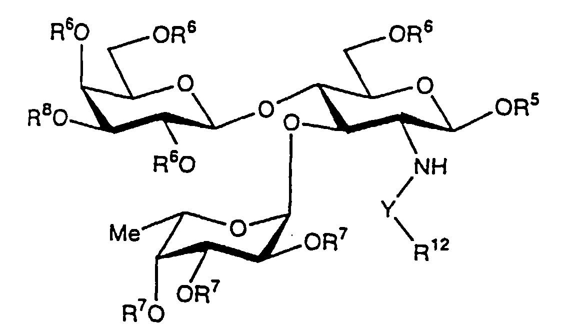 [wherein R⁵, R⁶, R⁷ and R⁸ are as defined above;
Y is C(O), SO₂, C(O)NH, C(O)O or C(O)S; and
R¹² is C₁-C₆ alkyl, aryl, substituted aryl or aryl C₁-C₆ alkyl], then subjecting the resultant compound to O-acylation (when R⁹ is hydrogen, together with esterification) (if none of R⁶, R⁷ and R⁸ is hydrogen and R⁸ does not have an unprotected hydroxy, this O-acylation is unnecessary) to give a compound of the formula: 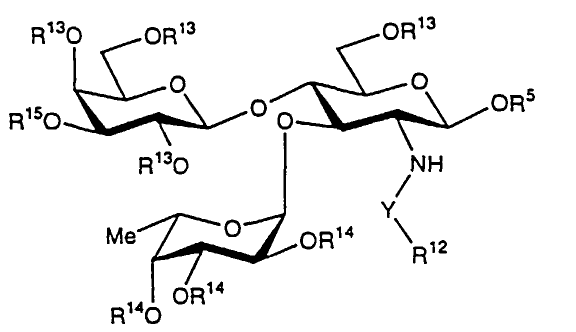 [wherein R⁵, Y and R¹² are as defined above;
R¹³ and R¹⁴ are C₁-C₆ alkanoyl or aroyl; and
R¹⁵ is C₁-C₆ alkanoyl, aroyl or a group of the formula: 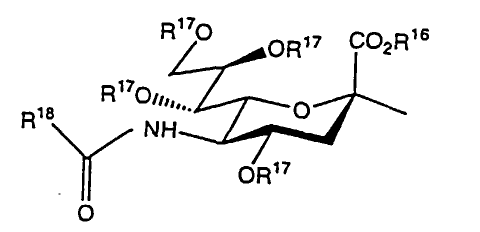 [wherein R¹⁶ is C₁-C₆ alkyl;
R¹⁷ is C₁-C₆ alkanoyl or aroyl; and
R¹⁸ is methyl, C₁-C₆ alkanoyloxymethyl or aroyloxymethyl]], then converting 2-tri(C₁-C₄ alkyl/phenyl) silylethyloxy at the reducing terminal to an appropriate leaving group to give a compound of the formula: 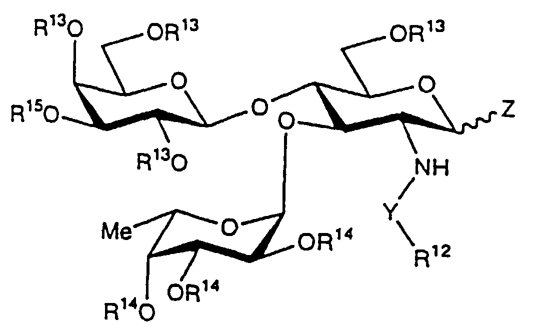 [wherein R¹³, R¹⁵, Y, R¹⁴ and R¹² are as defined above; and
Z is a leaving group], then glycosylating the resultant compound with a compound of the formula:

   R¹⁹OH

   [wherein R¹⁹ is unsubstituted C₁-C₁₈ alkyl, aryl or aryl C₁-C₁₂ alkyl, or substituted C₁-C₁₈ alkyl, aryl or aryl C₁-C₁₂ alkyl] to give a compound of the formula: 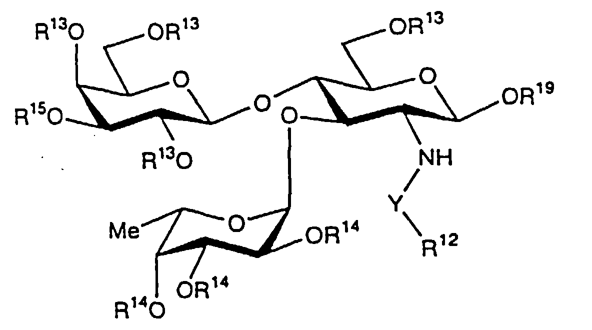 [wherein R¹³, R¹⁵, Y, R¹⁴, R¹² and R¹⁹ are as defined above], and finally hydrolyzing the resultant compound to give a Lewis X derivative of the formula: 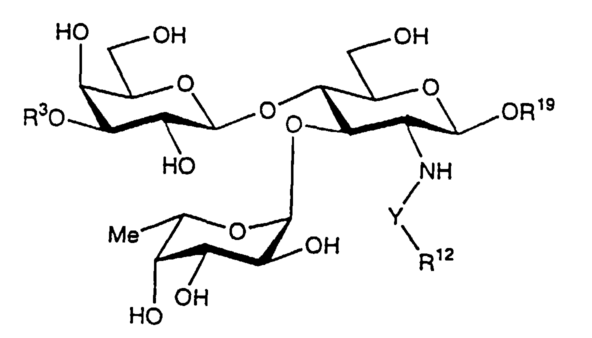 [wherein R³, Y, R¹² and R¹⁹ are as defined above].

Sixthly, it relates to a process for preparing a Lewis X derivative which comprises appropriately protecting the N-atom of a compound of the formula: 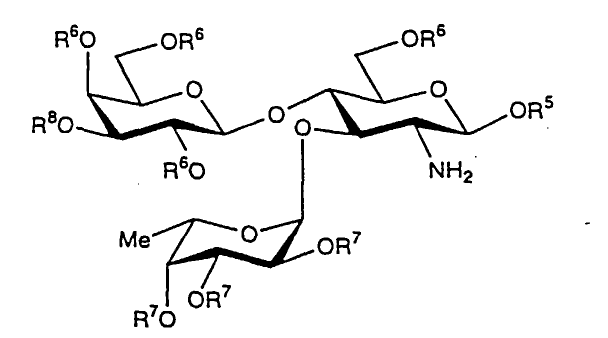 [wherein R⁵, R⁶, R⁷ and R⁸ are as defined above] to give a compound of the formula: 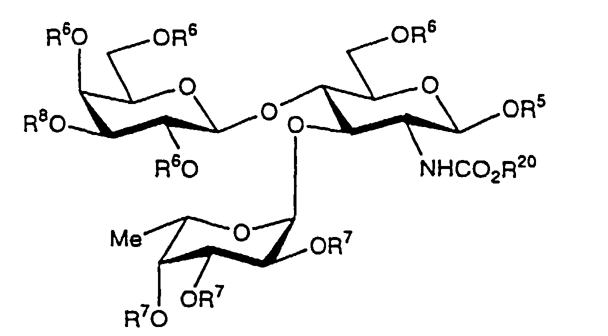 [wherein R⁵, R⁶, R⁷ and R⁸ are as defined above, and R²⁰ is allyl, t-butyl or benzyl], then subjecting the resultant compound to O-acylation (when R⁹ is hydrogen, together with esterification) (if none of R⁶, R⁷ and R⁸ is hydrogen and R⁸ does not have an unprotected hydroxy, this O-acylation is unnecessary) to give a compound of the formula: 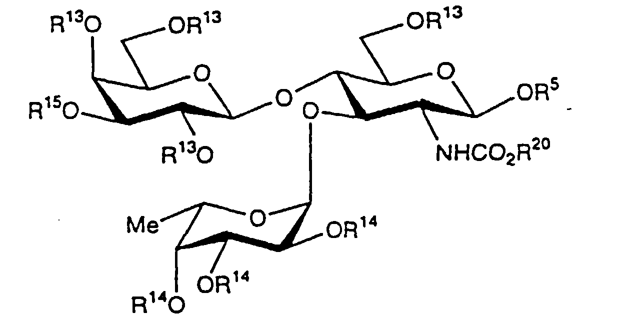 [wherein R⁵, R¹³, R¹⁴, R¹⁵ and R²⁰ are as defined above], then converting its 2-tri(C₁-C₄ alkyl/phenyl) silylethyloxy on the reducing terminal to an appropriate leaving group to give a compound of the formula: 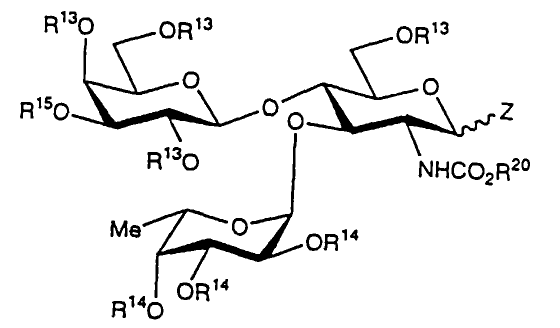 [wherein R¹³, R¹⁵, R¹⁴, R²⁰ and Z are as defined above], then glycosylating the resultant compound with a compound of the formula:

R¹⁹OH

[wherein R¹⁹ is as defined above] to give a compound of the formula: 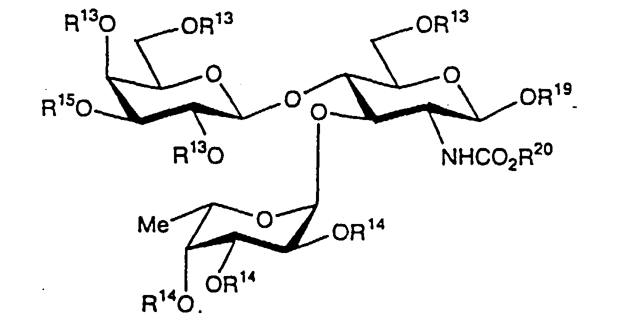 [wherein R¹³, R¹⁵, R¹⁴, R¹⁹ and R²⁰ are as defined above], then removing the protecting group on N-atom to give a compound of the formula: 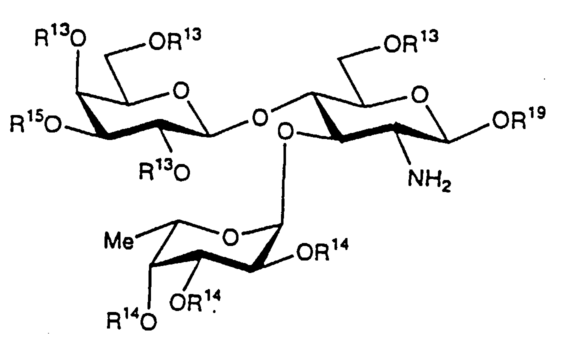 [wherein R¹³, R¹⁵, R¹⁴ and R¹⁹ are as defined above], then appropriately modifying the amino group of this compound to give a compound of the formula: 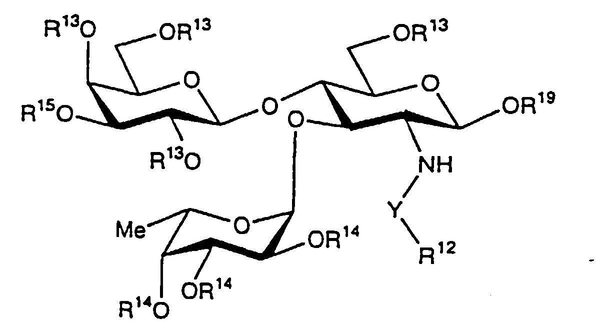 [wherein R¹³, R¹⁵, R¹⁴, R¹², Y and R¹⁹ are as defined above], and finally hydrolyzing the resultant compound to give a Lewis X derivative of the formula: 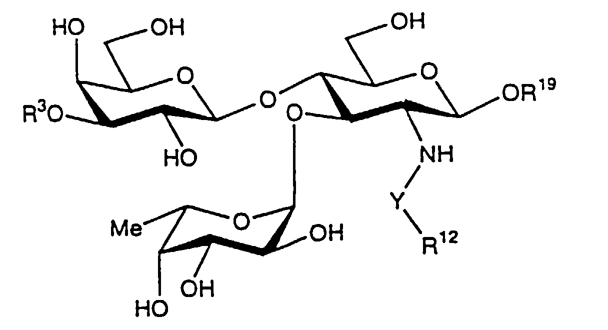 [wherein R¹², R¹⁹, Y and R³ are as defined above].

Seventhly, it relates to a process for preparing a compound which comprises appropriately protecting the N-atom of a compound of the formula: 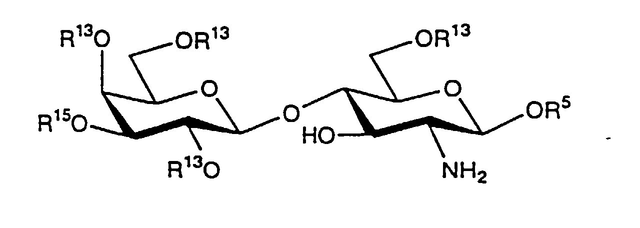 [wherein R⁵, R¹³ and R¹⁵ are as defined above] to give a compound of the formula: 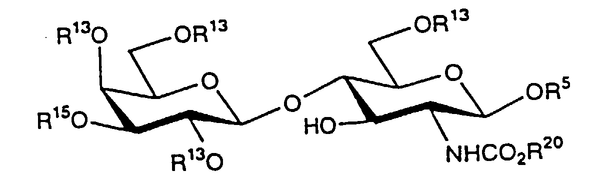 [wherein R⁵, R¹³, R¹⁵ and R²⁰ are as defined above], then subjecting the resultant compound to glycosylation with an L-fucopyranosyl derivative of the formula: 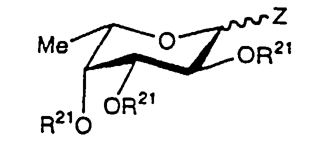 [wherein Z is as defined above; and
R²¹ is C₁-C₆ alkanoyl, aroyl, benzyl or substituted benzyl] to give a compound of the formula: 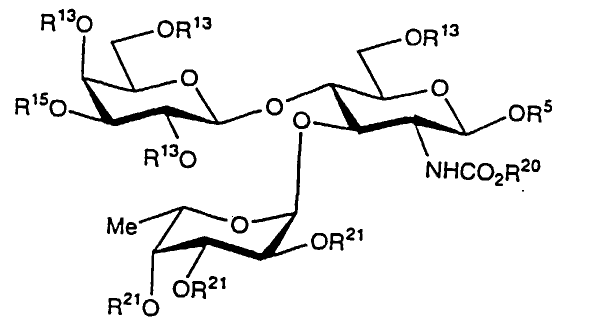 [wherein R⁵, R¹³, R²⁰, R¹⁵ and R²¹ are as defined above], then subjecting the resultant compound, if necessary (if R²¹ is benzyl or substituted benzyl, deprotection is indispensable), to deprotection of a protective group to give a compound of the formula: 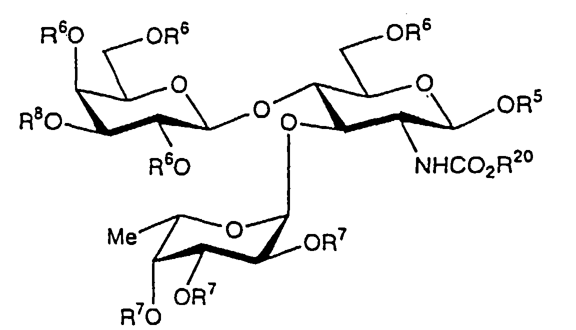 [wherein R⁵, R⁶, R⁷, R²⁰ and R⁸ are as defined above], and finally subjecting the resultant compound to subsequent N-deprotection to give a compound of the formula: 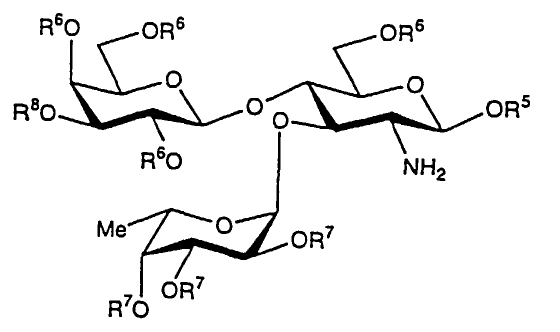 [wherein R⁵, R⁶, R⁷ and R⁸ are as defined above].

Eighthly, it relates to a process for preparing a compound which comprises reacting a compound of the formula: 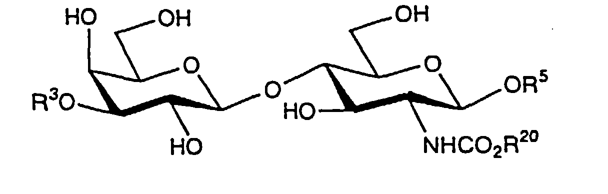 [wherein R⁵, R³ and R²⁰ are as defined above] with GDP-fucose using a fucosyl transferase to give a compound of the formula: 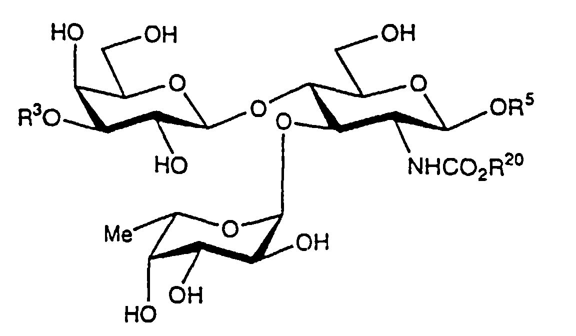 [wherein R⁵, R³, and R²⁰ are as defined above], then subjecting the resultant compound, if necessary, to O-acylation and carboxy- esterification and then to subsequent N-deprotection to give a compound of the formula: 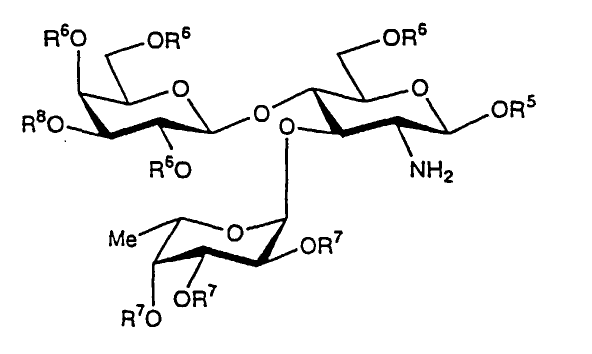 [wherein R⁵, R⁶, R⁷ and R⁸ are as defined above].

Ninthly, it relates to a process for preparing a compound which comprises reacting a glucosamine derivative of the formula: 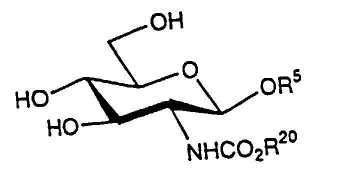 [wherein R⁵ and R²⁰ are as defined above] with UDP-galactose using a galactosyl transferase and then, if necessary, with CMP-N-acetylneuraminic acid using a sialyl transferase, to give a compound of the formula: 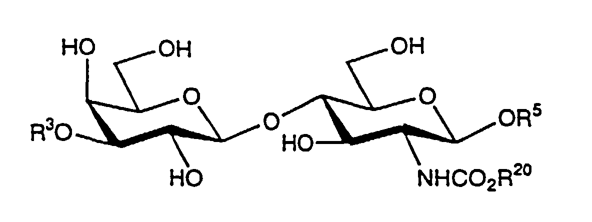 [wherein R⁵, R²⁰ and R³ are as defined above], then subjecting the resultant compound, if necessary, to O-acylation (if necessary, together with carboxy-esterification) to give a compound of the formula: 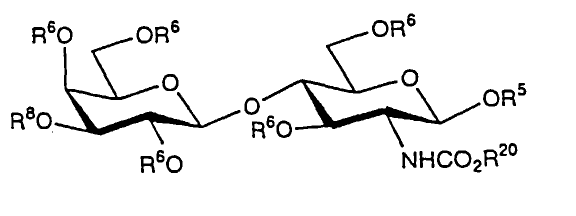 [wherein R⁵, R⁶, R²⁰ and R⁸ are as defined above], then removing the protecting group on N-atom to give a compound of the formula: 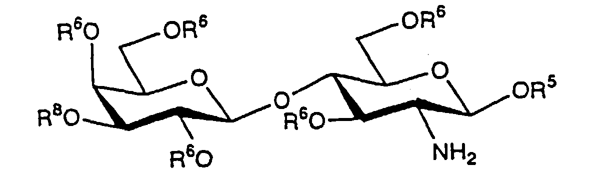 [wherein R⁵, R⁶ and R⁸ are as defined above], finally subjecting the resultant compound to a regioselective deacylation to give a compound of the formula: 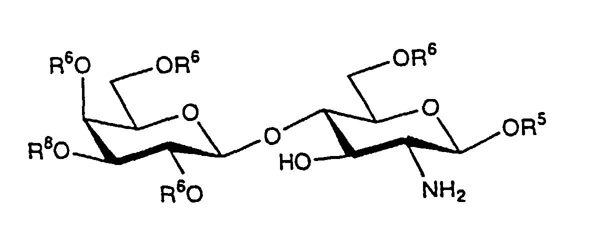 [wherein R⁵, R⁶ and R⁸ are as defined above].

The substituents in the present invention are explained below.

A substituent in R¹⁹ is, for example, a substituent X as defined above.

In the formulae, the C₁-C₁₈ alkyl group or C₁-C₁₈ alkyl in R¹, R¹⁹ and X contains 1 to 18 carbon atoms and includes straight or branched alkyl, cycloalkyl, (cycloalkyl)alkyl or (cycloalkyl)cycloalkyl,such as methyl, ethyl, propyl, isopropyl, butyl, isobutyl, 2-butyl, t-butyl, pentyl, 3-pentyl, isopentyl, neopentyl, hexyl, heptyl, 4-heptyl, octyl, nonyl, 5-nonyl, decyl, undecyl, 6-undecyl, dodecyl, tridecyl, 7-tridecyl, tetradecyl, pentadecyl, 8-pentadecyl, hexadecyl, heptadecyl, 9-heptadecyl, octadecyl, cyclopropyl, cyclobutyl, cyclopentyl, cyclohexyl, cycloheptyl, cyclooctyl, cyclopentylmethyl, cyclohexylmethyl, (4-cyclohexyl)cyclohexyl, and the like groups.

The aryl C₁-C₁₂ alkyl group in R¹ and R¹⁹ includes, for example, phenyl C₁-C₁₂ alkyl, i.e., a straight or branched alkyl group of 1 to 12 carbon atoms bearing a phenyl group at the terminal, such as benzyl, phenethyl, phenylpropyl, phenylbutyl, phenylpentyl, phenylhexyl, phenylheptyl, phenyloctyl, phenylnonyl, phenyldecyl, phenylundecyl, phenyldodecyl, and the like groups.

The halogen atom in X means a fluorine, chlorine, bromine, or iodine atom.

The C₁-C₁₈ alkoxy group in X is a straight, branched or cyclic alkoxy group containing 1 to 18 carbon atoms, including methoxy, ethoxy, propoxy, isopropoxy, butoxy, pentyloxy, cyclopentyloxy, hexyloxy, cyclohexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, and the like groups.

The aryl group or aryl in R¹, R², R¹², R¹⁹ and X is a group forming a cycle from either a hydrocarbon group, hydrocarbon group containing an oxygen atom, hydrocarbon group containing a sulfur atom, hydrocarbon group containing a nitrogen atom, or hydrocarbon group containing two nitrogen atoms to give an aromatic ring of five-membered monocyclic ring, six-membered monocyclic ring, fused polycyclic ring of condensed six-membered ring and five-membered ring, or a fused polycyclic ring of condensed six-membered rings. It may, for example, be a monocyclic aromatic hydrocarbon group such as phenyl, or the like; condensed polycyclic aromatic hydrocarbon group, such as naphthyl, anthracenyl (anthryl), phenanthrenyl, or the like; aromatic heterocyclic ring group containing oxygen, sulfur, or 1 to 2 nitrogen atoms, such as furyl, thienyl, pyridyl, pyrazinyl, benzofuranyl (benzo[b]furanyl), isobenzofuranyl (benzo[c]furanyl), benzothienyl (benzo[b]thienyl), isobenzothienyl (benzo[c]thienyl), pyrimidinyl, pyridazinyl, quinolinyl, isoquinolinyl, quinoxalinyl, naphthylidinyl, phthalazinyl, quinazolinyl, or the like group. The position of the valence in forming a group may optionally be selected from every available positions. Preferable aryl for R¹ is phenyl.

The C₁-C₁₈ alkanoyl group or C₁-C₁₈ alkanoyl in X is a straight or branched alkylcarbonyl or cycloalkanecarbonyl group containing 1 to 18 carbon atoms. It may, for example, be a formyl, acetyl, propionyl, butyryl, isobutyryl, valeryl, isovaleryl, pivaloyl, pentanoyl, isopentanoyl, neopentanoyl, hexanoyl, heptanoyl, octanoyl, nonyl, decanoyl, undecanoyl, dodecanoyl, tridecanoyl, tetradecanoyl, pentadecanoyl, hexadecanoyl, heptadecanoyl, octadecanoyl, cyclopentanecarbonyl, cyclohexanecarbonyl, or the like group.

The aroyl group in X is the same as an arylcarbonyl group.

The C₁-C₁₈ alkanoylamino group in X is the same as C₁-C₁₈ alkylcarboxamido and is an amino group substituted by a straight or branched alkanoyl or cycloalkanecarbonyl group containing 1 to 18 carbonatoms, such as acetylamino, propionylamino, butyrylamino, valerylamino, pentanoylamino, cyclopentanecarboxamido, hexanoylamino, cyclohexanecarboxamido, heptanoylamino, octanoylamino, nonanoylamino, decanoylamino, undecanoylamino, dodecanoylamino, tridecanoylamino, tetradecanoylamino, pentadecanoylamino, hexadecanoylamino, heptadecanoylamino, octadecanoylamino, or the like group.

The mono(C₁-C₁₈ alkyl)carbamoyl group in X is the same as mono(C₁-C₁₈ alkyl)aminocarbonyl, and is a carbonyl group substituted by a straight or branched alkylamino or cycloalkylamino containing 1 to 18 carbon atoms, such as methylcarbamoyl, ethylcarbamoyl, propylcarbamoyl, butylcarbamoyl, pentylcarbamoyl, cyclopentylcarbamoyl, hexylcarbamoyl, cyclohexylcarbamoyl, heptylcarbamoyl, octylcarbamoyl, nonylcarbamoyl, decylcarbamoyl, undecylcarbamoyl, dodecylcarbamoyl, tridecylcarbamoyl, tetradecylcarbamoyl, pentadecylcarbamoyl, hexadecylcarbamoyl, heptadecylcarbamoyl, octadecylcarbamoyl, or the like group.

The di(C₁-C₁₈ alkyl)carbamoyl group in X is the same as di(C₁-C₁₈ alkyl)aminocarbonyl group, such as dimethylcarbamoyl, diethylcarbamoyl, or the like group.

Substituent X which is substituted on its alkyl chain or aryl ring further once or twice by the substituent as defined above is also included within the substituent X. It may, for example, be a 2-(2-ethoxyethyl)oxy (i.e., 3-oxapentyloxy), 3,6-dioxaoctyloxy, 3,6,9-trioxaundecyloxy, (3,4,5- trimethoxybenzyl)oxy, (2-benzyloxyethyl)oxy, [2-(3,4,5-trimethoxybenzyl) oxyethyl]oxy, 7-phenyl-3,6-dioxaheptyloxy, (2-hydroxyethyl)oxy, [2-(2-hydroxyethyl)oxyethyl]oxy, (8-hydroxy-3,6-dioxaoctyloxy), 11-hydroxy-3,6,9-trioxaundecyloxy, or the like group.

The position of substitution on the alkyl chain or aryl ring of the substituent X noted above may be on every carbon atoms, excluding the carbon atom directly attached to the oxygen on the reducing terminus of saccharide.

Single or plural (2 to 5) number of such substituents X may be on the alkyl chain or aryl ring, and the substituents may be the same or different.

The aryl C₁-C₆ alkyl group or aryl C₁-C₆ alkyl in X, R² or R¹² is, e.g., phenyl C₁-C₆ alkyl, i.e., a straight or branched alkyl group containing 1 to 6 carbon atoms possessing a phenyl group at the terminal position, such as benzyl, phenethyl, phenylpropyl, phenylbutyl, phenylpentyl, phenylhexyl, or the like group.

The substituted aryl in R² and R¹² is an aryl group having one or more of one kind to several kinds of the following substituents on the aromatic ring. Such substituents may, for example, include halogen; nitro; trifluoromethyl; alkyl containing 1 to 18 carbon atoms, such as methyl, ethyl, propyl, isopropyl, butyl, isobutyl, t-butyl, pentyl, 3-pentyl, isopentyl, neopentyl, hexyl, heptyl, 4-heptyl, octyl, nonyl, 5-nonyl, decyl, undecyl, 6-undecyl, dodecyl, tridecyl, 7-tridecyl, tetradecyl, pentadecyl, 8-pentadecyl, hexadecyl, heptadecyl, 9-heptadecyl, octadecyl, cyclopropyl, cyclobutyl, cyclopentyl, cyclohexyl, cycloheptyl, cyclooctyl, (4-cyclohexyl)cyclohexyl, and the like; phenyl; alkoxy containing 1 to 18 carbon atoms, such as methoxy, ethoxy, propoxy, isopropoxy, butoxy, pentyloxy, cyclopentyloxy, hexyloxy, cyclohexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, and the like; phenoxy, benzyloxy; (substituted benzyl)oxy; amino; benzylamino; (substituted benzyl)amino; monoalkylamino containing 1 to 18 carbon atoms; dialkylamino having alkyl moieties each containing 1 to 18 carbon atoms; alkylbenzylamino having alkyl moiety containing 1 to 18 carbon atoms; alkanoylamino(alkylcarboxamido) containing 1 to 18 carbon atoms, such as acetylamino, propionylamino, butyrylamino, valerylamino, pentanoylamino, cyclopentanecarboxamido, hexanoylamino, cyclohexanecarboxamido, heptanoylamino, octanoylamino, nonanoylamino, decanoylamino, undecanoylamino, dodecanoylamino, tridecanoylamino, tetradecanoylamino, pentadecanoylamino, hexadecanoylamino, heptadecanoylamino, octadecanoylamino, and the like; aroylamino containing 1 to 12 carbon atoms, such as benzoylamino, naphthoylamino, and the like; carboxyl; alkylcarbamoyl(alkylaminocarbonyl), the alkyl moiety of which contains 1 to 18 carbon atoms, such as methylcarbamoyl, ethylcarbamoyl, propylcarbamoyl, butylcarbamoyl, pentylcarbamoyl, cyclopentylcarbamoyl, hexylcarbamoyl, cyclohexylcarbamoyl, heptylcarbamoyl, octylcarbamoyl, nonylcarbamoyl, decylcarbamoyl, undecylcarbamoyl, dodecylcarbamoyl, tridecylcarbamoyl, tetradecylcarbamoyl, pentadecylcarbamoyl, hexadecylcarbamoyl, heptadecylcarbamoyl, octadecylcarbamoyl, and the like; arylcarbamoyl; alkylthio containing 1 to 18 carbon atoms; arylthio; alkylsulfonyl containing 1 to 18 carbon atoms; arylsulfonyl; cyano; nitro; and the like atom or group.

The C₁-C₆ alkyl group in R⁹, R¹² and R¹⁶ includes a straight or branched alkyl group containing 1 to 6 carbon atoms, such as methyl, ethyl, propyl, isopropyl, butyl, isobutyl, t-butyl, pentyl, isopentyl, neopentyl, hexyl, and the like groups.

The C₁-C₆ alkanoyl group or C₁-C₆ alkanoyl in R⁶, R⁷, R⁸, R¹⁰, R¹¹, R¹³, R¹⁴, R¹⁵, R¹⁷, R¹⁸ and R²¹ is a straight or branched alkylcarbonyl group containing 1 to 6 carbon atoms, and its alkyl moiety may optionally be substituted by one or more of halogen atoms. It includes, for example, formyl, acetyl, chloroacetyl, dichloroacetyl, trichloroacetyl, propionyl, butyryl, isobutyryl, valeryl, isovaleryl, pivaloyl, pentanoyl, isopentanoyl, neopentanoyl, and the like groups, among which especially preferred are acetyl, chloroacetyl, trichloroacetyl, and pivaloyl groups.

The aroyl group or aroyl in R⁶, R⁷, R⁸, R¹⁰, R¹¹, R¹³, R¹⁴, R¹⁵, R¹⁷, R¹⁸ and R²¹ is the same as arylcarbonyl, and its aryl moiety may optionally be a substituted aryl defined above.

The substituted benzyl group in R²¹ is a benzyl group bearing a substituent such as halo, nitro, alkoxy containing 1 to 6 carbon atoms, or the like on its phenyl ring, and specific examples are 4-bromobenzyl, 4-nitrobenzyl, 4-methoxybenzyl, or the like. Among them, 4-methoxybenzyl is preferable.

R⁵ in the formulas refers to a 2-tri(C₁-C₄ alkyl/phenyl)silylethyl group. The 2-tri(C₁-C₄ alkyl/phenyl)silylethyl group is a 2-silylethyl group substituted by three of the same or different C₁-C₄ alkyl or phenyl groups on the silicon atom, such as 2-trimethylsilylethyl, 2-triethylsilylethyl, 2-(triisopropylsilyl)ethyl, 2-(t-butyldimethylsilyl)ethyl, 2-triphenylsilylethyl, 2-(diphenylmethylsilyl)ethyl, 2-(t-butyldiphenylsilyl)ethyl, or the like group.

The leaving group represented by Z in the formulas includes, for example, halogen such as fluorine, chlorine, bromine, iodine, and the like; alkanoyloxy containing 1 to 6 carbon atoms, such as acetoxy, propoxy, and the like; aroyloxy such as benzoyloxy, and the like; straight or branched alkylthio containing 1 to 6 carbon atoms, such as methylthio, ethylthio, propylthio, isopropylthio, and the like; phenylthio; pyridylthio; phenylsulfinyl; phenylselenyl; imidate such as acetoimidate, trichloroacetimidate, N-methylacetoimidate, and the like; dialkylphosphoryl containing 1 to 6 carbon atoms in each alkyl moiety, such as dimethylphosphoryl, diethylphosphoryl, and the like; diphenylphosphoryl;and the like atom or group.

A salt of Lewis X derivative of this invention includes sodium, lithium, potassium, magnesium, calcium, and the like salts.

Compounds of the formula: 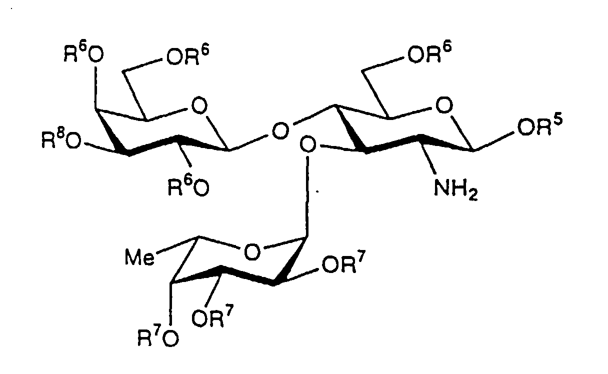 [wherein R⁵, R⁶, R⁷ and R⁸ are ss defined above] and compounds of the formula: 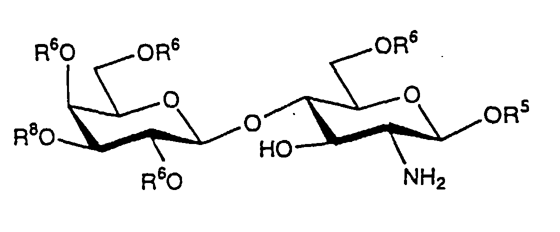 [wherein R⁵, R⁶ and R⁸ are as defined above], may be in the form of ammonium salts, for example, inorganic salt, such as hydrochloride, hydrobromide, hydroiodide, sulfate, nitrate, phosphate, and the like; and mono- or di-carboxylate, such as formate, acetate, oxalate, tartrate, mandelate, succinate, maleate, fumarate, and the like.

The present inventors found that the compounds of the formula: 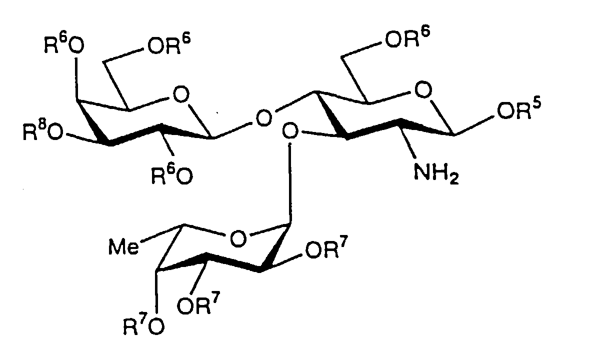 [wherein R⁵, R⁶, R⁷ and R⁸ are as defined above], i.e., 2-trisubstituted silylethyl β-D-galactopyranosyl-(1→4)-O-[α-L-fucopyranosyl-(1→3)-O]-(β-D-glucosaminopyranoside) derivatives, are useful compounds for preparing Lewis X and sialyl Lewis X derivatives variously modified at O-glycoside and N-substituent. The compounds were used for preparing the compounds of the present invention.

Thus, such Lewis X derivative (1) may be prepared from Compound (2) according to the methods as described below: 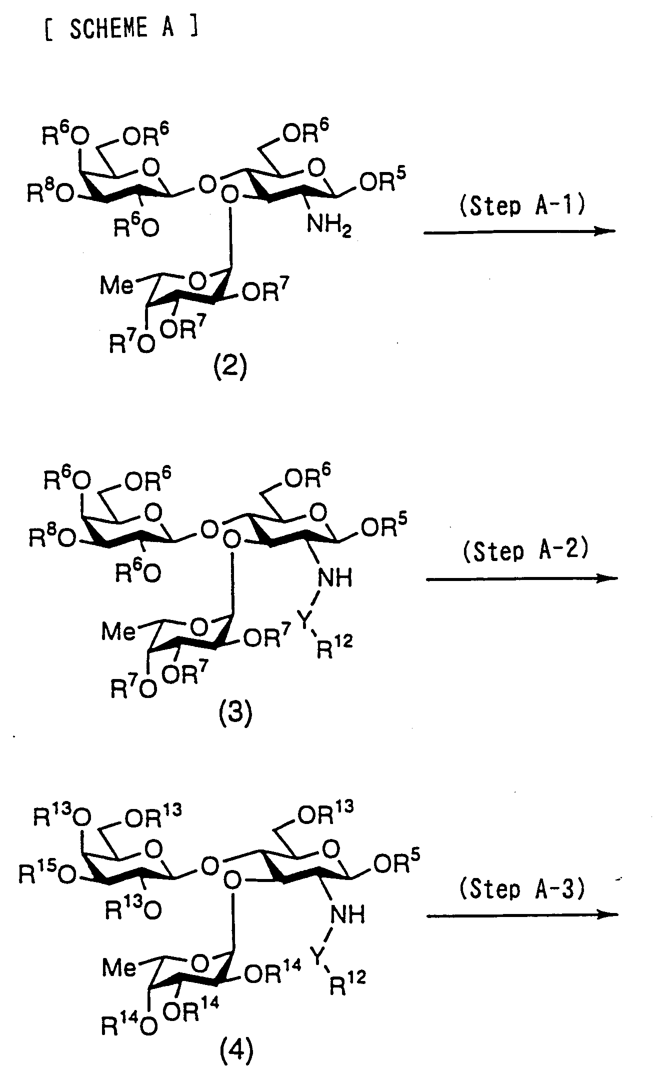 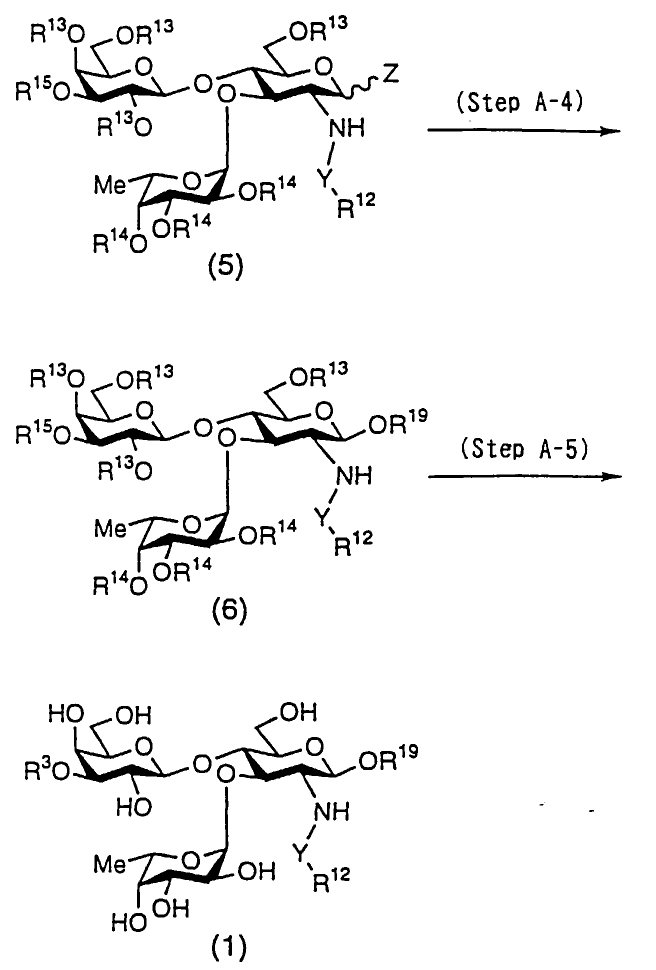 [wherein R⁵, R⁶, R⁷, R⁸, R¹², Y, R¹³, R¹⁴, R¹⁵, Z, R¹⁹ and R³ are as defined above]

### (Step A-1)

Compound (2) is treated with an electrophile under various conditions to give Compound (3).

The electrophiles for reactions in the presence of a base may be carboxyl halide, carboxylic anhydride, haloformate ester, pyrocarbonate, sulfonyl halide, sulfonic anhydride, or the like, having substituent R¹² previously defined. The halogen atom of the halides may be chlorine, bromine, iodine, or the like atom.

The base may be a carbonate, such as sodium carbonate, potassium carbonate, or the like; hydroxide, such as sodium hydroxide, potassium hydroxide, or the like; bicarbonate, such as sodium bicarbonate, potassium bicarbonate or the like; disodium hydrogenphosphate; dipotassium hydrogen phosphate; organic base, such as triethylamine, N,N-diisopropylethylamine, pyridine, dimethylaniline, 1,8-diazabicyclo[5.4.0] undecene (DBU), lithium hexamethyldisilazide, or the like.

The electrophiles for reactions under neutral condition may be isocyanate, isothiocyanate, or the like, having substituent R¹² previously defined.

The electrophiles for reactions in the presence of a condensing agent may be carboxylic acid, sulfonic acid, thiocarboxylic acid, or the like, having substituent R¹² previously defined.

The condensing agent may be dicyclohexylcarbodi imide (DCC), diisopropylcarbodiimide (DIPC), N-ethyl-N'-3-dimethylaminopropylcarbodiimide (WSCI) and its hydrochloride (WSC·HCl), benzotriazol-1-yl tris(dimethylamino)phosphonium hexafluorophosphate (BOP), diphenylphosphoryl azide (DPPA), or the like agent. These may be used alone or, alternatively, in combination with N-hydroxysuccinimide (HONSu), 1-hydroxybenzotriazole (HOBt), 3-hydroxy-4-oxo-3,4-dihydro-1,2,3-benzotriazine (HOObt), or the like agent.

Preferably the reaction is carried out in a solvent, and the solvent includes halogenated solvents, such as methylene chloride, 1,2- dichloroethane, and the like; etheral solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like; alcoholic solvents, such as methanol, ethanol, propanol, isopropanol, and the like; acetone; water; and the like solvents. These may be used alone or as a mixed solvent.

The reaction temperature is selected within the range of from -70°C to 100°C, preferably from 0°C to 60°C (or the boiling point of the solvent).

The reaction time mainly depends on the reaction temperature, starting material, reagent, solvent, and the like, and it is usually from 1 hour to 2 days.

If Compound (2) has a hydroxy group at any position, i.e. any of R⁶, R⁷ and R⁸ is hydrogen, or R⁸ has unprotected carboxyl or hydroxyl, a reaction of the electrophile onto the oxygen atom may also occur depending on the reaction condition. In order to avoid this reaction onto the oxygen atom, it is preferable that the electrophile is allowed to react under a neutral condition or while using a bicarbonate, such as sodium bicarbonate, potassium bicarbonate, or the like.

### (Step A-2)

Compound (3) obtained above is treated with an acylating agent in the presence of a base to give Compound (4).

The acylating agent may be a carboxyl halide, carboxylic anhydride, or the like corresponding to the acyl group represented by R¹³ or R¹⁴ as defined above. The halogen atom of the halide may be chlorine, bromine, iodine, or the like.

The base may be a carbonate, such as sodium carbonate, potassium carbonate, or the like; hydroxide, such as sodium hydroxide, potassium hydroxide, or the like; bicarbonate, such as sodium bicarbonate, potassium bicarbonate, or the like; disodium hydrogen phosphate; dipotassium hydrogen phosphate; organic base, such as triethylamine, N,N-diisopropylethylamine, pyridine, dimethylaniline, 1,8-diazabicyclo[5.4.0]undecene (DBU), lithium hexamethyldisilazide, and the like.

Preferably the reaction is carried out in a solvent, and the solvent includes halogenated solvents, such as methylene chloride, 1,2- dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like. These solvents may be used alone or as a mixed solvent.

The reaction temperature is selected within the range of from -70°C to 100°C, preferably from 0°C to 60°C (or the boiling point of the solvent).

The reaction time depends mainly on the reaction temperature, starting material, reagent, solvent, and the like, and it is usually from 1 hour to 2 days.

When Compound (3) has a carboxyl group, i.e., R⁹ is hydrogen, it is necessary to protect it prior to the reaction. The protection of the carboxyl group with C₁-C₆ alkyl includes, for example, a reaction with diazoalkane, such as diazomethane, diazoethane, and the like, and a reaction with the corresponding alcohol in the presence of a condensing agent and an organic base.

The condensing agent may be dicyclohexylcarbodi imide (DCC), diisopropylcarbodiimide (DIPC), N-ethyl-N'-3-dimethylaminopropylcarbodiimide (WSCI) or its hydrochloride (WSC·HCl), benzotriazol-1-yl tris(dimethylamino)phosphonium hexafluorophosphate (BOP), diphenylphosphoryl azide (DPPA), or the like agent. These may be used alone or in combination with N-hydroxysuccinimide (HONSu), 1-hydroxybenzotriazole (HOBt), 3-hydroxy-4-oxo-3,4-dihydro-1,2,3-benzotriazine (HOObt), or the like.

The organic base may, for example, betriethylamine, N,N-diisopropylethylamine, pyridine, N,N-dimethylaminopyridine, dimethylaniline, 1,8-diazabicyclo[5.4.0]undecene (DBU), or the like base.

The reaction is preferably carried out in a solvent, and the solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like; alcoholic solvents, such as methanol, ethanol, propanol, isopropanol, and the like; acetone; water; and the like solvents. These may be used alone or as a mixed solvent.

The reaction temperature is selected within the range of from -70°C to 100°C, preferably from 0°C to 60°C (or the boiling point of the solvent).

The reaction time depends mainly on the reaction temperature, starting material, reagent, solvent, and the like, and it is usually from 1 hour to 2 days.

When none of R⁶, R⁷ and R⁸ is hydrogen and R⁸ does not have an unprotected carboxyl nor unprotected hydroxyl, this step is unnecessary to carry out, because Compound (3) and Compound (4) are equivalent.

### (Step A-3)

Compound (4) obtained above is treated with a halogenating agent, e.g., 1,1-dihalomethyl methyl ether, in the presence of a catalytic amount of Lewis acid according to the method of Magnusson et al. (J. Org. Chem., 55: 3181 (1990)) to give the corresponding sugar halide (5). The halogen atom of the halide may be chlorine, bromine, iodine, or the like.

Alternatively, it may be converted to 1-O-acylated sugar (5) or 1-OH sugar by reacting with a carboxylic anhydride in the presence of Lewis acid or with a protic acid according to another method of Magnusson et al. (J. Org. Chem., 53: 5629 (1988)).

The Lewis acid to be used includes, for example, zinc chloride, zinc bromide, zinc iodide, tin(II) chloride, tin(II) bromide, tin(II) iodide, tin(IV) chloride, tin(IV) bromide, tin(IV) iodide, titanium tetrachloride, titanium tetrabromide, titanium tetraiodide, iron(III) chloride, iron(III) bromide, iron(III) iodide, aluminum chloride, aluminum bromide, aluminum iodide, trimethylsilyl trifluoromethanesulfonate (TMS triflate), tin triflate, boron trifluoride etherate, and the like Lewis acids.

The protic acid includes, for example, formic acid, acetic acid, trifluoroacetic acid, methanesulfonic acid, trifluoromethanesulfonic acid, hydrochloric acid, perchloric acid, sulfuric acid, and the like protonic acids.

The amount of Lewis acid is usually 0.001 to 3 equivalents. The amount of protonic acid is usually 0.01 equivalents or more. Occasionally, it is used in a large excess amount, which serves as a solvent for reaction.

The reaction is carried out in the presence or absence of a solvent. The solvent to be used includes halogenated solvents, such as chloroform, methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; polar aprotic solvents, such as dimethylformamide (DMF), dimethylacetamide, dimethyl sulfoxide (DMSO), acetonitrile, nitromethane, and the like. These may be used alone or as a mixed solvent.

The reaction temperature is selected within the range of from -70°C to 100°C, preferably from -20°C to 60°C (or the boiling point of the solvent).

The reaction time depends mainly on the reaction temperature, starting material, reagent, solvent, and the like, and it is usually from 1 hour to 2 days.

These halide, 1-O-acylated sugar, or 1-OH compound may be converted to Compound (5) having alkylthio, phenylthio, pyridylthio, phenylsulfinyl, phenylselenyl, imidate, dialkylphosphoryl, diphenylphosphoryl, or other various leaving group Z, according to known methods (see, e.g., "Jikken Kagaku Kouza", 4th Ed., Vol. 26, Organic Synthesis VII, pp. 267-354, Ed. by Japan Chemical Society (1992)).

### (Step A-4)

Compound (5) obtained above is treated with various alcohol or phenol in the presence of a Lewis acid or metal salt to give Compound (6) (see, e.g., "Jikken Kagaku Kouza", 4th Ed., Vol. 26, Organic Synthesis VII, pp. 267-354, Ed. by Japan Chemical Society (1992)).

The Lewis acid or metal salt for this reaction includes zinc chloride, zinc bromide, zinc iodide, tin(II) chloride, tin(II) bromide, tin(II) iodide, tin(IV) chloride, tin(IV) bromide, tin(IV) iodide, titanium tetrachloride, titanium tetrabromide, titanium tetraiodide, iron(III) chloride, iron(III) bromide, iron(III) iodide, copper(II) chloride, copper(II) bromide, aluminum chloride, aluminum bromide, aluminum iodide, mercury(II) chloride, mercury(II) bromide, mercury(II) iodide, mercury(II) cyanide, mercury(II) oxide, silver oxide, silver carbonate, silver perchlorate, silver(I) chloride, silver(I) bromide, silver(I) iodide, silver silicate, silver tetrafluoroborate, silver zeolite, trimethylsilyl trifluoromethanesulfonate (TMS triflate), silver triflate, tin triflate, methyl triflate, triflic anhydride, trityl perchlorate, silicone tetrafluoride, trimethylsilyl chloride, trimethylsilyl bromide, trimethylsilyl iodide, boron trifluoride etherate, and the like Lewis acids or metal salts, which may optionally be used as a combination of them.

The reaction is preferably carried out in a solvent, and the solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, acetonitrile, propionitrile, nitromethane, nitroethane, nitropropane, and the like; acetone; and the like solvents. These may be used alone or as a mixed solvent.

An acid scavenger for trapping the acid formed during the reaction, such as N,N,N',N'-tetramethylurea, pyridine, 2,6-di-t-butyl-pyridine, 2,6-lutidine, 2,4,6-collidine, triethylamine, and Molecular Sieves (MS3A, MS4A, or MS5A), or the like, may be added to the reaction mixture.

The reaction is preferably carried out under anhydrous condition. Then, the solvent, reagent, substrate, reaction vessel, and the like is freed from moisture as much as possible. A dehydrating agent, such as Molecular Sieves (MS3A, MS4A, or MS5A) or anhydrous calcium sulfate or the like may be added to the reaction medium to remove moisture.

The reaction using a silver salt is preferably carried out under protection from light.

The reaction temperature is selected within the range of from -70°C to 100°C, preferably from -20°C to 60°C (or the boiling point of the solvent).

The reaction time depends mainly on the reaction temperature, starting material, reagent, solvent, and the like, and it is usually from 1 hour to 5 days.

### (Step A-5)

Compound (6) obtained above is hydrolyzed under basic condition in a solvent to give Lewis X derivative (1).

The base includes carbonates, such as sodium carbonate, potassium carbonate, and the like; hydroxides, such as sodium hydroxide, potassium hydroxide, and the like; bicarbonates, such as sodium bicarbonate, potassium bicarbonate, and the like; disodium hydrogen phosphate; dipotassium hydrogen phosphate; organic bases, such as triethylamine, N,N-diisopropylethylamine, pyridine, dimethylaniline, 1,8-diazabicyclo[5.4.0]undecene (DBU), lithium hexamethyldisilazide, sodium methoxide, sodium ethoxide, and the like.

The reaction is preferably carried out in a solvent. The solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like; alcoholic solvents, such as methanol, ethanol, propanol, isopropanol, and the like; acetone; water; and the like solvents. These may be used alone or as a mixed solvent.

When Compound (6) has a protected carboxyl group, it may be deprotected either by using a mixed solvent containing water, by adding water or a basic aqueous solution after deprotection of a hydroxy group, or by using a base in water or an aqueous solvent after isolating a deprotected hydroxy compound.

The reaction temperature is selected within the range of from -70°C to 100°C, preferably from 0°C to 60°C (or the boiling point of the solvent).

The reaction time depends mainly on the reaction temperature, starting material, reagent, solvent, and the like, and it is usually from 1 hour to 2 days.

Alternatively, Lewis X derivative (1) may be prepared from Compound (2) by the method as outlined below. 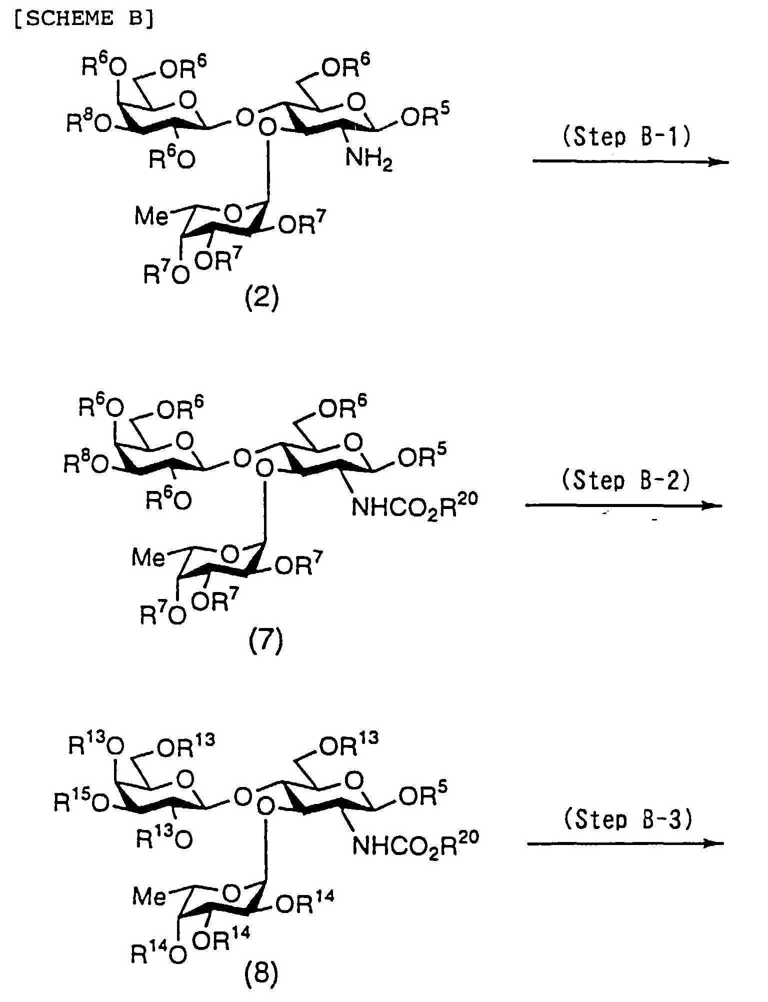 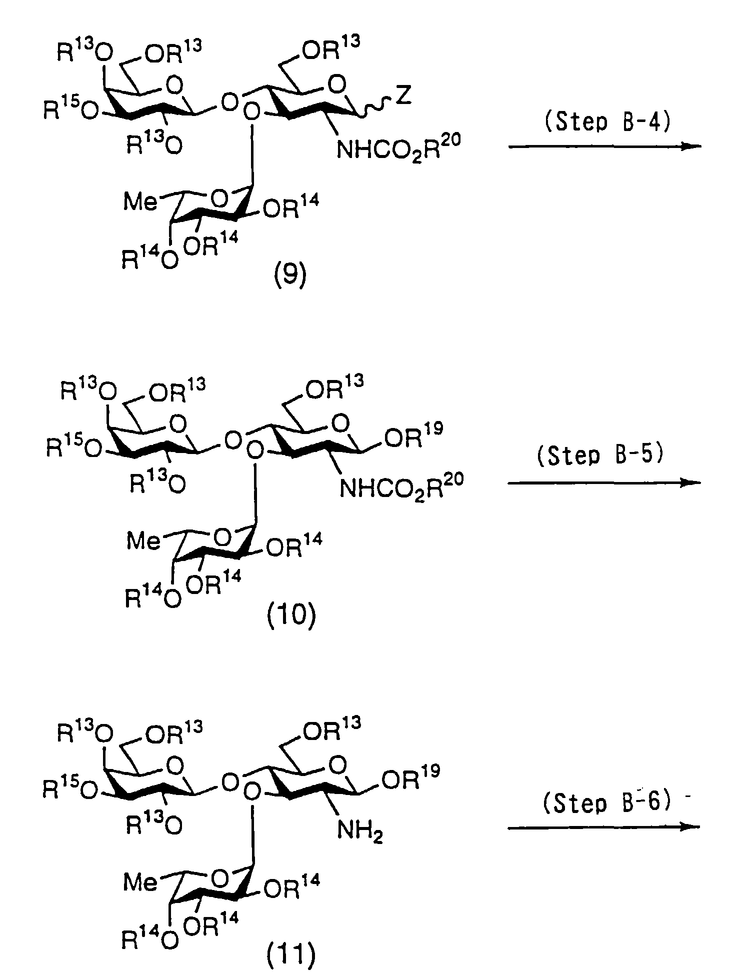 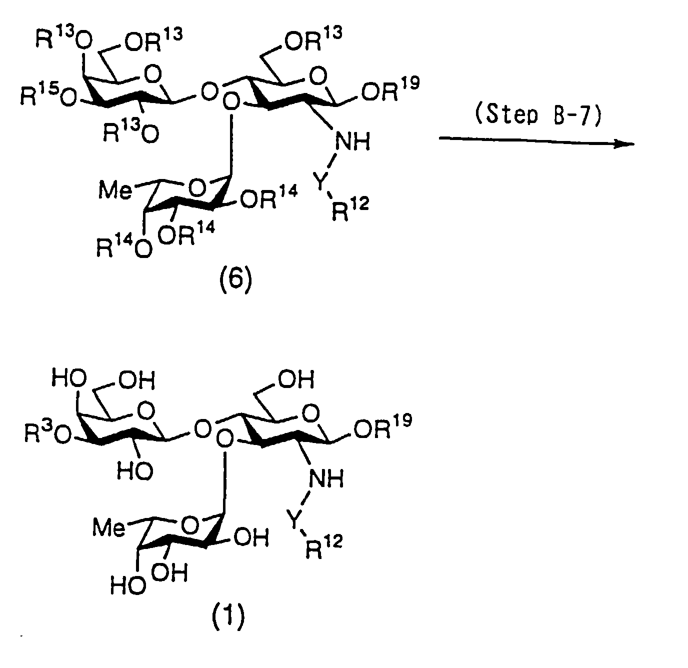 [wherein R⁵, R⁶, R⁷, R⁸, R²⁰, R¹³, R¹⁴, R¹⁵, Z, R¹⁹, Y, R¹² and R³ are as defined above]

### (Step B-1)

The nitrogen atom of Compound (2) is protected with allyloxycarbonyl (Alloc), t-butoxycarbonyl (Boc) or benzyloxycarbonyl (Cbz) to give Compound (7).

As a reagent for introducing the protective group, the corresponding haloformate or pyrocarbonate may be employed (See, e.g., T. W. Greene and P.G.M. Wuts, "Protective Groups in Organic Synthesis", 2nd Ed., pp. 327-338, John Wiley & Sons, Inc. (1991)).

The reaction is usually carried out under neutral condition or in the presence of a base.

The base may be a carbonate, such as sodium carbonate, potassium carbonate, or the like; hydroxide, such as sodium hydroxide, potassium hydroxide, or the like; bicarbonate, such as sodium bicarbonate, potassium bicarbonate, or the like; disodium hydrogen phosphate; dipotassium hydrogen phosphate; organic base, such as triethylamine, N,N-diisopropylethylamine, pyridine, dimethylaniline, 1,8-diazabicyclo[5.4.0]undecene (DBU), lithium hexamethyldisilazide, or the like.

The reaction is preferably carried out in a solvent. The solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like; alcoholic solvents, such as methanol, ethanol, propanol, isopropanol, and the like; acetone; water; and the like solvents. These may be used alone or as a mixed solvent.

The reaction temperature is selected within the range of from -70°C to 100°C, preferably from 0°C to 60°C (or the boiling point of the solvent).

The reaction time depends mainly on the reaction temperature, starting material, reagent, solvent, and the like, and it is usually from 1 hour to 1 day.

### (Step B-2)

Compound (7) obtained above is acylated by a method similar to that of Step A-2 to give Compound (8) wherein all functional groups are protected.

When Compound (7) has a carboxyl group, i.e., when R⁹ is hydrogen, it is protected prior to the reaction by a method similar to that in Step A-2.

When none of R⁶, R⁷ and R⁸ is hydrogen and R⁸ has no unprotected carboxyl group and unprotected hydroxyl group, this step is unnecessary to carry out, because Compound (7) and Compound (8) are equivalent.

### (Step B-3)

Compound (8) obtained above is converted to Compound (9) having a leaving group Z by a method similar to that of Step A-3.

### (Step B-4)

Compound (9) obtained above is reacted by a method similar to that of Step A-4 to prepare glycosidated Product (10).

### (Step B-5)

The protective group on the amino group of Compound (10) obtained above is selectively deprotected to give Compound (11).

The method for deprotection may, for example, be found in T. W. Greene and P.G.M. Wuts, "Protective Groups in Organic Synthesis", 2nd Ed., p. 327-338, John Wiley & Sons, Inc. (1991). In carrying out the deprotection, a method for selectively deprotecting only the protective group on an amino group may be chosen.

When such protective group is Alloc, it is, for example, deprotected easily with tetrakis(triphenyl phosphine) palladium in the presence of an allyl scavenger. The amount of tetrakis(triphenylphosphine) palladium may be catalytic and usually 0.001 to 1 equivalent. The allyl scavenger includes, for example, dimedone, diethyl malonate, morpholine, aniline, tributyltin hydride, polymethylhydrosiloxane, and the like.

When such protective group is Cbz, it is, for example, deprotected easily by hydrogenation in the presence of palladium on carbon. The amount of palladium on carbon may be catalytic and usually 0.001 to 1 equivalent. The hydrogen donor may, for example, be hydrogen molecule, formic acid, ammonium formate, cyclohexene, 1,4-cyclohexadiene, cis-decaline, or the like donor.

Reaction is preferably carried out in a solvent. The solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like; alcoholic solvents, such as methanol, ethanol, propanol, isopropanol, and the like. These may be used alone or as a mixed solvent.

The reaction temperature is selected within the range of from -70°C to 100°C, preferably from 0°C to 60°C (or the boiling point of the solvent).

The reaction time depends mainly on the reaction temperature, selected starting material, reagent, solvent, and the like conditions, and it is usually from 1 hour to 1 day.

### (Step B-6)

Compound (11) obtained above is treated with an electrophile by a method similar to that of Step A-1 to give Compound (6).

### (Step B-7)

Compound (6) obtained above is hydrolyzed by a method identical with that of Step A-5 to give Lewis X derivative (1).

Compound (2) employed in Scheme A and Scheme B may be prepared by the method as follows: 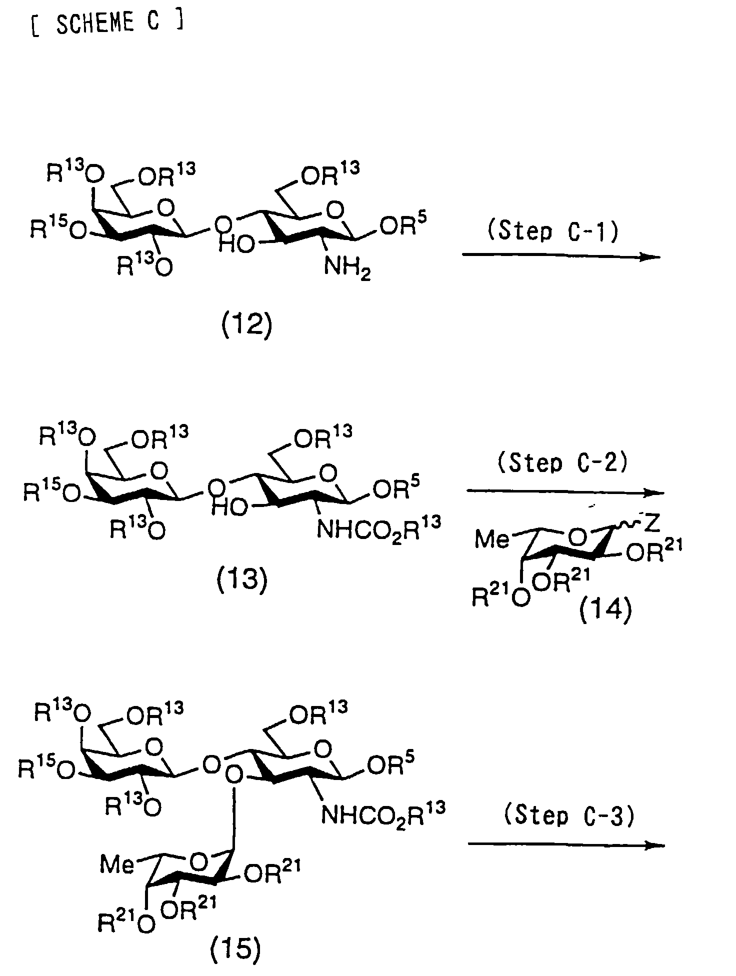 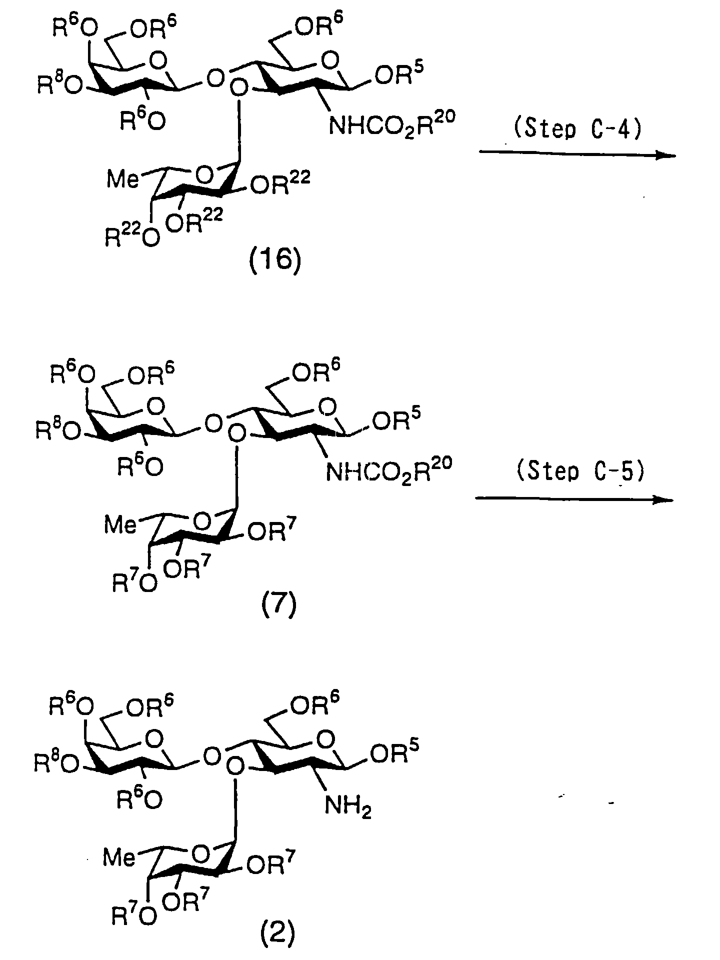 [wherein R⁵, R¹³, R¹⁵, R²⁰, R²¹, Z, R⁶, R⁸ and R⁷ are as defined above; and R²² is hydrogen, C₁-C₆ alkanoyl, aroyl, benzyl, or substituted benzyl]

### (Step C-1)

The nitrogen atom of Compound (12) is protected with allyloxycarbonyl (Alloc), t-butoxycarbonyl (Boc) or benzyloxycarbonyl (Cbz) by a method similar to that of Step B-1 to give Compound (13).

### (Step C-2)

Compound (13) obtained above is treated with Compound (14) having a leaving group Z, in the presence of a Lewis acid or metal salt to give glycosidated Product (15).

The Lewis acid or metal salt to be used in the reaction includes zinc chloride, zinc bromide, zinc iodide, tin(II) chloride, tin(II) bromide, tin(II) iodide, tin(IV) chloride, tin(IV) bromide, tin(IV) iodide, titanium tetrachloride, titanium tetrabromide, titanium tetraiodide, iron(III) chloride, iron(III) bromide, iron(III) iodide, copper(II) chloride, copper(II) bromide, aluminum chloride, aluminum bromide, aluminum iodide, mercury(II) chloride, mercury(II) bromide, mercury(II) iodide, mercury(II) cyanide, mercury(II) oxide, silver oxide, silver carbonate, silver perchlorate, silver(I) chloride, silver(I) bromide, silver(I) iodide, silver silicate, silver tetrafluoroborate, silver zeolite, trimethylsilyl trifluoromethanesulfonate (TMS triflate), silver triflate, tin triflate, methyl triflate, triflic anhydride, trityl perchlorate, silicone tetrafluoride, trimethylchlorosilane, trimethylsilyl bromide, trimethylsilyl iodide, boron trifluoride etherate, and the like Lewis acids or metal salts, which may optionally be used in combination of them (see, e.g., "Jikken Kagaku Kouza", 4th Ed., Vol. 26, Organic Synthesis VII, pp. 267-354, Ed. by the Japan Chemical Society (1992)).

The reaction is preferably carried out in a solvent. The solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, propionitrile, nitromethane, nitroethane, nitropropane, and the like; acetone; and the like solvents. These may be used alone or as a mixed solvent.

An acid scavenger, such as N,N,N',N'-tetramethylurea, pyridine, 2,6-di-t-butylpyridine, 2,6-lutidine, 2,4,6-collidine, triethylamine, Molecular Sieves (MS3A, MS4A, or MS5A), and the like scavenger for trapping the acid formed during the reaction may be added to the reaction medium.

The reaction is preferably carried out under anhydrous condition. Then the solvent, reagent, substrate, reaction vessel, and the like will preferably be freed from moisture as much as possible. A dehydrating agent, such as Molecular Sieves (MS3A, MS4A, or MS5A), anhydrous calcium sulfate, or the like, may be added to the reaction medium to remove moisture.

The reaction using silver salt is preferably carried out under protection from light.

The reaction temperature is selected within the range of from -70°C to 100°C, preferably from -20°C to 60°C (or the boiling point of the solvent).

The reaction time depends mainly on the reaction temperature, starting material, reagent, solvent, and the like, and it is usually from 1 hour to 5 days.

Compound (14) having a leaving group Z utilizable in this invention is prepared conventionally using 2,3,4-tri-O-protected-L-fucopyranose, alkyl 2,3,4-tri-O-protected-L-fucopyranoside or acyl 2,3,4-tri-O-protected-L-fucopyranoside (see, e.g., "Jikken Kagaku Kouza", 4th Ed., Vol. 26, Organic Synthesis VII, pp. 267-354, Ed. by Japan Chemical Society (1992)).

### (Step C-3)

Compound (15) obtained above is optionally hydrolyzed by a method similar to that of Step A-5 to convert to Compound (16).

When the protective groups in Compound (15) need not be deprotected, Compound (15) and Compound (8) are identical, and this step is unnecessary to carry out.

### (Step C-4)

When R²² in Compound (16) is a benzyl or substituted benzyl group, it is, for example, deprotected easily by hydrogenation in the presence of palladium on carbon to give Compound (7).

The amount of palladium on carbon may be catalytic and usually 0.001 to 1 equivalent. The hydrogen donor may, for example, be molecular hydrogen, formic acid, ammonium formate, cyclohexene, 1,4-cyclohexadiene, cis-decaline, or the like.

If R²² is 4-methoxybenzyl, it may be deprotected with cerium ammonium nitrate (CAN) or dichlorodicyanoquinone (DDQ) to give Compound (7).

Preferably each reaction is carried out in a solvent, and the solvent includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, dimethyl sulfoxide (DMSO), acetonitrile, and the like; alcoholic solvents, such as methanol, ethanol, propanol, isopropanol, and the like; water; and the like solvents. These may be used alone or as a mixed solvent.

The reaction temperature is selected within the range of from -70°C to 100°C, preferably from 0°C to 60°C (or the boiling point of the solvent).

The reaction time depends mainly on the reaction temperature, starting material, reagent, solvent, and the like, and it is usually from 1 hour to 1 day.

When R²² in Compound (16) is not benzyl nor substituted benzyl, Compound (16) and Compound (7) are the same, and this step is unnecessary to carry out.

When both of R²⁰ and R²² in Compound (16) are benzyl, i.e., the protective group on an amino group is Cbz and R²² is benzyl, the hydrogenation in this step deprotects both of them to give objective Compound (2) without carrying out the next Step C-5.

### (Step C-5)

The protective group of Compound (7) obtained above is removed by a method similar to Step B-5 to prepare objective Compound (2).

Alternatively, Compound (2) employed in Scheme A and Scheme B may be prepared by the methods as follows: 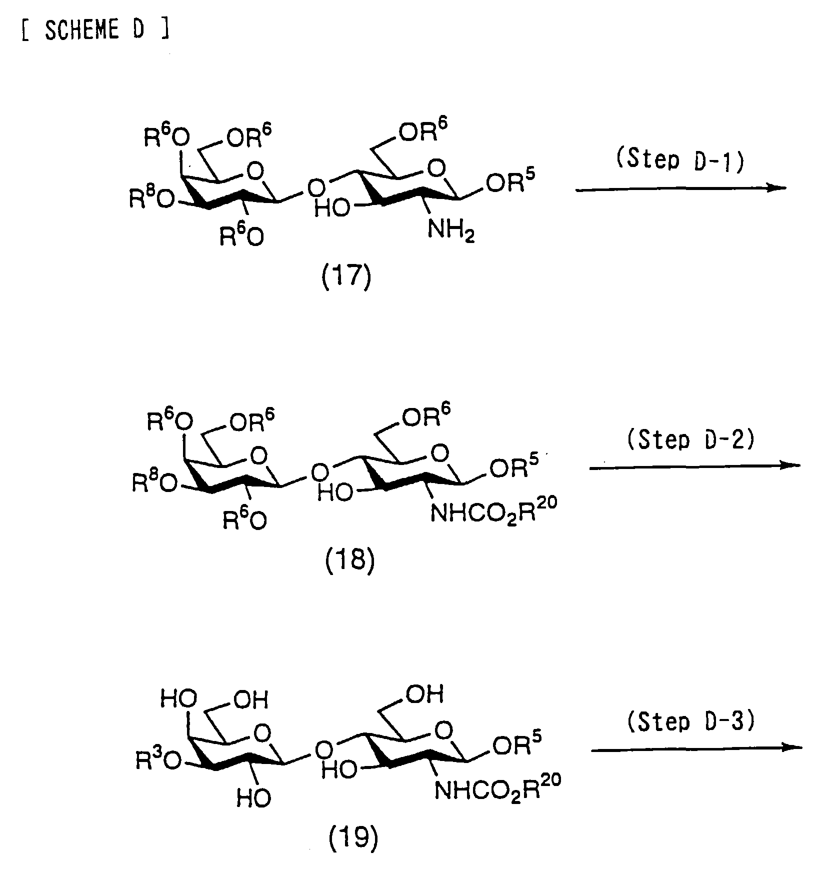 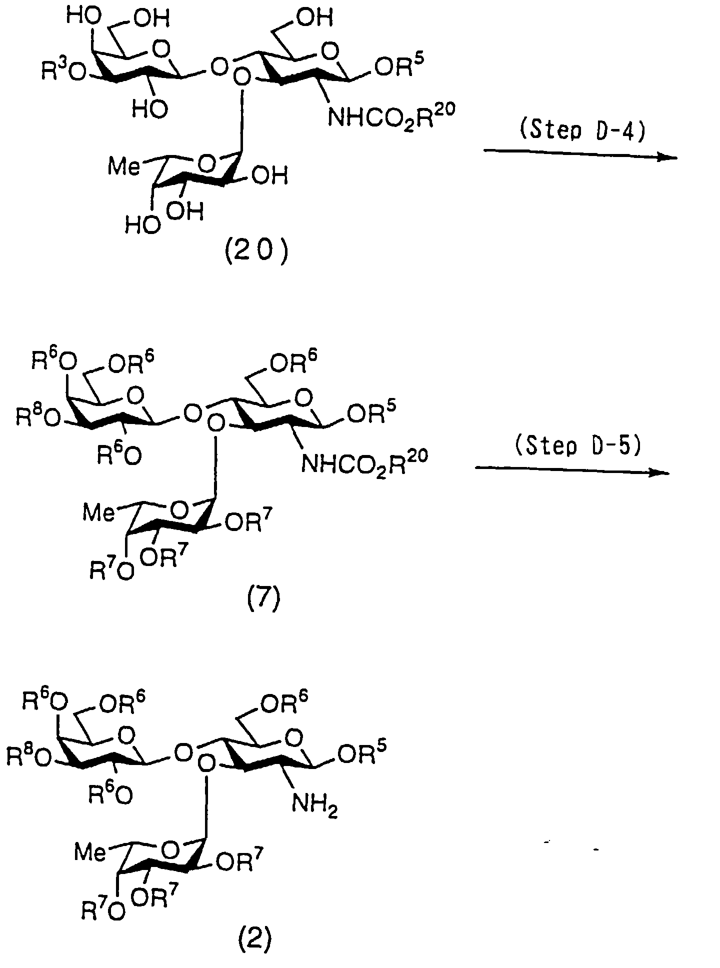 [wherein R⁵, R⁶, R⁸, R²⁰, R³ and R⁷ are as defined above]

### (Step D-1)

The nitrogen atom in Compound (17) may be protected with allyloxycarbonyl (Alloc), t-butoxycarbonyl (Boc), or benzyloxycarbonyl (Cbz) to give Compound (18).

### (Step D-2)

When the hydroxyl or carboxyl group in Compound (18) obtained above is protected, such group may be deprotected by a method similar to that of Step A-5 to give Compound (19).

Compound (19) may be prepared by Steps E-1 and E-2 as described below.

### (Step D-3)

Compound (19) obtained above may be treated with GDP-fucose using fucose transferase to prepare Compound (20).

This reaction may be carried out by a method known in literatures (M. M. Palcic, et al., Carbohydr. Res., 190: 1 (1989); C-H. Wong, et al., Bioorg. Med. Chem. Lett., 1: 425 (1991)).

Thus, GDP-fucose, manganese chloride, Compound (19), and fucosyl transferase are added to a pH-buffer to react for obtaining Compound (20). If necessary, sodium azide may be added.

pH-Buffer may, for example, be sodium arsenate buffer, potassium arsenate buffer, N-(2-hydroxyethyl) piperazine-N'-(2-ethanesulfonic acid) (HEPES) buffer, and the like.

The pH of the medium is selected within the range from 4 to 9, preferably from 6 to 8.

The reaction temperature is selected within the range of from 0°C to 60°C, preferably from 20°C to 45°C.

The reaction time depends mainly on the reaction temperature, pH, starting material selected, equivalent of the reagent, volume of solvent, and the like, and is usually from 1 day to 2 weeks.

GDP-fucose means guanosine-5'-diphospho-1-L-fucopyranoside, commercially available from Sigma Chemical Corp., and producible from L-fucopyranose by the method of R. R. Schmidt et al. (Tetrahedron Lett., 33: 1585 (1992)).

### (Step D-4)

The hydroxyl and carboxyl groups in Compound (20) obtained above may optionally be protected by a method similar to that of Step A-2 to give Compound (7).

If protection of the hydroxyl and carboxyl groups is not necessary in Compound (20), this step is unnecessary to carry out, as Compound (20) and Compound (7) are the same.

### (Step D-5)

The protective group on the amino group in Compound (7) obtained above is removed by the method similar to that of Step B-5 to give the objective Compound (2).

The starting materials (12) and (17) in the Scheme C and Scheme D may be prepared by the method as described below: 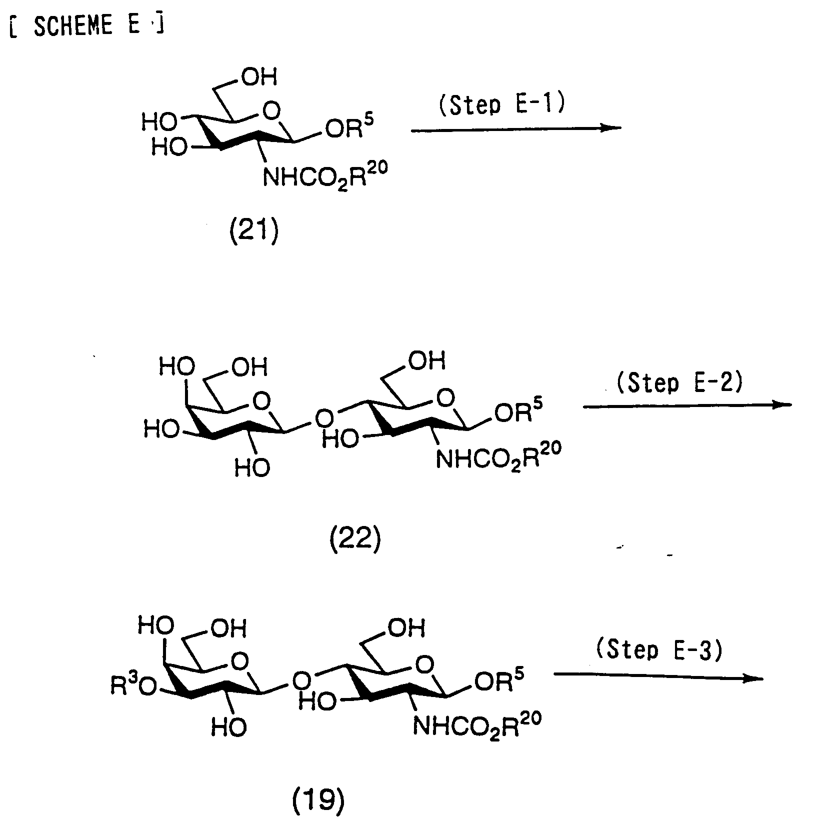 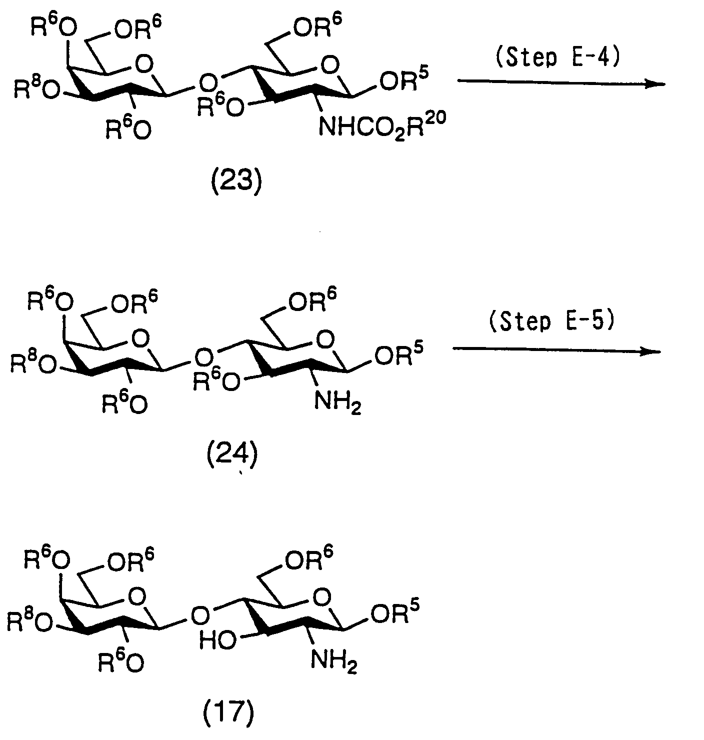 [wherein R⁵, R²⁰, R³, R⁶ and R⁸ are as defined above]

### (Step E-1)

Glucosamine derivative (21) may be treated with UDP-galactose using a galactosyl transferase to give Compound (22).

This reaction may be carried out by a method known in literatures (e.g., J. C. Paulson et al., J. Am. Chem. Soc., 108: 2068 (1986); S. L. Flitsch et al., J. Chem. Soc., Chem. Commun., 1526 (1992)). These methods utilize easily available UDP-glucose and UDP-galactose-4-epimerase for synthesizing UDP-galactose in situ, in place of utilizing UDP-galactose which is not readily available.

Thus, UDP-glucose, bovine serum albumin, manganese chloride, Compound (21), and UDP-galactose 4-epimerase, and galactosyl transferase are added to a pH buffering solution to prepare Compound (20). If necessary, sodium azide and/or alkaline phosphatase may be added.

The pH buffer may, for example, be sodium arsenate buffer, potassium arsenate buffer, N-(2-hydroxyethyl)piperazine- N'-(2-ethanesulfonic acid) (HEPES) buffer, or the like.

When the substrate has a poor solubility to a buffering solution, a small amount of surfactant (e.g., Triton CF-54, etc., 0.1-2.0%) or a small amount of alcohol (e.g., methanol, etc., 0.1-10%) may be added.

The pH of the medium is selected within the range from 4 to 9, preferably from 6 to 8.

The reaction temperature is selected within the range of from 0°C to 60°C, preferably from 20°C to 45°C.

The reaction time depends mainly on the reaction temperature, pH, starting material used, equivalent of reagent, volume of solvent, and the like, and it is usually from 1 day to 2 weeks.

UDP-galactose means uridine-5'-diphospho-D-galactopyranoside and UDP-glucose means uridine-5'-diphospho-D-glucopyranoside. The latter is available from Sigma Chemical Corp.

A method using D-glucose-1-phosphate (e.g., C. Auge et al., Tetrahedron Lett., 25: 1467 (1984); C-H. Wong et al., J. Am. Chem. Soc.,113: 6300 (1991)), a method using D-glucose-6-phosphate (G. M. Whitesides et al, J. Org. Chem., 47: 5416 (1982)), and a method using D-galactose (C-H. Wong et al., J. Org. Chem., 57: 4343 (1992)), in place of said UDP-galactose have been reported. These methods may be employed for converting glucosamine derivative (21) into Compound (22).

### (Step E-2)

Compound (22) obtained above is optionally treated with CMP-N-acetylneuraminic acid using sialyl transferase to give Compound (19).

This method may be carried out according to a method known in literatures (e.g., C-H. Wong et al., J. Am. Chem. Soc., 113: 4698 (1991); J. C. Paulson et al., J. Am. Chem. Soc., 115: 1603 (1993)). In these methods, CMP-N-acetylneuraminic acid which is not readily available is prepared from sialic acid utilizing a CMP-N-acetylneuraminic acid synthetase in situ.

Thus, sialic acid (N-acetylneuraminic acid), bovine serum albumin, magnesium chloride, potassium chloride, manganese chloride, Compound (23), phospho(enol)pyruvate, a catalytic amount of cytidine-5'-monophosphate (CMP), a catalytic amount of adenosine-5'-triphosphate (ATP), myokinase, pyruvate kinase, CMP-N-acetylneuraminic acid synthetase, and sialyl transferase are added to a pH buffering solution to prepare Compound (19). 2-Mercaptoethanol and/or alkaline phosphatase may optionally be added.

The pH buffer may, for example, be sodium arsenate buffer, potassium arsenate buffer, N-(2-hydroxyethyl)piperazine- N'-(2-ethanesulfonic acid) (HEPES) buffer, and the like.

When the substrate has a poor solubility to a buffering solution, a small amount of surfactant (e.g., Triton CF-54, etc., 0.1-2.0%) or a small amount of alcohol (e.g., methanol, etc., 0.1-10%) may be added.

The pH of the medium is selected within the range of from 4 to 9, preferably from 6 to 8.

The reaction temperature is selected within the range of from 0°C to 60°C, preferably from 20°C to 45°C.

The reaction time depends mainly on the reaction temperature, pH, starting material used, equivalent of reagent, volume of solvent, and the like, and it is usually from 1 day to 2 weeks.

CMP-N-acetylneuraminic acid means cytidine-5'-monophospho-N-acetylneuraminic acid, which is commercially available from Sigma Corp although it is very expensive. This may, however, be prepared from sialic acid or N-acetylglucosamine according to a method known in literatures (C. Auge et al., Carbohydr. Res., 200: 257 (1990); G. M. Whitesides et al, J. Am. Chem. Soc., 110: 7159 (1988)).

When one equivalent or more of CMP-N-acetylneuraminic acid is allowed to use for the reaction, Compound (22) can be converted to Compound (19) similarly to the above using a method known in literatures (e.g., J.C. Paulson et al., J. Am. Chem. Soc., 108: 2068 (1986); S. L. Flitsch etal., J. Chem. Soc., Chem. Commun., 1526 (1992)).

When introduction of sialic acid into Compound (22) is not necessary, Compound (22) and Compound (19) are the same, and this step is unnecessary.

### (Step E-3)

The hydroxyl and carboxyl groups in Compound (19) obtained above may optionally be protected by a method similar to that of Step A-2 to give Compound (23).

When protection of the hydroxyl and carboxyl groups in Compound (22) is unnecessary, Compound (19) and Compound (23) are the same and this step is not necessary.

### (Step E-4)

The protective group on the amino group in Compound (23) obtained above may be removed by a method similar to that of Step B-5 to give Compound (24).

### (Step E-5)

In Compound (24) obtained above, only the protective group of 3-hydroxyl group in glucosamine moiety may selectively be deprotected to give the objective Compound (17).

Such regiospecific deprotection proceeds in a solvent under neutral or weakly acidic condition.

The solvent to be used includes halogenated solvents, such as methylene chloride, 1,2-dichloroethane, and the like; ethereal solvents, such as diethyl ether, tetrahydrofuran, dimethoxyethane, dioxane, and the like; aromatic hydrocarbon solvents, such as benzene, toluene, chlorobenzene, and the like; aliphatic hydrocarbon solvents, such as hexane, cyclohexane, and the like; polar aprotic solvents, such as dimethylformamide (DMF), dimethylacetamide, acetonitrile, propionitrile, nitromethane, nitroethane, nitropropane, and the like; alcoholic solvents, such as methanol, ethanol, propanol, isopropanol, butanol, t-butanol, amyl alcohol, isoamyl alcohol, and the like; acetone; water; and the like solvents. These may be used alone or as a mixed solvent.

The pH of the medium is selected within the range of from 2 to 8, preferably from 4 to 7.

The reaction temperature is selected within the range of from -70°C to 100°C, preferably from -20°C to 60°C (or the boiling point of the solvent).

The reaction time depends mainly on the reaction temperature, pH, starting material used, solvent, and the like, and it is usually from 1 day to 2 weeks.

When R⁶ of Compound (24) is hydrogen, Compound (24) and Compound (17) are equivalent, and this step is unnecessary to carry out.

Another objective compound (12) is included in a category of Compound (17) and it is producible by the method of this scheme, while requiring Step E-3.

The starting compound in the method of Scheme E, i.e., glucosamine derivative (21), may be prepared by the method as shown below: 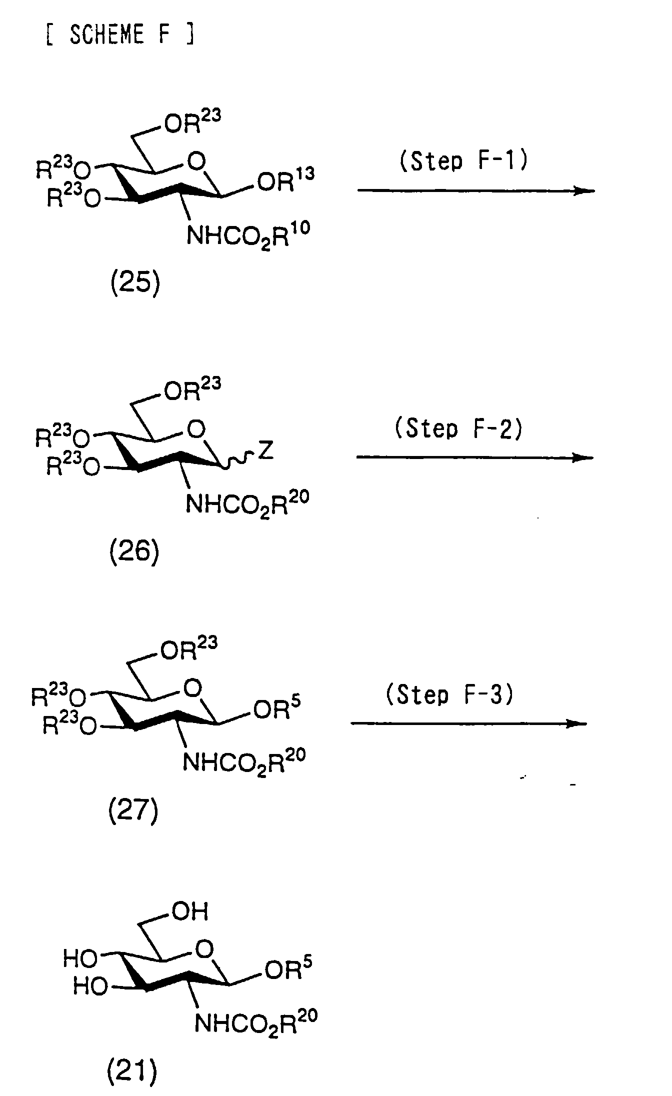 [wherein R¹, R¹⁰ and Z are as defined above; and R²³ is C₁-C₆ alkanoyl or aroyl]

### (Step F-1)

Compound (25) obtained from glucosamine by a method known in literatures (e.g., P. Boullanger et al., Can. J. Chem., 65: 1343 (1987)) is converted to Compound (26) having a leaving group Z by a method similar to that of Step A-3.

### (Step F-2)

Compound (26) obtained above is treated with 2-tri(C₁-C₄ alkyl/phenyl)silyl ethanol by a method similar to that of Step A-4 to give glycosilated product (27).

### (Step F-3)

Compound (27) obtained above is hydrolyzed by a method similar to that of Step A-5 to give desired glucosamine derivative (21).

In each reactions described above, the objective product of the reaction may be collected from the reaction mixture in a conventional manner.

For example, when an objective product of the reaction is insoluble in water, a hydrophobic organic solvent is added to the reaction mixture, and after washing with water, the solvent is removed by evaporation to give the objective product. When an objective product is not lipophilic, the solvent in the reaction mixture is optionally evaporated, and the mixture is dissolved in water, washed with a hydrophobic organic solvent, and water is evaporated off to give the objective product.

Optionally, the resulting product may further be purified by a conventional manner, e.g., recrystallization, reprecipitation, chromatography, or the like.

When used as a pharmaceutical composition, the compound of this invention may block or inhibit cellular adhesion associated with a number of disorders. For example, many inflammatory diorders are associated with selectins expressed on vascular endothelial cells and platelets, and such diseases may be treated with a pharmaceutical composition containing a compound of the present invention. The term "inflammation" is used here to refer to reactions of both the specific and non-specific defense systems. A reaction in the specific defense system is a reaction in specific immune system against an antigen. Examples of reactions in the specific defense system include a response of antibody against its antigen, e.g. virus, and delayed-type hypersensitivity. An example of reaction of the non-specific defense system is an inflammatory response mediated by leukocytes generally incapable immunological memory. Such leukocytes include macrophages, eosinophils and neutrophils. Examples of non-specific response include immediate swelling after bee-sting and aggregation of leukocytes at sites of bacterial infection (e.g., pulmonary infiltrates in bacterial pneumonia and pus formation in abscess).

Other diorders treatable by the composition of the invention include rheumatoid arthritis, post-ischemic leukocyte-mediated tissue damages (reperfusion injury), myocardial infarction, front-bite injury or shock, systemic inflammatory response syndrome (SIRS), acute leukocyte-mediated lung injury [e.g., adult respiratory distress syndrome (ARDS), and the like], asthma, traumatic shock, septic shock, multiple organ failure (MOF), nephritis, acute and chronic inflammation (e.g., atopic dermatitis, psoriasis, inflammatory bowel disease) and the like. Various pathologies associated with platelet [e.g., atherosclerosis, disseminated intravascular coagulation (DIC) syndrome and embolism] may also be treatable by the compound of the invention.

Furthermore, the compound of the invention may inhibit or prevent tumor metastasis by inhibiting adhesion of tumor cells circulating in blood. Such tumor cells are colon carcinoma, melanoma, and the like.

Further, it may be applied to postoperative restenosis after percutaneous transluminal coronary angioplasty (PTCA) and percutaneous transluminal coronary recanalization (PTCR).

The amount of the Lewis X derivative of this invention to be administered in the form of a pharmaceutical composition varies in general depending on the particular compound selected, the manner of administration, the particular disease to be treated and its severity, the overall health and condition of the patient, the prescribing physician, etc. For example, the dose for treating reperfusion injury is in the range of from about 0.5 mg to 2000 mg per day for a patient of 70 kg body weight. Ideally, administration of the compound for therapeutic treatment should begin as early as possible after myocardial infarction or other injury.

A pharmaceutical composition containing the compound of the invention may be administered parenterally, topically, orally, or transdermally. The pharmaceutical composition may be administered for prophylactic and/or therapeutic treatment. The pharmaceutical composition may be administered in various unit dosage forms depending on the method of administration. For example, suitable unit dosage forms for oral administration include powder, tablet, pill, capsule, and dragee. A unit dosage form suitable for topical dosing includes, for example, aerosol.

Preferably, the pharmaceutical composition containing the compound of the invention may be administered intravenously. The composition for intravenous administration comprises a solution of the compound of the invention dissolved or suspended in a pharmaceutically acceptable carrier, preferably an aqueous carrier. The aqueous carrier to be used may, for example, be water, buffer, 0.4% physiological saline, or the like. These compositions may be sterilized by a conventional, well known technique or by sterile filtration. The resulting aqueous solution may be packaged as such, or after lyophilization. The lyophilized preparations are combined with a sterile aqueous solution prior to administration. The composition may contain pharmaceutically acceptable auxiliary substances, for example, pH adjusting and buffering agents, tonicity adjusting agent, wetting agent, and the like, e.g., sodium acetate, sodium lactate, sodium chloride, potassium chloride, calcium chloride, sorbitan monolaurate, triethanolamine oleate, and the like as is required to approximate physiological conditions.

The pharmaceutical composition containing a compound of the invention is administered for prophylactic and/or therapeutic treatment. For therapeutic application, the composition is administered as stated above to a patient already suffering from a disease in an amount sufficient to cure or at least partially arrest the symptoms of the disease and its complication. The amount suitable for achieving this purpose is defined as "therapeutically effective dose for administration". The effective dose for this use depends on severity of disease and body weight and general state of the patient, and generally it is in the range of from about 0.5 mg to about 2,000 mg of the compound of this invention per day for a patient of 70 kg body weight, and preferably the dosage is in the range of from about 5 mg to about 500 mg of the compound of this invention per day for a patient of 70 kg body weight.

For prophylactic application, the composition containing a compound of this invention is administered to a patient susceptible to the particular disease, or alternatively, to a patient prone to such disease. The dosage for this purpose is defined as "prophylactically effective dose". For such use, the dosage varies depending on the condition and body weight of the patient, and generally in the range of from about 0.5 mg to about 1,000 mg of the compound of this invention per day for a patient of 70 kg body weight, and preferably in the range of from about 5 mg to about 500 mg of the compound of the invention per day for a patient of 70 kg body weight.

In administering the compound of the invention, single or multiple dosing of the composition are possible, and the level and administration pattern may be selected by a treating physician. In every case, the pharmaceutical composition will be provided in an amount sufficient for effectively treating the patient with a compound of this invention.

The compound of this invention may also be used as a diagnostic agent. For example, a labeled compound is administered to a patient suspected to have inflammation to identify the locus of inflammation or tumor metastasis. For this use, the compound may be labeled with ¹²⁵I, ¹⁴C or tritium.

### EXAMPLES

The following examples further illustrate the present invention.

### Example 1

The structural formulae of Compounds (1) to (15) in Example 1 are as follows.

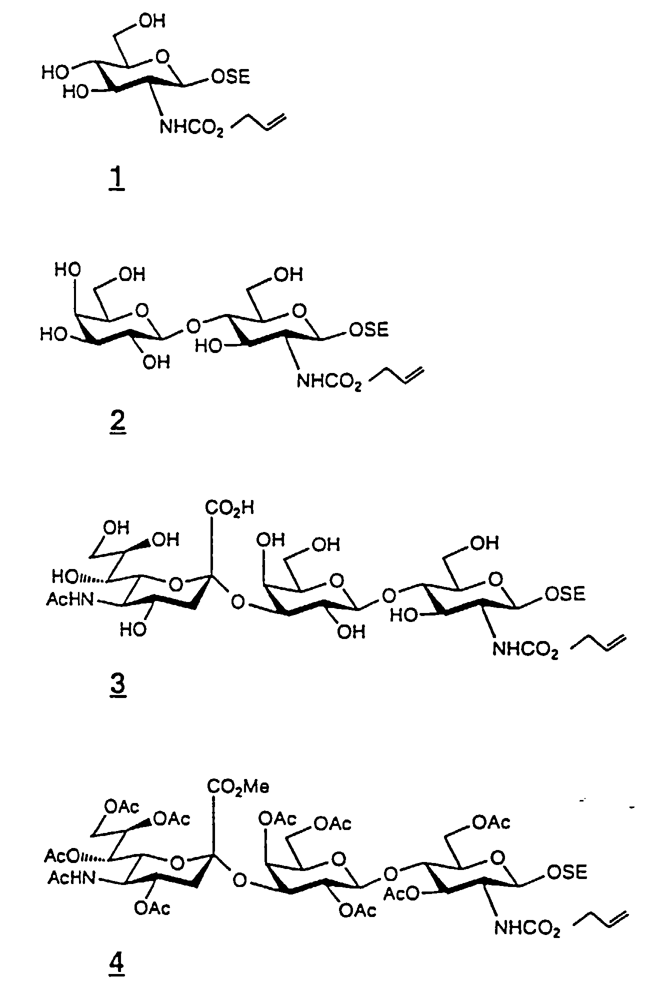

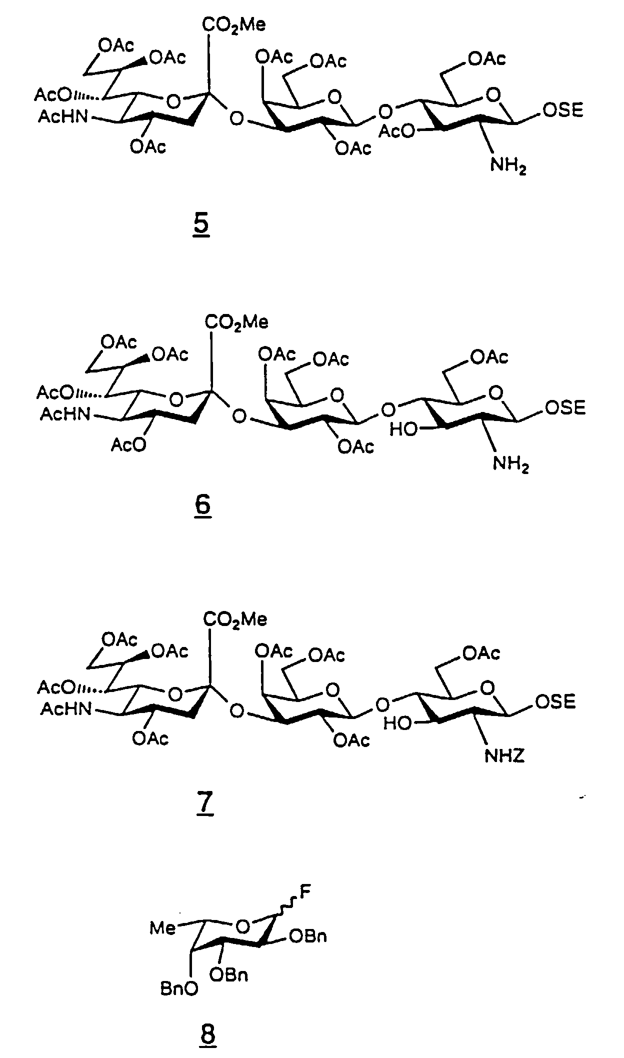

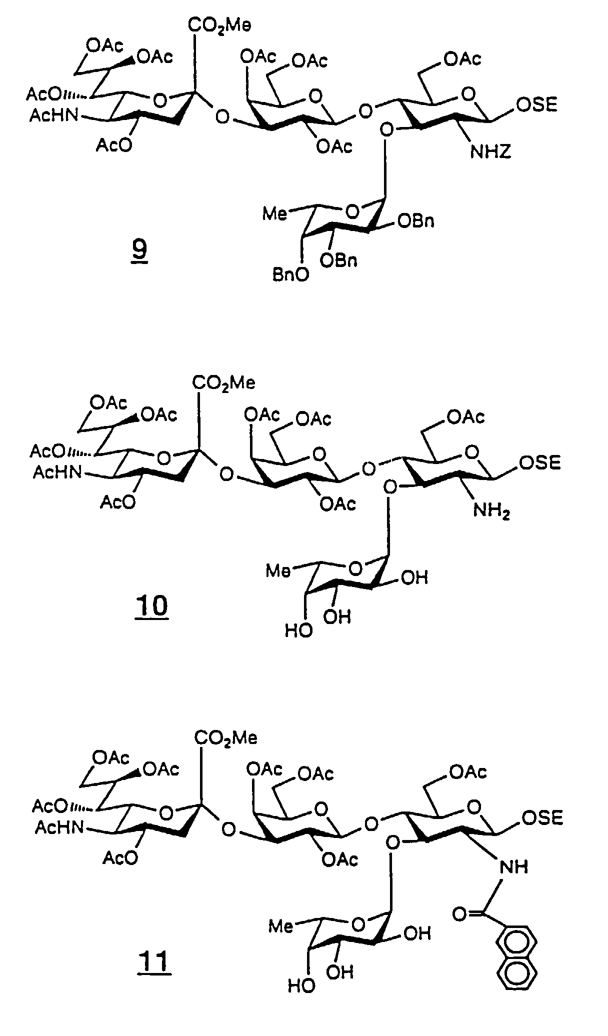

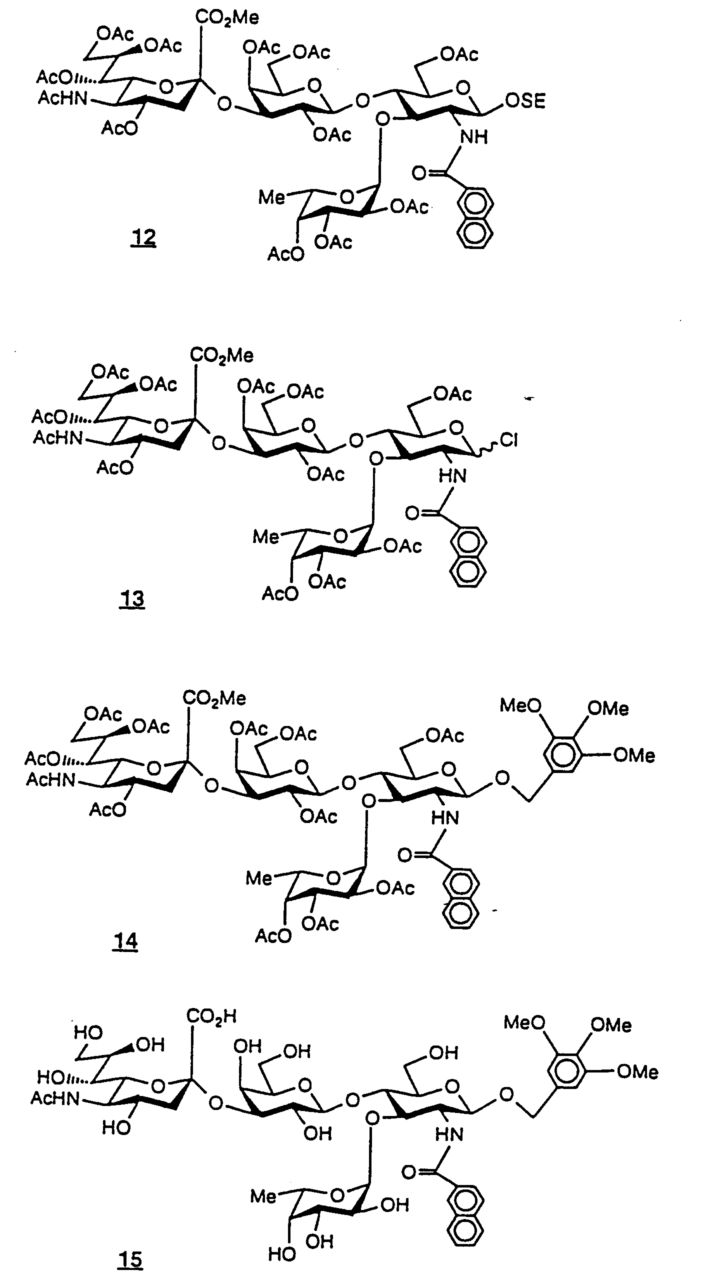

### [Example 1-1]

### Synthesis of 2-(trimethylsilyl)ethyl β-D-galactopyranosyl-(1→4)-O-(2-N-allyloxycarbonyl-2-amino-2-deoxy-β-D-glucopyranoside) (2).

To a solution of 2-(trimethylsilyl)ethyl 2-N-allyloxycarbonyl-2-amino-2-deoxy-β-D-glucopyranoside (1) (2.18 g, 6.00 mmol) in 50 mM sodium arsenate buffer (120 ml) were added uridine 5'-diphosphoglucose (UDP-Glc) (4.56g, 8.05 mmol), 5% bovine serum albumin (5%-BSA) (2.46 ml),aqueous 0.35M manganese (II) chloride solution (780 µl), aqueous 0.5N sodium azide solution (1.68 ml), alkaline phosphatase (1320 U), uridine 5'-diphosphogalactose 4-epimerase (UDP-Gal epimerase) (240 U), and 1,4-galactosyl transferase (GT) (30 U) successively, and the mixture was kept standing at 37°C. After 4 days, GT (10 U) was added thereto and the mixture was kept standing for further 4 days. After confirming the completion of reaction, a precipitate was removed by filtration, the filtrate was concentrated, and the resulting residue was purified by chromatography over polyacrylamide gel to give objective Compound 2 (3.01 g, Yield: 98%) as white solid. Rf = 0.38 (developed with a 10:2:1 mixture of ethyl acetate, ethanol and water).
¹H NMR (D₂O): δ = 5.96 (1H, m), 5.33 (1H, d, J=17.1Hz), 5.25 (1H,d, J=10.5 Hz), 4.74-4.50 (3H, m), 4.47 (1H, d, J=7.6Hz), 4.06-3.40 (14H,m), 1.08-0.93 (2H, m, OCH₂CH₂SiMe₃), and 0.00 (9H, s, OCH₂SiMe₃).

### [Example 1-2]

### Synthesis of 2-(trimethylsilyl)ethyl (5-acetamido-3,5-dideoxy-α-D-glycero-D-galacto-2-nonulopyranosylonic acid)-(2→3)-O-(β-D-galactopyranosyl)-(1→4)-O-(2-N-allyloxycarbonyl-2-amino-2-deoxy-β-D-glucopyranoside) (3).

To 200 mM N-(2-hydroxyethyl)piperazine-N'-2'-ethanesulfonic acid (HEPES) buffer (pH 7.5, 820 ml) were added cytidine-5'-monophosphate (CMP) (656 mg, 2.03 mmol), adenosine-5'-triphosphate (ATP)(112 mg, 0.203 mmol), phospho(enol)pyruvate (PEP·3Na) (2.03 g, 9.83 mmol), aqueous 1M-magnesium chloride solution (20.3 ml), aqueous 1M-manganese (II) chloride solution (5.38 ml), aqueous 1M-potassium chloride solution (20.3 ml), myokinase (32587 U) and pyruvate kinase (52956 U), and the mixture was stirred at room temperature for 1 hour.

After confirming the formation of cytidine 5'-triphosphate (CTP) by TLC, neuraminic acid (6.30 g, 20.3 mmol), 5%-bovine serum albumin (5%-BSA) (16.3 ml), inorganic pyrophosphatase (PPase) (2444 U), 2-mercaptoethanol (64 µl) and CMP-neuramic acid synthetase (62 U) were added to the medium, and the mixture was kept at room temperature for 1 hour.

After confirming production of CMP-neuraminic acid by TLC, PEP·3Na (9.28 g, 397 mmol), 2,3-sialyl transferase (62 U) and 2-(trimethylsilyl)ethyl β-D-galactopyranosyl-(1→4)-O-(2-N-allyloxycarbonyl-2-amino-2-deoxy-β-D-glucopyranoside) (2) (8.30 g, 15.8 mmol) were added to the medium, and the mixture was kept at room temperature for 5 days.

After confirming completion of the reaction, the reaction mixture was diluted with methanol and concentrated to give a residue containing objective Compound 3 (theoretical amount: 12.88 g). This was used in the next reaction without further purification. Rf = 0.49 (developed with a 4:2:1 mixture of ethyl acetate, ethanol and water).
¹H NMR (D₂O): δ = 5.96 (1H, m), 5.34 (1H, dd, J=1.3 and 17.1Hz), 5.25 (1H, dd, J=1.3 and 10.5Hz), 4.60-4.51 (4H, m), 4.14-3.37 (21H, m), 2.75 (1H, dd, J=4.6 and 12.5Hz, H-3e of NeuAc), 2.03 (3H, s, Ac), 1.80 (1H, t, J=12.2Hz, H-3a of NeuAc), 1.05-0.82 (2H, m, OCH₂CH₂SiMe₃), and 0.00 (9H, s, OCH₂CH₂SiMe₃).

### [Example 1-3]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(2-N-allyloxycarbonyl-2-amino-2-deoxy-3,6-di-O-acetyl- β-D-glucopyranoside) (4).

To a solution of the residue obtained in Example 1-2 containing 2-(trimethylsilyl)ethyl (5-acetamido-3,5-dideoxy-α-D-glycero-D-galacto-2-nonulopyranosylonic acid)-(2→3)-O-(β-D-galactopyranosyl)-(1→4)-O-(2-N-allyloxycarbonyl-2-amino-2-deoxy-β- D-glucopyranoside) (3) (theoretical amount: 12.88 g) in pyridine (377 ml) were added acetic anhydride (227 ml) and dimethylaminopyridine (500 mg) under ice cooling, and the mixture was stirred at room temperature for 12 hours. After confirming consumption of the starting material by TLC, methanol (580 ml) was added to the ice cold solution and the mixture was stirred at room temperature for 24 hours. Then the reaction mixture was concentrated, the resulting residue was redissolved in pyridine (240 ml), acetic anhydride (150 ml) was added thereto under ice cooling, and the mixture was stirred at room temperature for 3 hours. After confirming completion of the reaction, methanol (500 ml) was added to ice-cold reaction mixture, stirred at room temperature for 30 minutes, and concentrated. The resulting residue was dissolved in ethyl acetate and washed with aqueous copper(II) sulfate, aqueous sodium bicarbonate, and brine, successively. The organic layer was dried over sodium sulfate, filtered, the filtrate was concentrated, and the residue was purified by column chromatography over silica gel to give objective Compound 4 as a colorless amorphous (16.77 g, Yield: 88% from Compound 2 in 2 steps). Rf = 0.40 (developed with a 10:10:3 mixture of hexane, ethyl acetate and ethanol). mp. > 200 °C (dec.).
¹H NMR(CDCl₃): δ = 5.86 (1H, m), 5.55-3.50 (29H, m), 3.85 (3H, s,CO₂Me), 2.59 (1H, dd, J=4.3 and 12.5 Hz, H-3e of NeuAc), 2.24 (3H, s, OAc), 2.15 (3H, s, OAc), 2.10 (3H, s, OAc), 2.08 (3H, s, OAc), 2.07 (3H,s, OAc), 2.06 (3H, s, OAc), 2.05 (3H, s, OAc), 2.04 (3H, s, OAc), 2.00 (3H, s, OAc), 1.85 (3H, s, NAc), 1.68 (1H, t, J=12.5Hz, H-3a of NeuAc), 0.98-091 (2H, m, OCH₂CH₂SiMe₃), and -0.01 (9H, s, OCH₂CH₂SiMe₃).

| | | | |
|---|---|---|---|
| Anal. Calcd for C₅₁H₇₆O₂₉N₂Si: | C, 50.66; | H, 6.33; | N, 2.32. |
| Found: | C, 50.30; | H, 6.32; | N, 2.29. |

### [Example 1-4]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(2-amino-2-deoxy-3,6-d i-O-acetyl-β-D-glucopyranoside) (5).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(2-N-allyloxycarbonyl-2-amino-2-deoxy-3,6-di-O-acetyl-β- D-glucopyranoside) (4) (16.5 g, 13.7 mmol) in tetrahydrofuran (165 ml) were added tetrakis(triphenylphosphine) palladium (3.30 g) and polymethylhydrosiloxane (1.60 ml) at room temperature, and the mixture was stirred. After 2.5 hours, additional tetrakis(triphenylphosphine) palladium (3.30 g) and polymethylhydrosiloxane (1.60 ml) were added to the reaction mixture, and the mixture was stirred for 12 hours. After confirming the completion of reaction, the reaction mixture was diluted with dichloromethane and washed with water. The organic layer was dried over magnesium sulfate, filtered, and the filtrate was concentrated. The resulting residue was purified by column chromatography over silica gel to give objective Compound 5 as a pale yellow amorphous (13.61 g, Yield: 89%). Rf = 0.19 (developed with a 10:10:3 mixture of hexane, ethyl acetate and ethanol).
¹H NMR (CDCl₃): δ = 5.50 (1H, m), 5.38 (1H, m), 5.11-4.85 (4H, m), 4.65-3.45 (19H, m), 3.83 (3H, s, CO₂Me), 2.76 (1H, t, J=8.9Hz, H-2 of GlcN), 2.60 (1H, dd, J=4.6 and 12.9 Hz, H-3e of NeuAc), 2.26 (3H, s, OAc), 2.15 (3H, s, OAc), 2.10 (3H, s, OAc), 2.08 (3H, s, OAc), 2.07 (3H,s, OAc), 2.06 (3H, s, OAc), 2.05 (3H, s, OAc), 2.04 (3H, s, OAc), 1.99 (3H, s, OAc), 1.84 (3H, s, NAc), 1.70 (1H, m, H-3a of NeuAc), 1.05-0.93 (2H, m, OCH₂CH₂SiMe₃), and 0.01 (9H, s, OCH₂CH₂SiMe₃).

| | | | |
|---|---|---|---|
| Anal. Calcd. for C₄₇H₇₂O₂₇N₂Si·2H₂O: | C, 48.62; | H, 6.60; | N, 2.41. |
| Found: | C, 48.85; | H, 6.49; | N, 2.45. |

### [Example 1-5]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(6-O-acetyl-2-amino-2-deoxy-β- D-glucopyranoside) (6).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(2-amino-2-deoxy-3,6-di-O-acetyl-β-D-glucopyranoside) (5) (13.61 g, 12.1 mmol) in a mixture of methanol (1089 ml) and water (272 ml) was added acetic acid (0.72 ml), and the mixture was stirred at 50°C for 4 days. After confirming the completion of reaction, the reaction mixture was concentrated to give a residue containing the objective compound 6 (theoretical amount: 13.10 g). This was used in the next reaction without further purification. Rf = 0.76 (developed with a 9:1 mixture of chloroform and methanol).
¹H NMR (CDCl₃): δ = 5.50 (1H, m), 5.40 (1H, m), 5.12-4.85 (4H, m), 4.65-3.48 (20H, m), 3.83 (3H, s, CO₂Me), 2.76 (1H, t, J=8.7Hz, H-2 of GlcN), 2.56 (1H, dd, J= 4.3 and 12.5Hz, H-3e of NeuAc), 2.27 (3H, s, OAc), 2.15 (3H,s, OAc), 2.10 (3H, s, OAc), 2.08 (3H, s, OAc), 2.07 (3H, s, OAc), 2.06 (3H, s, OAc), 2.05 (3H, s, OAc), 2.00 (3H, s, OAc), 1.85 (3H, s, NAc), 1.67 (1H, t, J=12.5 Hz, H-3a of NeuAc), 1.02-0.92 (2H, m, OCH₂CH₂SiMe₃), and 0.01 (9H, s, OCH₂CH₂SiMe₃).

| | | | |
|---|---|---|---|
| Anal. Calcd for C₄₅H₇₀O₂₆N₂Si·H₂O: | C, 49.08; | H, 6.59; | N, 2.54. |
| Found: | C, 48.75; | H, 6.56; | N, 2.60. |

### [Example 1-6]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(6-O-acetyl-2-amino-2-N-benzyloxycarbonyl-2-deoxy- β-D-glucopyranoside) (7).

To a solution of the residue as prepared in Example 1-5 containing 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(6-O-acetyl-2-amino-2-deoxy-β-D-glucopyranoside) (6) (theoretical amount: 13.10 g) in dichloromethane (262 ml) at room temperature was added sodium bicarbonate (3.05 g, 36.3 mmol), then benzyloxycarbonyl chloride (2.60 ml, 18.2 mmol) was added dropwise to the solution, and the mixture was stirred for 12 hours. After confirming the completion of reaction, the reaction mixture was diluted with ethyl acetate and washed with water. The organic layer was dried over magnesium sulfate, filtered, and the filtrate was concentrated. The resulting residue was purified by column chromatography over silica gel to give objective Compound 7 (11.30 g, Yield: 77% from Compound 5 in 2 steps) as white solid. Rf = 0.71 (developed with a 9:1 mixture of chloroform and methanol). mp. 115-120°C.
¹H NMR (CDCl₃): δ = 7.34-7.27 (5H, m, Ph-H), 5.51 (1H, m), 5.41 (1H, m), 3.82 (3H, s, CO₂Me), 2.58 (1H, dd, J=4.6 and 12.5Hz, H-3e of NeuAc), 2.28 (3H, s, OAc), 2.17 (3H, s, OAc), 2.11 (3H, s, OAc), 2.10 (3H, s, OAc), 2.09 (3H, s, OAc), 2.07 (3H, s, OAc), 2.02 (3H, s, OAc), 2.01 (3H, s, OAc ), 1.86 (3H, s, NAc), 1.66 (1H, t, J=12.5 Hz, H-3a of NeuAc), 0.95-0.82 (2H, m, OCH₂CH₂SiMe₃), and -0.01 (9H, s, OCH₂CH₂SiMe₃).

| | | | |
|---|---|---|---|
| Anal. Calcd. for C₅₃H₇₆O₂₈N₂Si·H₂O: | C, 51.53; | H, 6.36; | N, 2.27. |
| Found: | C, 51.56; | H, 6.38; | N, 2.26. |

### [Example 1-7]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-benzyl-α-L-fucopyranosyl-(1→3)-O]-(6-O-acetyl-2-amino-2-N-benzyloxycarbonyl-2-deoxy-β-D-glucopyranoside) (9).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-(6-O-acetyl-2-amino-2-N-benzyloxycarbonyl-2-deoxy-β-D-glucopyranoside) (7) (5.60 g, 4.60 mmol) in dichloroethane (25 ml) were added Molecular Sieves 4A (2.6 g), tetramethylurea (3.30 ml, 27.6 mmol) and 2,3,4-tri-O-benzyl-L-fucopyranosyl fluoride (8) (12.0 g, 27.5 mmol). After stirring for 90 minutes at room temperature, the reaction vessel was protected from light, cooled to -20°C, and mixed with tin(II) chloride (3.49 g, 18.4 mmol) and silver perchlorate (3.85 g, 18.4 mmol). The reaction mixture was warmed to room temperature over 90 minutes and stirred for 24 hours. After confirming the completion of reaction, the reaction mixture was filtered through Celite, and the filtrate was washed with water, the organic layer was dried over sodium sulfate, and concentrated. The residue was purified by column chromatography over silica gel to give objective Compound 9 as a pale yellow amorphous (6.37 g, Yield: 85%). Rf = 0.41 (developed with a 10:10:3 mixture of hexane, ethyl acetate and methanol). mp. 102-108°C.
¹H NMR(CDCl₃): δ = 7.46-7.24 (20H, m, Ph-H), 5.43 (1H, d, J=9.5Hz), 5.46 (1H, d, J=9.5Hz), 5.20-3.50 (36H, m), 3.94 (3H, s, CO₂Me), 2.60 (1H, dd, J=4.6 and 12.5Hz, H-3e of NeuAc), 2.24 (3H, s, OAc) , 2.18 (3H,s, OAc), 2.10 (3H, s, OAc), 2.08 (3H, s, OAc),2.07 (3H, s, OAc), 2.04 (6H, s, OAc x 2), 1.88 (3H, s, OAc), 1.84 (3H, s, NAc), 1.70 (1H, t, J=12.5 Hz, H-3a of NeuAc), 1.26 (3H, d,J=6.3 Hz, Me of Fuc), 0.94-0.84 (2H, m, OCH₂CH₂SiMe₃), and 0.00 (9H, s, OCH₂CH₂SiMe₃).

| | | | |
|---|---|---|---|
| Anal. Calcd for C₈₀H₁₀₄O₃₂N₂Si·3H₂O: | C, 56.93; | H, 6.57; | N, 1.66. |
| Found: | C, 56.72; | H, 6.22; | N, 1.71. |

### [Example 1-8]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fucopyranosyl-(1→ 3)-O]-(6-O-acetyl-2-amino-2-deoxy-β-D-glucopyranoside) (10).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-benzyl-α- L-fucopyranosyl-(1→3)-O]-(6-O-acetyl-2-amino-2-N-benzyloxycarbonyl-2-deoxy-β-D-glucopyranoside) (9) (6.25 g, 3.83 mmol) in ethanol (125 ml) were added ammonium formate (10 g) and 10% Pd-C (wet, 10 g), and the mixture was refluxed for 8 hours. Then additional ammonium formate (10 g) and 10% Pd-C (wet, 10 g) were added and refluxed for 8 hours. After confirming the completion of reaction, the reaction mixture was filtered through Celite and the filtrate was concentrated to give objective Compound 10 as a colorless amorphous (4.45 g, Yield: 95%). Rf = 0.44 (developed with a 10:2:1 mixture of ethyl acetate, ethanol and water). mp. 149-152°C.
¹H NMR (CDCl₃): δ = 5.48-5.40 (2H, m), 5.34 (1H, d, J=3.3Hz), 5.13 (1H, d, J=10.6Hz), 4.96-4.84 (3H, m), 4.68-4.53 (4H, m), 4.35-3.50 (22H,m), 3.83 (3H, s, CO₂Me), 2.75 (1H, t, J=9.4Hz, H-2 of GlcN), 2.57 (1H, dd, J=4.6 and 12.5 Hz, H-3e of NeuAc), 2.20 (3H, s, OAc), 2.15 (3H, s, OAc), 2.12 (3H, s, OAc), 2.09 (3H, s, OAc), 2.06 (9H, s, OAc x 3), 1.99 (3H, s, OAc), 1.84 (3H, s, NAc), 1.67 (1H, t, J=12.5 Hz, H-3a of NeuAc),1.33 (3H, d, J=6.6 Hz, Me of Fuc), 0.99-0.91 (2H, m, OCH₂CH₂SiMe₃), and 0.01 (9H, s, OCH₂CH₂SiMe₃).
¹³C NMR (CDCl₃): δ = 170.9, 170.8, 170.6, 170.5, 170.4, 170.1, 169.4, 169.3, 167.9, 103.1, 100.0, 99.6, 96.8, 79.0, 75.2, 73.3, 72.3, 72.0, 71.3, 71.0, 70.9, 69.7, 69.5, 69.4, 68.0, 67.7, 67.4, 66.6, 66.0, 62.3, 61.7, 61.6, 58.9, 53.2, 49.1, 37.4, 23.1, 21.5, 20.9, 20.8, 20.8, 20.7, 20.7, 20.6, 18.1, 16.2, and -1.4

| | | | |
|---|---|---|---|
| Anal. Calcd for C₅₁H₈₀O₃₀N₂Si·3H₂O: | C, 47.73; | H, 6.75; | N, 2.18. |
| Found: | C, 47.92; | H, 6.66; | N, 2.41. |

### [Example 1-9]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fucopyranosyl-(1→ 3)-O]-(6-O-acetyl-2-deoxy-2-naphthamido-β-D-glucopyranoside ) (11).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fucopyranosyl-(1→ 3)-O]-(6-O-acetyl-2-amino-2-deoxy-β-D-glucopyranoside) (10) (2.45 g, 1.99 mmol) in dichloromethane (50 ml) were added sodium bicarbonate (670 mg, 7.98 mmol) and β-naphthoyl chloride (760 mg, 3.99 mmol), and the mixture was stirred for 12 hours. After confirming the completion of reaction, methanol (10 ml) and pyridine (5.0 ml) were added to the ice-cooled reaction mixture, and the mixture was stirred at room temperature for 15 minutes. The reaction mixture was concentrated to give a residue containing objective Compound 11 (theoretical amount: 2.76 g). This was used in the next reaction without further purification.

### [Example 1-10]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (12).

To a solution of the residue prepared in Example 1-9 containing 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fucopyranosyl-(1→ 3)-O]-(6-O-acetyl-2-deoxy-2-naphthamido-β-D-glucopyranoside ) (11) (theoretical amount: 2.76 g) in pyridine (25 ml) were added acetic anhydride (15 ml) and 4-dimethylaminopyridine (100 mg), and the mixture was stirred at room temperature for 8 hours. After confirming the completion of reaction, methanol (15 ml) was added to the reaction mixture under ice cooling, and the mixture was stirred at room temperature for 30 minutes. The reaction mixture was concentrated, the residue was diluted with ethyl acetate and washed with saturated aqueous copper(II) sulfate solution and then with brine. The organic layer was dried over sodium sulfate, filtered, and the filtrate was concentrated. The residue was purified by column chromatography over silica gel to give objective Compound 12 as a pale yellow amorphous (2.58 g, Yield: 86% from Compound 10 in 2 steps).
¹H NMR (CDCl₃): δ = 8.28 (1H, s, Napht-H), 7.92-7.82 (4H, m, Napht-H), 7.58- 7.53 (2H, m, Napht-H), 6.37 (1H, d, J=9.6Hz, NH), 5.60-4.65 (14H,m), 4.56 (1H, dd, J=4.0 and 9.9Hz), 4.45-3.80 (11H, m), 3.86 (3H, s, CO₂Me), 3.70-3.45 (3H, m), 2.59 (1H, dd, J=4.6 and 12.5Hz, H-3e of Neu Ac), 2.23 (3H, s, OAc), 2.17 (3H, s, OAc), 2.11 (3H, s, OAc), 2.08 (9H, s, OAc z 3), 2.07 (9H, s, OAc x 3), 2.00 (3H, s, OAc), 1.93 (3H, s, OAc), 1.85 (3H, s, NAc), 1.70 (1H, t, J=12.5Hz, H-3a of NeuAc), 1.18 (3H, d, J=6.6Hz, Me of Fuc), 0.90-0.82 (2H, m OCH₂CH₂SiMe₃), and -0.08 (9H, s, OCH₂CH₂SiMe₃).

### [Example 1-11]

### Synthesis of [methyl (5-acetamido-3,5-dideoxy-4,7,8,9-tetra-O-acetyl-α-D-glycero-D-galacto-2-nonulopyrano sylonate)]-(2→3)-O-(2,4,6-tri-O-acetyl-β-D-galactopyranosyl )-(1→4)-O-[2,3,4-tri-O-acetyl-α-L-fucopyranosyl-(1→3)-O]-(6 -O-acetyl-2-deoxy-2-naphthamido-β-D- glucopyranosyl) chloride (13).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (12) (182.8 mg, 0.121 mmol) in chloroform (25 ml) were added dichloromethyl methyl ether (55 µl, 0.608 mmol) and zinc chloride (4.2 mg, 3.08 × 10⁻² mmol), and the mixture was stirred for 6 hours. After confirming the completion of reaction, the reaction mixture was concentrated to give a residue containing the objective Compound 13 (theoretical amount: 172.9 mg). This was used in the next reaction without further purification.

### [Example 1-12]

### Synthesis of 3,4,5-trimethoxybenzyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (14).

To a solution of the residue prepared in Example 1-11 containing [methyl (5-acetamido-3,5-dideoxy-4,7,8,9-tetra-O-acetyl-α-D-glycero-D-galacto-2-nonulopyranosylonate )]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acety 1-2-deoxy-2-naphthamido-β-D-glucopyranosyl) chloride (13) (theoretical amount: 75.7 mg) in dichloromethane (5.0 ml) were added Molecular Sieves 4A (200 mg), tin(II) trifluoromethanesulphonate (33.1 mg, 0.078 mmol). To this mixture was added dropwise a solution of 3,4,5-trimethoxybenzyl alcohol (21.0 mg, 0.106 mmol) and tetramethyl urea (9.5 µl, 0.078 mmol) in dichloromethane (3.0 ml), and the mixture was stirred for 40 hours. After confirming the completion of reaction, the reaction mixture was filtered through a pad of Celite and the filtrate was washed with saturated aqueous sodium bicarbonate and brine successively. The organic layer was dried over magnesium sulfate and filtered, and the filtrate was concentrated. The residue was purified by column chromatography over silica gel to give objective Compound 14 as a colorless amorphous (68.0 mg, Yield: 81% from Compound 12 in 2 steps).
¹H NMR (CDCl₃): δ = 8.25 (1H, s, Napht-H), 7.98-7.72 (4H, m, Napht-H), 7.63-7.50 (2H, m, Napht-H), 6.39 (2H, s, Ph-H), 6.35 (1H, d, J=9.6 Hz, NH), 5.60-3.35 (29H, m), 3.87 (3H, s, CO₂Me), 3.74 (3H, s, OMe), 3.47 (6H, s, OMe x 2), 2.60 (1H, dd, J=4.5 and 12.5 Hz, H-3e of NeuAc), 2.23 (3H, s, OAc), 2.18 (3H, s, OAc), 2.15 (3H, s, OAc), 2.09 (6H, s, OAc x 2), 2.08 (9H, s, OAc x 3), 2.05 (3H, s, OAc ), 2.01 (3H, s, OAc), 1.93 (3H, s, OAc), 1.86 (3H, s, NAc), 1.70 (1H, t, J=12.5Hz, H-3a of NeuAc), and 1.18 (3H, d, J=6.6 Hz, Me of Fuc).

### [Example 1-13]

### Synthesis of 3,4,5-trimethoxybenzyl (5-acetamido-3,5-dideoxy-α-D-glycero-D-galacto-2-nonulopyra nosylonic acid)-(2→3)-O-(β-D-galacto-pyranosyl)-(1→4)-O-[α-L-fucopyranosyl-(1→3)-O]-(2-deoxy-2-naphthamido-β-D-glucopyranoside) (15).

To a solution of 3,4,5-trimethoxybenzyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (14) (68.0 mg, 4.28 × 10⁻²mmol) in methanol (5.0 ml) was added 28% sodium methoxide solution (in methanol, 0.50 ml), and the mixture was kept at room temperature. After 14 hours, water (5.0 ml) was added to the mixture, and the mixture was left to stand for further 12 hours. After confirming the completion of reaction, the reaction mixture was neutralized with an acid type ion exchange resin (DOWEX 50W-X8), filtered, and the filtrate was concentrated. The resulting residue was purified by column chromatography using polyacrylamide gel and the eluate was lyophilized to give objective Compound 15 (36.6 mg, Yield: 77%) as white powder.
¹H NMR (D₂O): δ = 7.97 (1H, s, Napht-H), 7.92-7.72 (4H, m, Napht-H), 7.56-7.47 (2H, m, Napht-H), 6.44 (2H, s, Ph-H), 5.02 (1H, d, J=4.0Hz, 1-H of Fuc), 4.51-4.43 (2H, m, 1-H of GlcN and 1-H of Gal), 4.20-3.40 (25H, m), 3.33 (6H, s, OMe x 2), 3.26 (3H, s, OMe), 2.68 (1H, dd, J=4.5 and 12.5Hz, H-3e of NeuAc), 1.95 (3H, s, NAc), 1.73 (1H, t, J=12.5 Hz, H-3a of NeuAc), and 1.07 (3H, d, J= 6.3Hz, Me of Fuc).

### [Example 2]

According to the methods similar to those of Example 1, Compound 13 was treated with various alcohols to give the following glycosidated Products (16) to (30).

The structural formulae of Compounds (16) to (30) are as follows:

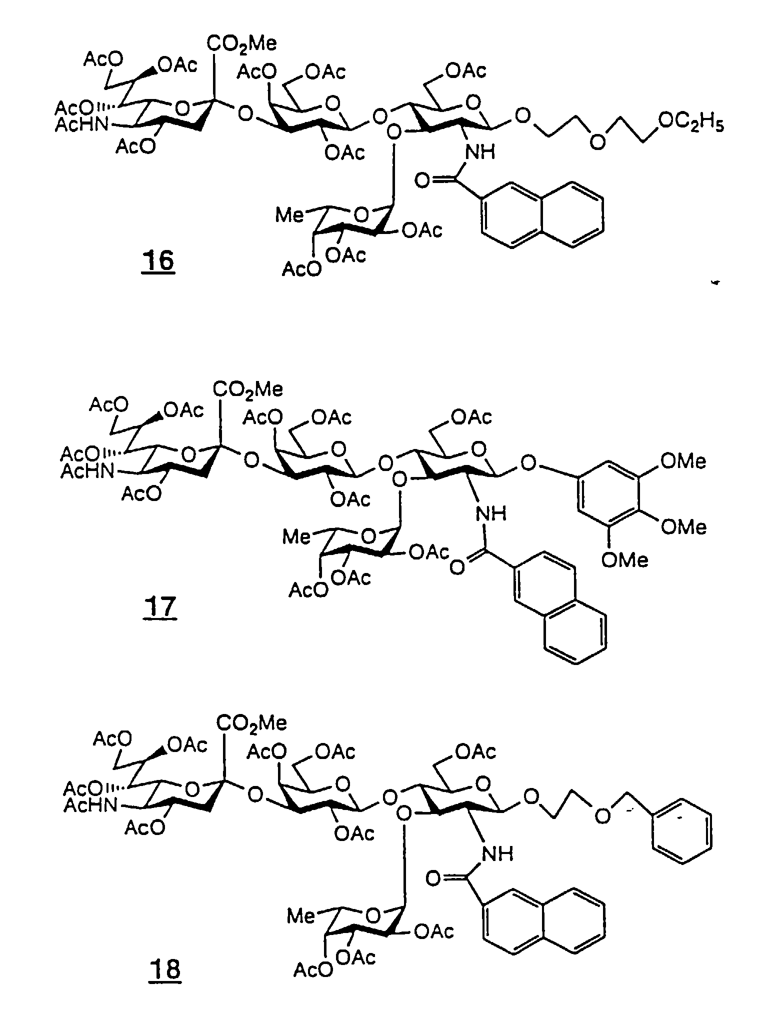

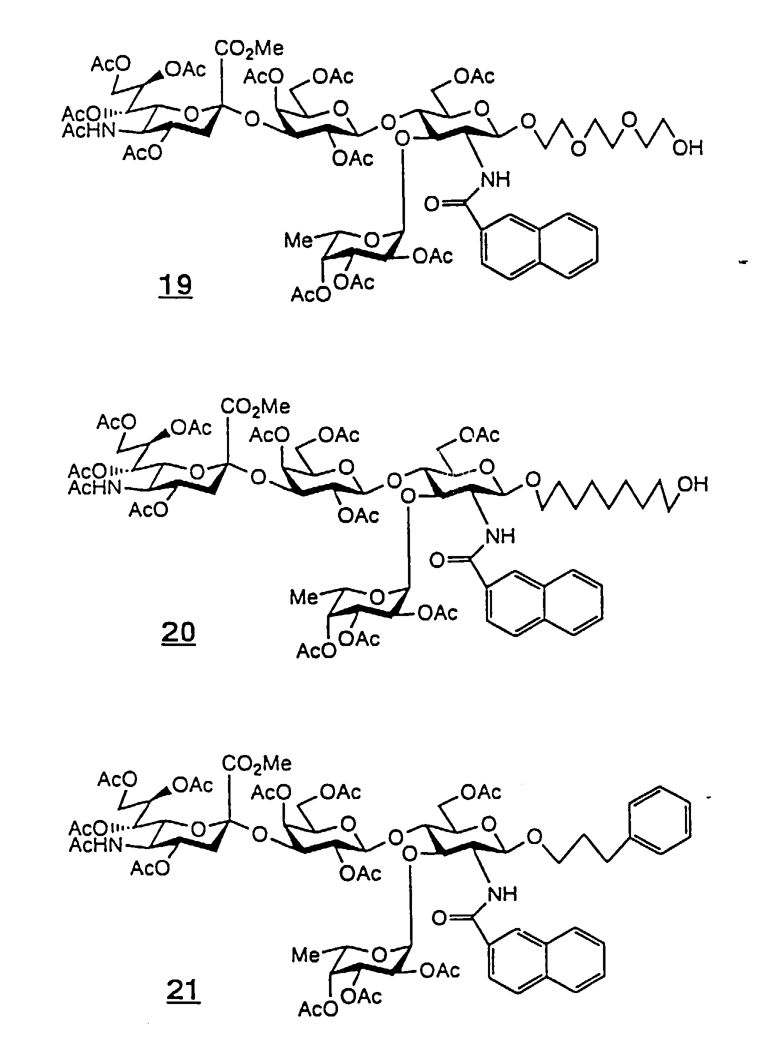

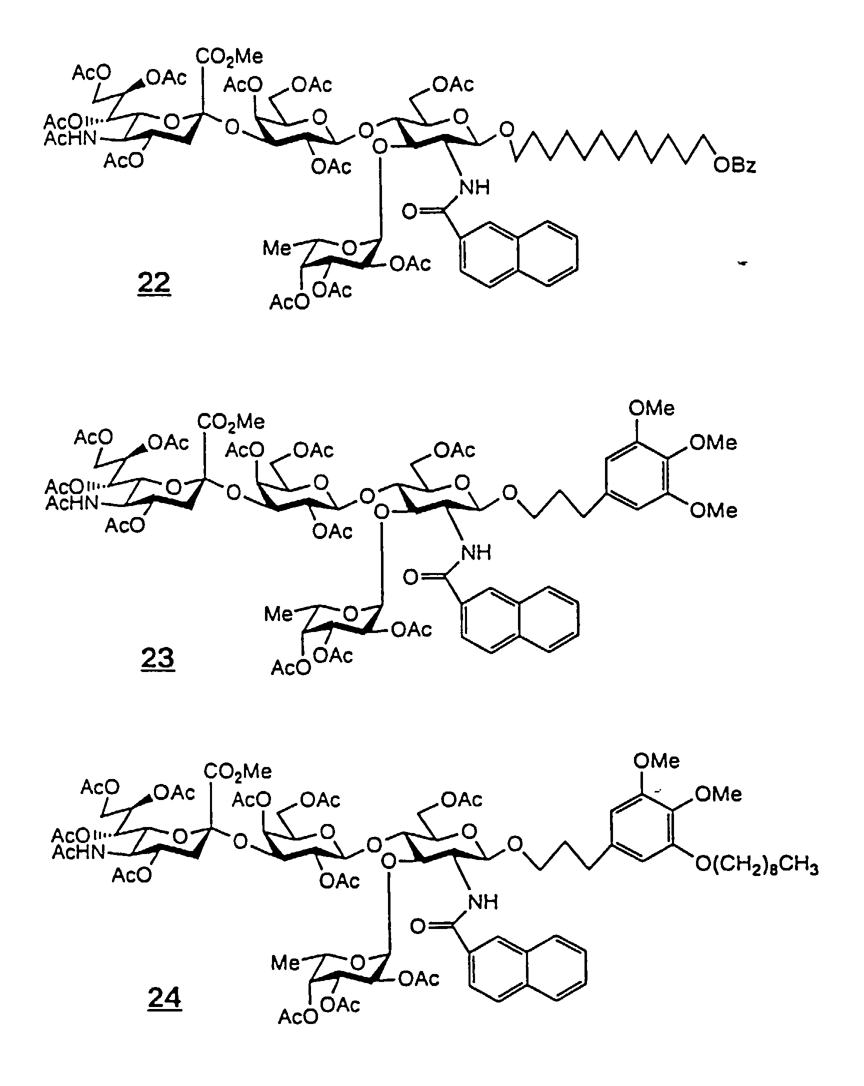

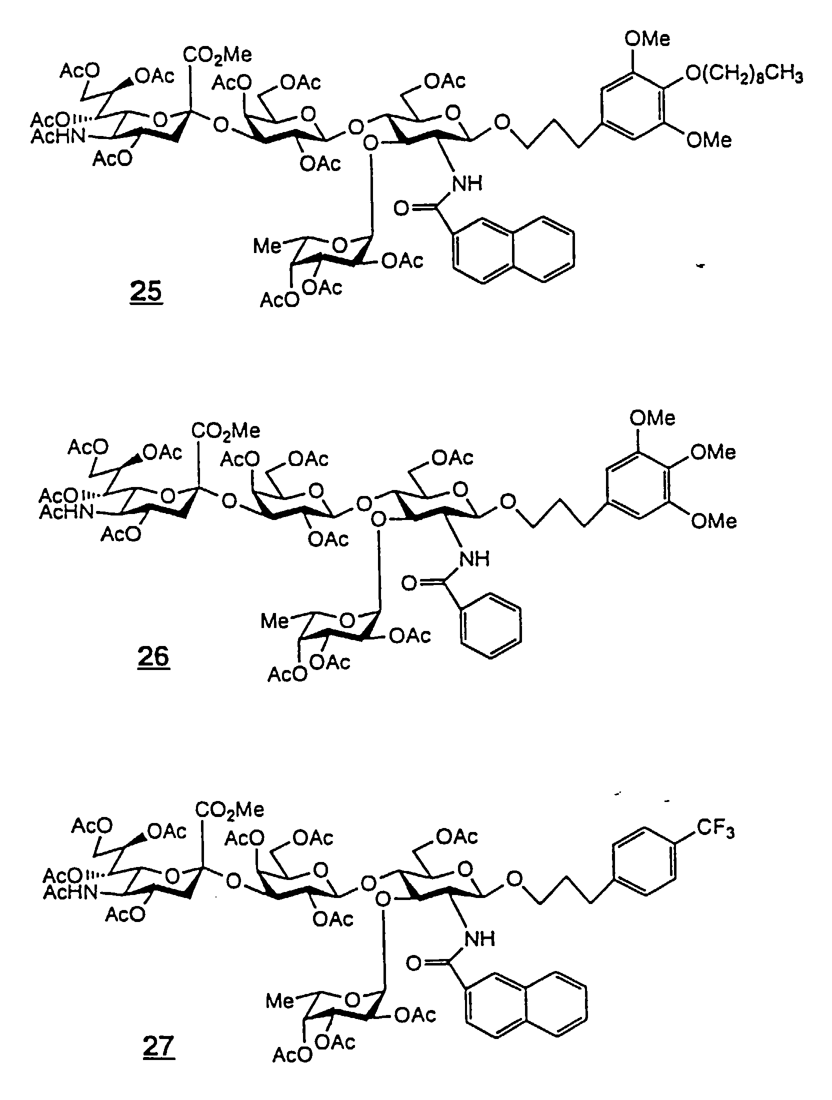

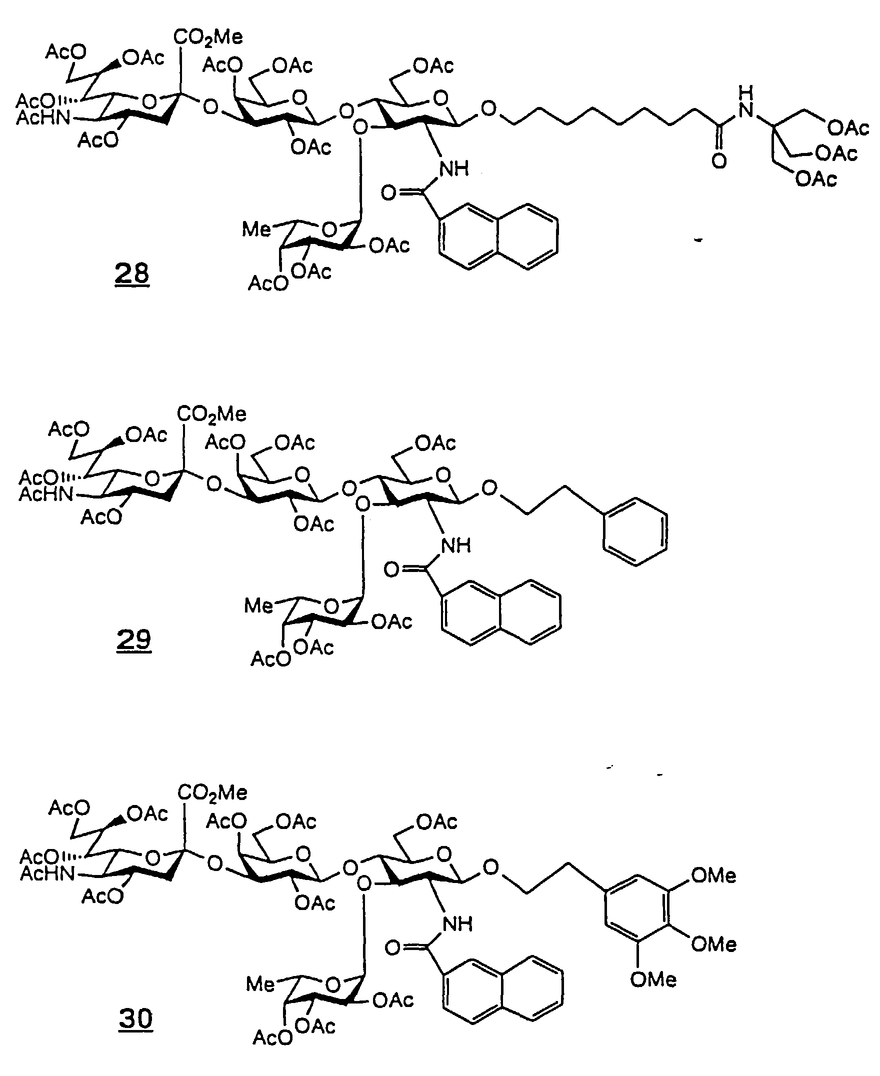

### 2-(2-Ethoxyethoxy)ethyl [methyl (5-acetamido-3,5-dideoxy-4,7,8,9-tetra-O-acetyl-α-D-glycero-D-galacto-2-nonu lopyranosylonate)]-(2→3)-O-(2,4,6-tri-O-acetyl-β-D-galacto pyranosyl)-(1→4)-O-[2,3,4-tri-O-acetyl-α-L-fucopyranosyl-(1 →3)-O]-(6-O-acetyl-2-deoxy-2-naphthamido-β-D-glucopyranosid e) (16).

¹H-NMR (270MHz, CDCl₃): δ = 8.36 (1H, s, Napht-H), 7.95-7.80 (4H,m, Napht-H), 7.60-7.50 (2H, m, Napht-H), 7.00 (1H, d, J=9.5 Hz, NHNapht), 5.60-3.25 (37H, m), 3.86 (3H, s, CO₂Me), 2.60 (1H, dd, J=4.5 and 12.5 Hz, H-3e of NeuAc), 2.23 (3H, s, OAc), 2.18 (3H, s, OAc), 2.12 (3H, s, OAc), 2.11(3H, s, OAc), 2.10 (3H, s, OAc), 2.09 (3H, s, OAc), 2.07 (3H, s, OAc), 2.05 (3H, s, OAc), 2.02 (3H, s, OAc), 2.01 (3H, s, OAc), 1.91 (3H, s, OAc), 1.86 (3H, s, NAc), 1.70 (1H, m, H-3a of NeuAc), 1.23 (3H, t, J=6.9 Hz, OCH₂CH₃), and 1.21(3H, d, J=6.6 Hz, Me of Fuc).

### 3,4,5-Trimethoxyphenyl [methyl (5-acetamido-3,5-dideoxy-4,7,8,9-tetra-O-acetyl-α-D-glycero-D-galacto-2-nonu lopyranosylonate)]-(2→3)-O-(2,4,6-tri-O-acetyl-β-D-galactop yranosyl)-(1→4)-O-[2,3,4-tri-O-acetyl-α-L-fucopyranosyl-(1→ 3)-O]-(6-O-acetyl-2-deoxy-2-naphthamido-β-D-glucopyranoside ) (17).

¹H-NMR (270MHz, CDCl₃): δ = 8.33 (1H, s, Napht-H), 8.00-7.80 (4H,m, Napht-H), 7.65-7.50 (2H, m, Napht-H), 6.67 (1H, d, J=9.5 Hz, NHNapht), 6.24 (2H, s, Ph-H), 5.65-3.60 (27H, m), 3.87 (3H, s, CO₂Me), 3.74 (3H, s, OMe), 3.71 (6H, s, OMe x 2), 2.62 (1H, dd, J=4.5 and 12.5 Hz, H-3e of NeuAc), 2.24 (3H, s, OAc), 2.18 (3H, s, OAc), 2.12 (3H, s, OAc), 2.11 (3H, s, OAc), 2.10 (3H, s, OAc), 2.09 (3H, s, OAc), 2.08 (3H,s, OAc), 2.07 (3H, s, OAc), 2.06 (3H, s, OAc), 2.02 (3H, s, OAc), 1.95 (3H, s, OAc), 1.86 (3H, s, NAc), 1.70 (1H, m, H-3a of NeuAc), and 1.19 (3H, d, J=6.6 Hz, Me of Fuc).

### 2-Benzyloxyethyl [methyl (5-acetamido-3,5-dideoxy-4,7,8,9-tetra-O-acetyl-α-D-glycero-D-galacto-2-nonu lopyranosylonate)]-(2→3)-O-(2,4,6-tri-O-acetyl-β-D-galacto pyranosyl)-(1→4)-O-[2,3,4-tri-O-acetyl-α-L-fucopyranosyl-(1 →3)-O]-(6-O-acetyl-2-deoxy-2-naphthamido-β-D-glucopyranoside) (18).

¹H-NMR (270MHz, CDCl₃): δ = 8.25 (1H, s, Napht-H), 7.95-7.75 (4H,m, Napht-H), 7.60-7.50 (2H, m, Napht-H), 7.20-7.25 (3H, m, Ph-H), 7.10- 7.00 (2H, m, Ph-H), 6.43 (1H, d, J=9.2 Hz, NHNapht), 5.55-3.45 (33H, m), 3.87 (3H, s, CO₂Me), 2.60 (1H, dd, J=4.5 and 12.5 Hz, H-3e of NeuAc), 2.23 (3H, s, OAc), 2.17 (3H, s, OAc), 2.11 (3H, s, OAc), 2.10 (6H, s, OAc x 2), 2.09 (3H, s, OAc), 2.08 (3H, s, OAc), 2.07 (3H, s, OAc), 2.05 (3H, s, OAc), 2.00 (3H, s, OAc), 1.92 (3H, s, OAc), 1.86 (3H, s, NAc), 1.70 (1H, m, H-3a of NeuAc), and 1.20 (3H, d, J=6.6 Hz, Me of Fuc).

### 2-[2-(2-Hydroxyethoxy)ethoxy]ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (19).

¹H-NMR (270MHz, CDCl₃): δ = 8.48 (1H, s, Napht-H), 8.03-7.95 (2H,m, Napht-H), 7.92-7.83 (2H, m, Napht-H), 7.60-7.51 (2H, m, Napht-H), 7.46 (1H, d, J=8.9 Hz, NHNapht), 5.60-3.10 (39H, m), 3.87 (3H, s, CO₂Me), 2.60 (1H, dd, J=4.5 and 12.5Hz, H-3e of NeuAc), 2.23 (3H, s, OAc), 2.18 (3H, s, OAc), 2.13 (3H, s, OAc), 2.12 (3H, s, OAc), 2.11 (3H,s, OAc), 2.10(3H, s, OAc), 2.08 (6H, s, OAc x 2), 2.03 (3H, s, OAc), 2.01 (3H, s, OAc), 1.91 (3H, s, OAc), 1.86 (3H, s, NAc), 1.80 (1H, br., OH), 1.70 (1H, t, J=12.5Hz, H-3a of NeuAc), and 1.22 (3H, d, J=6.3 Hz, Me of Fuc).

### 9-Hydroxynonyl [methyl (5-acetamido-3,5-dideoxy-4,7,8,9-tetra-O-acetyl-α-D-glycero-D-galacto-2-nonulopyrano sylonate)]-(2→3)-O-(2,4,6-tri-O-acetyl-β-D-galactopyranosyl )-(1→4)-O-[2,3,4-tri-O-acetyl-α-L-fucopyranosyl-(1→3)-O]-(6 -O-acetyl-2-deoxy-2-naphthamido- β-D-glucopyranoside) (20).

¹H-NMR (270MHz, CDCl₃): δ = 8.28 (1H, s, Napht-H), 8.00-7.75 (4H,m, Napht-H), 7.60-7.50 (2H, m, Napht-H), 6.39 (1H, d, J=8.9Hz, NHNapht),5.55-3.40 (31H, m), 3.86 (3H, s, CO₂Me), 2.60 (1H, dd, J=4.5 and 12.5Hz, H-3e of NeuAc), 2.23 (3H, s, OAc), 2.17 (3H, s, OAc), 2.11 (3H, s, OAc), 2.09 (6H, s, OAc x 2), 2.08 (6H, s, OAc x 2), 2.07 (6H, s, OAc x 2), 2.01 (3H, s, OAc), 1.93 (3H, s, OAc), 1.86 (3H, s, NAc), 1.80-1.60 (6H, m, H-3a of NeuAc, OH and CH₂ x 2), 1.55-1.30 (4H, m, CH₂ x 2), 1.20 (3H, d, J=6.6 Hz, Me of Fuc), and 1.25-0.95 (6H, m, CH₂x3).5

### 3-Phenylpropyl [methyl (5-acetamido-3,5-dideoxy-4,7,8,9-tetra-O-acetyl-α-D-glycero-D-galacto-2-nonulopyrano sylonate)]-(2→3)-O-(2,4,6-tri-O-acetyl-β-D-galactopyranosyl )-(1→4)-O-[2,3,4-tri-O-acetyl-α-L-fucopyranosyl-(1→3)-O]-(6 -O-acetyl-2-deoxy-2-naphthamido-β-D-glucopyranoside) (21).

¹H-NMR (270MHz, CDCl₃): δ = 8.29 (1H, s, Napht-H), 7.97-7.80 (4H,m, Napht-H), 7.65-7.50 (2H, m, Napht-H), 7.12-7.08 (3H, m, Ph-H), 6.98- 6.93 (2H, m, Ph-H), 6.38 (1H, d, J=8.6 Hz, NHNapht), 5.60-3.40 (29H, m), 3.86 (3H, s, CO₂Me), 2.81-2.53 (3H, m, CH₂ and H-3e of NeuAc), 2.20-2.00 (2H, m, CH₂), 2.22 (3H, s, OAc), 2.14 (3H, s, OAc), 2.06 (6H, s, OAc), 2.05 (6H, s, OAc x 2), 2.04 (6H, s, OAc x 2), 2.03 (6H, s, OAc x 2), 1.99 (3H, OAc), 1.98 (3H, s, OAc), 1.89 (3H, s, NAc), 1.70 (1H, m, H-3a of NeuAc), and 1.20 (3H, d, J=6.3 Hz, Me of Fuc).

### 12-Benzoyloxydodecyl [methyl (5-acetamido-3,5-dideoxy-4,7,8,9-tetra-O-acetyl-α-D-glycero-D-galacto-2-nonu lopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (22).

¹H-NMR (270MHz, CDCl₃): δ = 8.27 (1H, s, Napht-H), 8.04 (2H, d, J=7.5Hz, Bz-H), 7.97-7.79 (4H, m, Napht-H), 7.59-7.52 (3H, m, Napht-H x 2 and Bz-H), 7.44 (2H, t, J=7.5 Hz, Bz-H), 6.67 (1H, d, J=8.6 Hz, NHNapht), 5.60-3.75 (27H, m), 3.86 (3H, s, CO₂Me), 3.64 (2H, dd, J=2.8 and 10.8 Hz, CH₂), 3.41 (2H, m, CH₂), 2.60 (1H, dd, J=4.5 and 12.5 Hz, H-3e of NeuAc), 2.23 (3H, s, OAc), 2.17 (3H, s, OAc), 2.11 (3H, s, OAc), 2.10 (3H, s, OAc), 2.09 (6H, s, OAc x 2), 2.08 (6H, s, OAc x 2), 2.07 (3H, s, OAc), 2.01 (3H, s, OAc), 1.93 (3H, s, OAc), 1.86 (3H, s, NAc), 1.75-1.55 (5H, m, H-3a of NeuAc and CH₂ x 2), 1.52-1.32 (4H, m, CH₂), and 1.30-0.90 (15H, m, Me of Fuc and CH₂ x 6).

### 3-(3,4,5-Trimethoxyphenyl)propyl [methyl (5-acetamido-3,5-dideoxy-4,7,8,9-tetra-O-acetyl-α-D-glyceroD-g alacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (23).

¹H-NMR (270MHz, CDCl₃): δ = 8.28 (1H, s, Napht-H), 7.93-7.79 (4H, m, Napht-H), 7.58-7.52 (2H, m, Napht-H), 6.35 (1H, d, J=7.9Hz, NHNapht), 6.20 (2H, s, Ph-H), 5.60-3.80 (29H, m), 3.86 (3H, s, CO₂Me), 3.76 (3H, s, OMe), 3.67 (6H, s, OMe x 2), 2.65-2.38 (3H, m, H-3e of NeuAc and CH₂), 2.23 (3H, s, OAc), 2.17 (3H, s, OAc), 2.11 (3H, s, OAc), 2.09 (6H, s, OAc x 2), 2.08 (6H, s, OAc x 2), 2.05 (3H, s, OAc), 2.04 (3H, s, OAc), 2.01 (3H, s, OAc), 1.93 (3H, s, OAc), 1.86 (3H, s, NAc), 2.00-1.80 (2H, m, CH₂), 1.70 (1H, t, J=12.8 Hz, H-3a of NeuAc), and 1.20 (3H, d, J=6.3 Hz, Me of Fuc).

### 3-(4,5-Dimethoxy-3-nonyloxypheny)propyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (24).

¹H-NMR (270MHz, CDCl₃): δ = 8.28 (1H, s, Napht-H), 7.93-7.79 (4H, m, Napht-H), 7.58-7.52 (2H, m, Napht-H), 6.32 (1H, d, J=8.5Hz, NHNapht),6.20 (1H, s, Ph-H), 6.18 (1H, s, Ph-H), 5.60- 3.60 (29H, m), 3.87 (3H, s, CO₂ Me), 3.76 (3H, s, OMe), 3.65 (3H, s, OMe), 3.55- 3.40 (2H, m, CH₂), 2.60 (1H, dd, J=4.1 and 12.7 Hz, H-3e of NeuAc), 2.55-2.40 (2H, m,CH₂), 2.23 (3H, s, OAc), 2.18 (3H, s, OAc), 2.11 (6H, s, OAc x 2), 2.10 (6H, s, OAc x 2), 2.08 (6H, s, OAc x 2), 2.07 (3H, s, OAc), 2.01(3H, s, OAc), 1.93 (3H, s, OAc), 1.86 (3H, s, NAc), 1.95-1.55 (5H, m, H-3e of NeuAc and CH₂ x 2), 1.45-1.15 (12H, m, CH₂ x 6), 1.19 (3H, d, J=6.3 Hz, Me of Fuc), and 0.88 (3H, t, J=6.6Hz, (CH₂)₈CH₃).

### 3-(3,5-Dimethoxy-4-nonyloxypheny)propyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (25).

¹H-NMR (270MHz, CDCl₃): δ = 8.28 (1H, s, Napht-H), 7.94-7.79 (4H,m, Napht-H), 7.59-7.52 (2H, m, Napht-H), 6.37 (1H, d, J=8.6Hz, NHNapht), 6.19 (2H, s, Ph-H), 5.60-3.80 (29H, m), 3.86 (3H, s, CO₂Me), 3.64 (6H, s, OMe x 2), 3.55-3.40 (2H, m, CH₂), 2.60 (1H, dd, J=4.6 and 12.2Hz, H-3e of NeuAc), 2.55-2.40 (2H, m, CH₂), 2.23 (3H, s, OAc), 2.18 (3H, s, OAc), 2.11 (3H, s, OAc), 2.10 (9H, s, OAc x 3), 2.08 (6H, s, OAc x 2), 2.07 (3H, s, OAc), 2.01 (3H, s, OAc), 1.94 (3H, s, OAc), 1.86 (3H, s, NAc), 1.95-1.55 (5H, m, H-3a of NeuAc and CH₂ x 2), 1.45-1.15 (12H, m, CH₂ x 6), 1.20 (3H, d, J=6.3 Hz, Me of Fuc), and 0.88 (3H, t, J=6.6 Hz, (CH₂)₈CH₃).

### 3-(3,4,5-Trimethoxyphenyl)propyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-benzamido-β-D-glucopyranoside) (26).

¹H-NMR (270MHz, CDCl₃): δ = 7.73 (2H, d, J=7.7 Hz, Bz-H), 7.51-7.41 (3H, m, Bz-H), 6.25 (2H, s, Ph-H), 6.20 (1H, d, J=8.6 Hz, NHBz), 5.60- 3.60 (27H, m), 3.86 (3H, s, CO₂Me), 3.78 (3H, s, OMe), 3.75 (6H, s, OMe x 2), 3.50-3.40 (2H, m, CH₂), 2.59 (1H, dd, J=4.6 and 12.5 Hz, H-3e of NeuAc), 2.53-2.40 (2H, m, CH₂), 2.21 (3H, s, OAc), 2.16 (3H, s, OAc), 2.11 (3H, s, OAc), 2.10 (3H, s, OAc), 2.09 (3H, s, OAc), 2.08 (3H, s, OAc), 2.06 (6H, s, OAc x 2), 2.04 (3H, s, OAc), 2.00 (3H, s, OAc), 1.93 (3H, s, OAc), 1.85 (3H, s, NAc), 1.90-1.65 (3H, m, H-3a of NeuAc and CH₂), and 1.20 (3H, d, J=6.6 Hz, Me of Fuc).

### 3-(4-Trifluoromethylphenyl)propyl [methyl (5-acetamido-3,5-dideoxy-4,7,8,9-tetra-O-acetyl-α-D-glycero-D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (27).

¹H-NMR (270MHz, CDCl₃): δ = 8.30 (1H, s, Napht-H), 7.98-7.78 (4H, m, Napht-H), 7.62-7.48 (2H, m, Napht-H), 7.53 (2H, d, J=8.6 Hz, Ph-H), 7.30 (2H, d, J=8.6 Hz, Ph-H), 6.68 (1H, d, J=8.5 Hz, NHNapht), 5.65-3.35 (29H, m), 3.86 (3H, s, CO₂Me), 2.77 (2H, t, J=7.9 Hz, CH₂), 2.59 (1H, m, H-3e of NeuAc), 2.23 (3H, s, OAc), 2.18 (3H, s, OAc), 2.12 (3H, s, OAc), 2.10 (6H, s, OAc x 2), 2.09 (6H, s, OAc x 2), 2.07 (6H, s, OAc x 2), 2.00 (3H, s, OAc), 1.93 (3H, s, OAc), 1.86 (3H, s, NAc), 2.00-1.80 (2H, m, CH₂), 1.70 (1H, t, J=12.5 Hz, H-3a of NeuAc), and 1.20 (3H, d, J=6.3Hz, Me of Fuc).

### 8-[(1,1,1-Tri(acetoxymethyl)methyl]amino carbonyl]octyl [methyl (5-acetamido-3,5-dideoxy-4,7,8,9-tetra-O-acetyl-α-D-glycero-D-galacto-2-nonulopyranosylonate )]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acety l-2-deoxy-2-naphthamido-β-D-glucopyranoside) (28).

¹H-NMR (270MHz, CDCl₃): δ = 8.28 (1H, s, Napht-H), 8.00-7.78 (4H, m, Napht-H), 7.62-7.50 (2H, m, Napht-H), 6.65 (1H, d, J=8.5Hz, NHNapht),5.87 (1H, s, NH), 5.60-3.30 (29H, m), 4.29 ( 6H, s, CH₂OAc x 3), 3.86 (3H, s, CO₂Me), 2.60 (1H, dd, J=4.5 and 12.2 Hz, H-3e of NeuAc), 2.23 (3H, s, OAc), 2.18 (6H, s, OAc x 2), 2.17 (6H, s, OAc x 2), 2.12 (3H, s, OAc), 2.10 (6H, s, OAc x 2), 2.08 (6H, s, OAc x 2), 2.06 (3H, s, OAc), 2.01 (3H, s, OAc), 1.98 (3H, s, OAc), 1.93 (3H, s, OAc), 1.86 (3H, s, NAc), 2.30-1.80 (4H, m, CH₂ x 2), 1.70 (1H, t, J=12.2 Hz, H-3a of NeuAc), 1.55-0.90 (10H, m, CH₂ x 5), and 1.20 (3H, d, J=6.3 Hz, Me of Fuc).

### 2-Phenylethyl [methyl (5-acetamido-3,5-dideoxy-4,7,8,9-tetra-O-acetyl-α-D-glycero-D-galacto-2-nonulupyrano sylonate)]-(2→3)-O-(2,4,6-tri-O-acetyl-β-D-galactopyranosyl )-(1→4)-O-[2,3,4-tri-O-acetyl-α-L-fucopyranosyl-(1→3)-O]-(6 -O-acetyl-2-deoxy-2-naphthamido- β-D-glucopyranoside) (29).

¹H-NMR (270MHz, CDCl₃): δ = 8.20 (1H, s, Napht-H), 7.98-7.81 (3H, m, Napht-H), 7.77 (1H, dd, J=1.7 and 8.6 Hz, Napht-H), 7.62-7.55 (2H, m, Napht-H), 7.07-6.92 (5H, m, Ph-H), 6.23 (1H, d, J=8.6 Hz, NHNapht), 5.60-3.75 (27H, m), 3.86 (3H, s, CO₂Me), 3.68-3.61 (2H, m), 2.80 (2H, t, J=6.9Hz, CH₂), 2.59 (1H, dd, J=4.3 and 12.5Hz, H-3e of NeuAc), 2.23 (3H, s, OAc), 2.18 (3H, s, OAc), 2.13 (3H, s, OAc), 2.10 (3H, s, OAc), 2.09 (3H, s, OAc), 2.08 (6H, s, OAc x 2), 2.05 (3H, s, OAc), 2.04 (3H, s, OAc), 2.01 (3H, s, OAc), 1.93 (3H, s, OAc), 1.86 (3H, s, NAc), 1.70 (1H,t, J=12.5Hz, H-3a of NeuAc), and 1.19 (3H, d, J=6.6Hz, Me of Fuc).

### 2-(3,4,5-Trimethoxyphenyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-deoxy-2-naphthamido-β-D-glucopyranoside) (30).

¹H-NMR (270MHz, CDCl₃): δ = 8.26 (1H, s, Napht-H), 7.98-7.86 (3H, m, Napht-H), 7.78 (1H, dd, J=1.7 and 8.6 Hz, Napht-H), 7.61-7.52 (2H, m, Napht-H), 6.35 (1H, d, J=8.9 Hz, NHNapht), 6.33 (2H, s, Ph-H), 5.60-3.60(29H, m), 3.86 (3H, s, CO₂Me), 3.72 (9H, s, OMe x 3), 2.76 (2H, t, J=7.3 Hz, CH₂), 2.60 (1H, dd, J=4.5 and 12.9 Hz, H-3e of NeuAc), 2.23 (3H, s, OAc), 2.17 (3H, s, OAc), 2.11 (3H, s, OAc), 2.10 (3H, s, OAc), 2.09 (3H,s, OAc), 2.08 (3H, s, OAc), 2.07 (3H, s, OAc), 2.06 (3H, s, OAc), 2.05 (3H, s, OAc), 2.01 (3H, s, OAc), 1.93 (3H, s, OAc), 1.86 (3H, s, NAc), 1.70 (1H, t, J=12.9 Hz, H-3a of NeuAc), and 1.19 (3H, d, J=6.3 Hz, Me of Fuc).

In a manner similar to that of Example 1, the following Compounds (31) to (45) of this invention were prepared from Compounds (16) to (30) as described above. Some compounds were isolated without neutralization using an acid type ion exchange resin (DOWEX 50W-8).

The structural formulae of Compounds (31) to (45) are as follows.

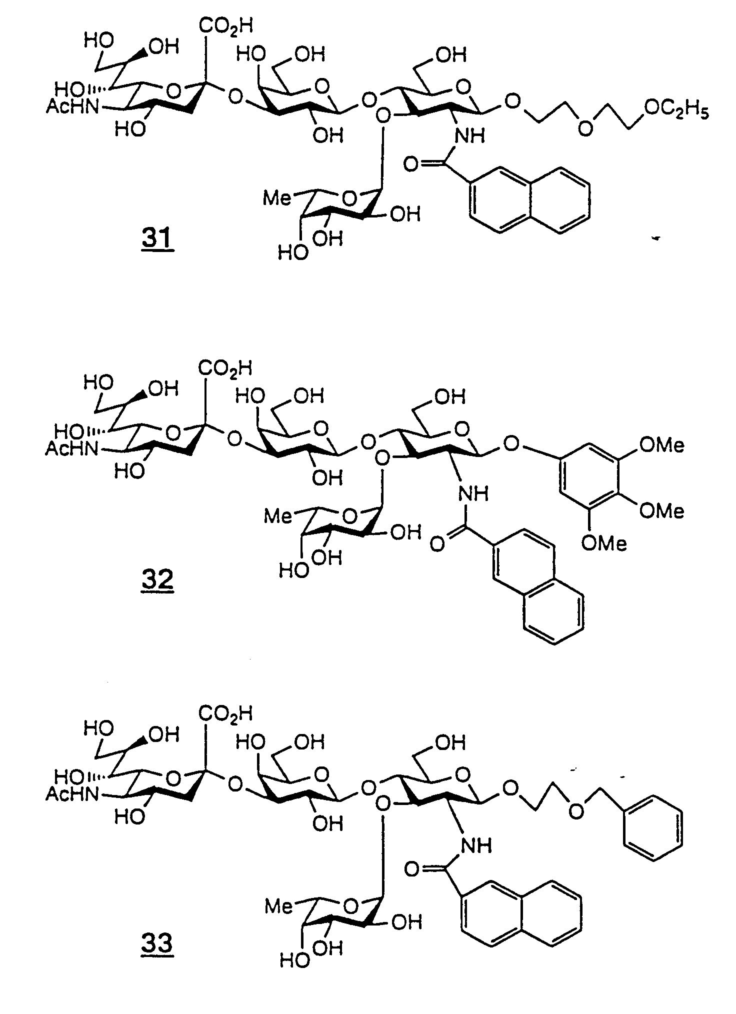

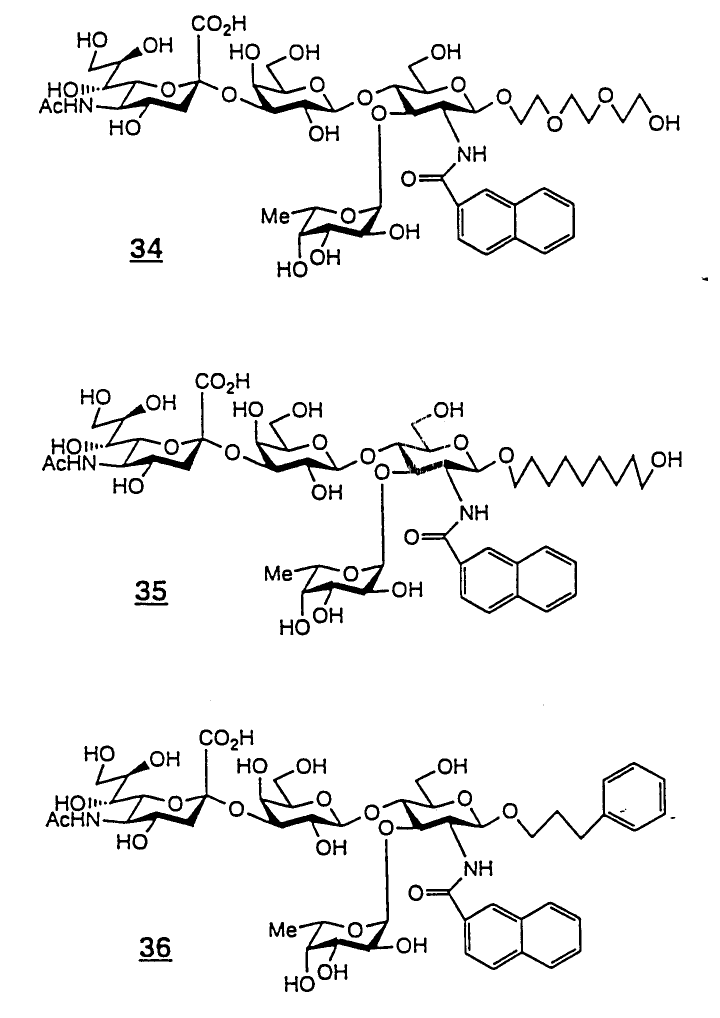

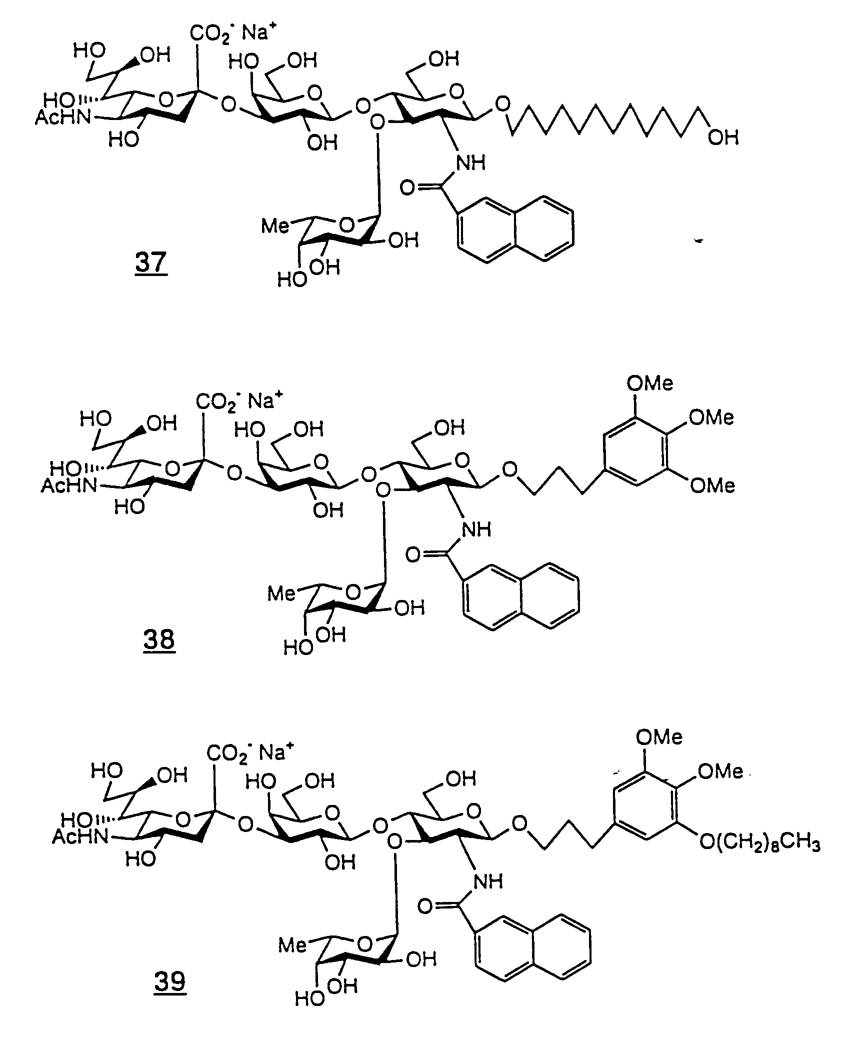

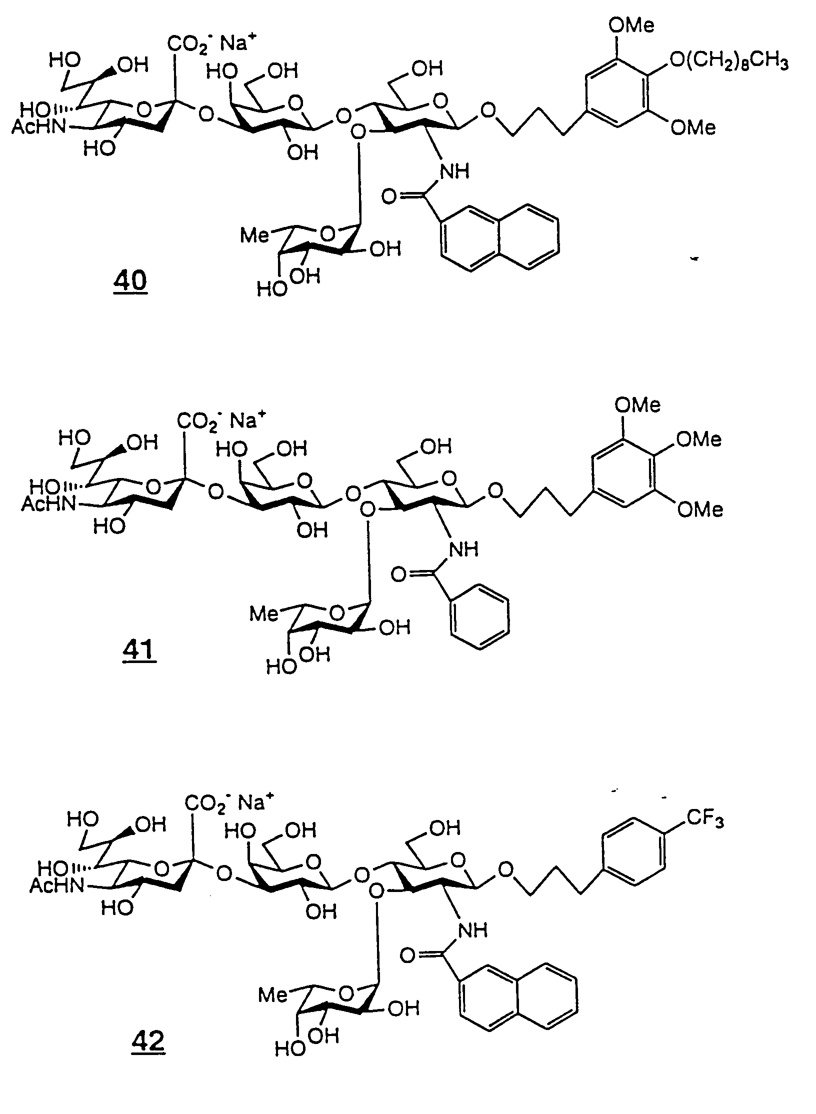

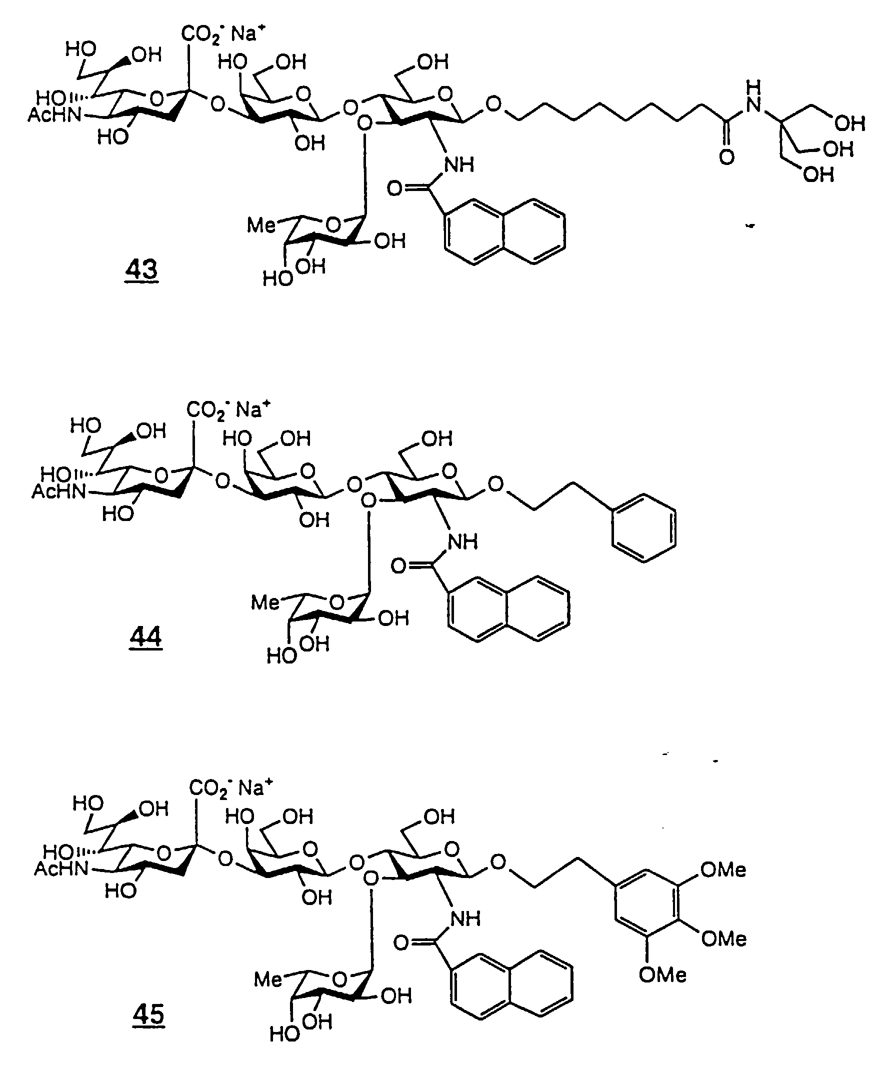

### 2-(2-Ethoxyethoxy)ethyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (31).

¹H-NMR (270MHz, CD₃OD): δ = 8.30 (1H, s, Napht-H), 7.95-7.80 (4H, m, Napht-H), 7.55-7.40 (2H, m, Napht-H), 5.06 (1H, d, J=3.3 Hz, H-1 of Fuc), 4.63 (1H, d, J=7.6 Hz), 4.45 (1H, d, J=7.6 Hz), 4.25-3.10 (33H), 2.78 (1H, dd, J=4.5 and 12.5 Hz, H-3e of NeuAc), 1.91 (3H, s, NAc), 1.63 (1H, t, J=12.5 Hz, H-3a of NeuAc), 1.06 (3H, d, J=6.6 Hz, Me of Fuc) and 0.91 (3H, t, J= 6.9 Hz, OCH₂CH₃).

### 3,4,5-Trimethoxyphenyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (32).

¹H-NMR (270MHz, D₂O): δ = 8.24 (1H, s, Napht-H), 8.00-7.80 (4H, m, Napht-H), 7.65-7.50 (2H, m, Napht-H), 6.28 (2H, s, Ph-H), 5.28 (1H, d, J=7.6 Hz), 5.11 (1H, d, J=4.0 Hz, H-1 of Fuc), 4.50 (1H, d, J=7.6 Hz), 4.10 (23H, m), 3.50 (6H, s, OMe x 2), 3.45 (3H, s, OMe) , 2.67 (1H, m, H-3e of NeuAc), 1.94 (3H, s, NAc), 1.76 (1H, m, H-3a of NeuAc), and 1.09(3H, d, J=6.6 Hz, Me of Fuc).

### 2-Benzyloxyethyl (5-acetamido-3,5-dideoxy-α-D-glycero- D-galacto-2-nonulopyranosylonic acid)-(2→3)-O-(β-D-galactopyranosyl)-(1→4)-O-[α-L-fucopyranosyl-(1→3)-O ]-(2-deoxy-2-naphthamido-β-D-glucopyranoside) (33).

¹H-NMR (270MHz, D₂O) δ = 8.20 (1H, s, Napht-H), 7.87-7.83 (3H, m, Napht-H), 7.68 (1H, d, J=8.6 Hz, Napht-H), 7.60-7.51 (2H, m, Napht-H), 6.92-6.74 (5H, m, Ph-H), 5.11 (1H, d, J=3.6 Hz, H-1 of Fuc), 4.76 (1H, d, J=7.6 Hz), 4.48 (1H, d, J=7.3 Hz), 4.30-3.40 (29H, m), 2.68 (1H, dd, J=4.5 and 12.2 Hz, H-3e of NeuAc), 1.95 (3H, s, NAc), 1.72 (1H, t, J=12.2 Hz, H-3a of NeuAc), and 1.09 (3H, d, J=6.3 Hz, Me of Fuc).

### 2-[2-(2-Hydroxyethoxy)ethoxy]ethyl (5-acetamido-3,5-dideoxy-α-D-glycero-D-galacto-2-nonulopyra nosylonic acid)-(2→3)-O-(β-D-galactopyranosyl)-(1→4)-O-[α-L-fucopyranosyl-(1→3)-O]-(2-deoxy-2-paphthamido-β-D-glucopyranoside) (34).

¹H-NMR (270MHz, D₂O): δ = 8.34 (1H, s, Napht-H), 8.03-7.94 (3H, m, Napht-H), 7.82 (1H, m, Napht-H), 7.66-7.60 (2H, m, Napht-H), 5.16 (1H, d, J=3.6 Hz, H-1 of Fuc), 4.78 (1H, d, J=8.2 Hz), 4.51 (1H, d, J=7.9 Hz), 4.25-3.43 (27H, m), 3.30-3.24 (4H, m), 3.00-2.84 (4H, m), 2.71 (1H, dd, J=4.2 and 12.5 Hz, H-3e of NeuAc), 1.97 (3H, s, NAc), 1.74 (1H, t, J=12.5 Hz, H-3a of NeuAc), and 1.11 (3H, d, J=6.6 Hz, Me of Fuc).

### 9-Hydroxynonyl (5-acetamido-3,5-dideoxy-α-D-glycero- D-galacto-2-nonulopyranosylonic acid)-(2→3)-O-(β-D-galactopyranosyl)-(1→4)-O-[α-L-fucopyranosyl-(1→3)-O ]-(2-deoxy-2-naphthamido-β-D-glucopyranoside) (35).

¹H-NMR (270MHz, D₂O): δ = 8.29 (1H, s, Napht-H), 7.98-7.86 (3H, m, Napht-H), 7.76 (1H, d, J=8.6 Hz, Napht-H), 7.60-7.50 (2H, m, Napht-H), 5.10 (1H, d, J=3.6 Hz, H-1 of Fuc), 4.65 (1H, d), 4.49 (1H, d, J=7.6 Hz), 4.15-3.35 (25H, m), 3.21 (2H, t, J=6.8 Hz, CH₂), 2.67 (1H, dd, J=4.3 and 11.9 Hz, H-3e of NeuAc), 1.95 (3H, s, NAc), 1.72 (1H, t, J=11.9 Hz, H-3a of NeuAc), 1.40-1.23 (2H, m, CH₂), 1.15 (3H, d, J=6.6 Hz, Me of Fuc), 1.02-0.87 (4H, m, CH₂ x 2), 0.82 (2H, m, CH₂), and 0.40 (6H, m, CH₂ x 3).

### 3-Phenylpropyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (36).

¹H-NMR (270MHz, D₂O): δ = 8.26 (1H, s, Napht-H), 7.89-7.84 (3H, m, Napht-H), 7.75 (1H, d, J=10.6Hz, Napht-H), 7.58-7.47 (2H, m, Napht-H), 6.88-6.77 (3H, m, Ph-H), 6.61 (2H, d, J=6.6 Hz, Ph-H), 5.17 (1H, d, J=4.0 Hz, H-1 of Fuc), 4.77 (1H, d), 4.49 (1H, d, J=7.9 Hz), 4.25-3.40 (25H, m), 2.70 (1H, dd, J =3.6 and 12.2 Hz, H-3e of NeuAc), 2.38-2.23 (2H, m, CH₂), 1.95 (3H, s, NAc), 1.77 (1H, t, J=12.2 Hz, H-3a of NeuAc), 1.70-1.58 (2H, m, CH₂), and 1.10 (3H, d, J=6.6 Hz, Me of Fuc).

### 12-Hydroxydodecyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (37).

¹H-NMR (270MHz, CD₃OD): δ = 8.28 (1H, s, Napht-H), 7.89-7.81 (4H, m, Napht-H), 7.50-7.45 (2H, m, Napht-H), 5.04 (1H, d, J=4.3 Hz, H-1 of Fuc), 4.56 (1H, d, J=8.3 Hz), 4.46 (1H, d, J=7.6 Hz), 4.20-3.30 (27H, m), 2.79 (1H, dd, J=3.3 and 11.9 Hz, H-3e of NeuAc), 1.91 (3H, s, NAc), 1.63 (1H, m, H-3a of NeuAc), 1.42-1.30 (4H, m, CH₂ x 2), 1.20-0.70 (16H, m, CH₂ x 8), and 1.06 (3H, d, J= 66 Hz, Me of Fuc).

### 3-(3,4,5-Trimethoxyphenyl)propyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyranosyl-(1→3)-O]-(2-deoxy-2-naphthamido-β-D-glucopyra noside) (38).

¹H-NMR (270MHz, CD₃OD): δ = 8.26 (1H, s, Napht-H), 7.82-7.78 (4H, m, Napht-H), 7.49-7.43 (2H, m, Napht-H), 6.15 (2H, s, Ph-H), 5.06 (1H, d, J=4.3 Hz, H-1 of Fuc), 4.56 (1H, d, J=8.0 Hz), 4.45 (1H, d, J=7.6 Hz), 4.20-3.30 (25H, m), 3.54 (3H, s, OMe), 3.45 (6H, s, OMe x 2), 2.80 (1H, dd, J=4.5 and 12.5 Hz, H-3e of NeuAc), 2.52-2.30 (2H, m, CH₂), 1.90 (3H, s, NAc), 1.80-1.55 (3H, m, CH₂ and H-3a of NeuAc), and 1.05 (3H, d, J=6.3 Hz, Me of Fuc).

### 3-(4,5-Dimethoxy-3-nonyloxyphenyl)propyl (5-acetamido-3,5-dideoxy-α-D-glycero-D-galacto-2-nonulopyra nosylonic acid)-(2→3)-O-(β-D-galactopyranosyl)-(1→4) -O-[α-L-fucopyranosyl-(1→3)-O]-(2-deoxy-2-naphthamido-β-D-g lucopyranoside) (39).

¹H-NMR (270MHz, CD₃OD): δ = 8.26 (1H, s, Napht-H), 7.81-7.78 (4H, m, Napht-H), 7.51-7.43 (2H, m, Napht-H), 6.15 (1H, s), 6.12 (1H, s), 5.06 (1H, d, J=4.3 Hz, H-1 of Fuc), 4.57 (1H, d, J=8.0 Hz), 4.46 (1H, d, J=7.6 Hz), 4.20-3.30 (27H, m), 3.56 (3H, s, OMe), 3.47 (3H, s, OMe), 2.79 (1H, dd, J=4.5 and 12.5 Hz, H-3e of NeuAc), 2.55-2.35 (2H, m, CH₂), 1.91 (3H, s, NAc), 1.80-1.58 (3H, m, CH₂ and H-3a of NeuAc), 1.55-1.40 (2H, m, CH₂), 1.30-1.10 (12H, m, CH₂ x 6), 1.07 (3H, d, J=6.6 Hz, Me of Fuc), and 0.80 (3H, t, J=6.7 Hz, (CH₂)₈CH₃).

### 3-(3,5-Dimethoxy-4-nonyloxyphenyl)propyl (5-acetamido-3,5-dideoxy-α-D-glycero-D-galacto-2-nonulopyra nosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (40).

¹H-NMR (270MHz, CD₃OD): δ = 8.27 (1H, s, Napht-H), 7.81-7.78 (4H, m, Napht-H), 7.49-7.42 (2H, m, Napht-H), 6.14 (2H, s, Ph-H), 5.06 (1H, d, J=4.0 Hz, H-1 of Fuc), 4.57 (1H, d, J=8.0 Hz), 4.46 (1H, d, J=7.6 Hz), 4.20-3.30 (27H, m), 3.43 (6H, s, OMe x 2), 2.78 (1H, dd, J=4.5 and 12.5 Hz, H-3e of NeuAc), 2.47-2.39 (2H, m, CH₂), 1.91 (3H, s, NAc), 1.80-1.58 (3H, m, CH₂ and H-3a of NeuAc), 1.55-1.40 (2H, m, CH₂), 1.30-1.10 ( 12H, m, CH₂ x 6), 1.06 (3H, d, J=6.3 Hz, Me of Fuc), and 0.80 (3H, t, J=6.7 Hz, (CH₂)₈CH₃).

### 3-(3,4,5-Trimethoxyphenyl)propyl (5-acetamido-3,5-dideoxy-α- D-glycero-D-galacto-2-nonulopyranosylonic acid)-(2→3)-O-(β-D-galactopyranosyl)-(1→4)-O-[α-L-fucopyra nosyl-(1→3)-O]-(2-deoxy-2-benzamido-β-D-glucopyranoside) (41).

¹H-NMR (270MHz, CD₃OD): δ = 7.72 (2H, d, J=7.3 Hz, Bz-H), 7.42-7.30 (3H, m, Bz-H), 6.23 (2H, s, Ph-H), 4.99 (1H, d, J=4.3 Hz, H-1 of Fuc), 4.54 (1H, d, J=7.6 Hz), 4.45 (1H, d, J=7.9 Hz), 4.20-3.30 (25H, m), 3.61 (6H, s, OMe x 2), 3.59 (3H, s, OMe), 2.78 (1H, dd, J=4.5 and 12.5 Hz, H-3e of NeuAc), 2.45-2.41 (2H, m, CH₂), 1.91 (3H, s, NAc), 1.80-1.55 (3H, CH₂ and H-3a of NeuAc), and 1.05 (3H, d, J=6.6Hz, Me of Fuc).

### 3-(4-Trifluoromethyl)phenylpropyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyra nosyl-(1→3)-O]-(2-deoxy-2-naphthamido-β-D-glucopyranoside) (42).

¹H-NMR (270MHz, D₂O): δ = 8.06 (1H, s, Napht-H), 7.60-7.50 (1H, m, Napht-H), 7.40 7.10 (3H, m, Napht-H), 7.05-6.88 (2H, m, Napht-H), 6.66 (2H, d, J=8.0 Hz, Ph-H), 6.32 (2H, d, J=8.0 Hz, Ph-H), 5.16 (1H, d, J=4.0 Hz, H-1 of Fuc), 4.60-4.45 (2H, m, H-1 of Gal and H-1 of G1cN), 4.30-3.40 (23H, m), 3.35-3.20 (2H, m, CH₂), 2.68 (1H, dd, J=4.5 and 12.5 Hz, H-3e of NeuAc), 2.15-2.00 (2H, m, CH₂), 1.94 (3H, s, NAc), 1.72 (1H, t, J=12.5 Hz, H-3a of NeuAc), 1.45-1.25 (2H, m, CH₂), and 1.10 (3H, d, J=6.5Hz, Me of Fuc).

### 8-[[1,1,1-Tri(hydroxymethyl)methyl]aminocarbonyl] octyl (5-acetamido-3,5-dideoxy-α-D-glycero-D-galacto-2-nonu lopyranosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (43).

¹H-NMR (270MHz, D₂O): δ = 8.30 (1H, s Napht-H), 7.99-7.84 (3H, m, Napht-H), 7.78 (1H, dd, J=1.5 and 8.9 Hz, Napht-H), 7.62-7.50 (2H, m, Napht-H), 5.14 (1H, d, J=4.0 Hz, H-1 of Fuc), 4.80-4.60 (1H, m), 4.49 (1H, d, J=7.9 Hz), 4.20-3.40 (31H, m), 2.69 (1H, dd, J=4.5 and 12.4 Hz, H-3e of NeuAc), 1.95 (3H, s, NAc), 1.72 (1H, dd, J=12.4 Hz, H-3a of NeuAc), 1.66 (2H, t, J=7.8 Hz, CH₂), 1.40-1.25 (2H, m, CH₂), 1.10 (3H, d, J=6.6 Hz, Me of Fuc), 1.02-0.89 (2H, m, CH₂), 0.84-0.68 (4H, m, CH₂ x 2), and 0.62-0.35 (4H, m, CH₂ x 2).

### 2-Phenylethyl (5-acetamido-3,5-dideoxy-α-D-glycero-D-galacto-2-nonulopyranosylonic acid)-(2→3)-O-(β-D-galactopyranosyl)-(1→4)-O-[α-L-fucopyranosyl-(1→3)-O]-(2-deoxy-2-naphthamido-β-D-glucopyranoside) (44).

¹H-NMR (270MHz, D₂O): δ = 8.00-7.91 (4H, m, Napht-H), 7.66-7.54 (3H, m, Napht-H), 6.98 (2H, d, J=7.3 Hz, Ph-H), 6.67 (2H, t, J=7.3 Hz, Ph-H), 6.49 (1H, t, J=7.3 Hz, Ph-H), 5.03 (1H, d, J=4.3 Hz, H-1 of Fuc), 4.70-4.50 (1H, d), 4.48 (1H, d, J=7.9Hz), 4.20-3.40 (25H, m), 2.75-2.60 (3H, m, OCH₂CH₂Ph and H-3e of NeuAc), 1.95 (3H, s, NAc), 1.72 (1H, t, J=12.2 Hz, H-3a of NeuAc), and 1.07 (3H, d, J=6.6 Hz, Me of Fuc).

### 2-(3,4,5-Trimethoxyphenyl)ethyl (5-acetamido-3,5-dideoxy-α-D-glycero-D-galacto-2-nonulopyranosylonic acid)-(2→3)-O-(β-D-galactopyranosyl)-(1→4)-O-[α-L-fuco pyranosyl-(1→3)-O]-(2-deoxy-2-naphthamido-β-D-glucopyra noside) (45).

¹H-NMR (270MHz, D₂O): δ = 7.84-7.74 (4H, m, Napht-H), 7.55-7.50 (2H, m, Napht-H), 7.42 (1H, d, J=8.6 Hz, Napht-H), 6.33 (2H, s, Ph-H), 4.95 (1H, d, J=4.0 Hz, H-1 of Fuc), 4.70-4.60 (1H, d), 4.48 (1H, d, J=7.6 Hz), 4.20-3.35 (25H, m), 3.47 (9H, s, OMe x 3), 2.78-2.65 (3H, m, OCH₂CH₂Ph and H-3e of NeuAc), 1.95 (3H, s, NAc), 1.78 (1H, t, J=12.5 Hz, H-3a of NeuAc), and 1.05 (3H, d, J=6.3 Hz, Me of Fuc).

### [Reference Example 1]

### Synthesis of 2-(trimethylsilyl)ethyl 2-N-allyloxycarbonyl-2-amino-2-deoxy-3,4,6-tri-O-acetyl-β-D -glucopyranoside (46).

To a solution of 2-N-allyloxycarbonyl-2-amino-2-deoxy-1,3,4,6-tetra-O-acetyl-β-D-glucopyranose (47) (172.56 g, 0.40 mmol) in dichloromethane (1035 ml) cooled to -15°C was added dropwise a solution of 25% hydrogen bromide in acetic acid (388.4 g, 1.20 mmol) over 1 hour. The mixture was stirred at -15°C for 2 hours and after confirming the production of 2-N-allyloxycarbonyl-2-amino-2-deoxy-3,4,6-tri-O-acetyl-α-D-glucopyranosyl bromide (48), the reaction mixture was washed with water, 5% aqueous sodium bicarbonate, and water successively, and the organic layer was dried over Molecular Sieves 4A, and filtered to give a solution of Compound 48 in dichloromethane. Then the resulting solution of Compound 48 was added dropwise into a mixture of 2-(trimethylsilyl)ethanol (94.6 g, 0.80 mmol), silver (I) carbonate (331g, 1.2 mmol), and Molecular Sieves 4A (429 g) in dichloromethane (1035 ml) at -5°C over 90 minutes, and the mixture was stirred for 1 hour. After confirming the completion of reaction, the reaction mixture was filtered, washed with aqueous 5% sodium bicarbonate and water successively, and the organic layer was concentrated. The resulting residue was dissolved in toluene, added dropwise into hexane, and precipitated crystals were collected by filtration to give objective Compound 46 (153.0 g, Yield: 78%) as white crystals. mp. 70-72°C.
¹H-NMR (270MHz, CDCl₃): δ = 5.81 (1H, m), 5.27-5.08 (4H, m), 4.96(1H, t, J=9.6 Hz), 4.65-4.45 (2H, m), 4.47 (1H, d, J=4.3 Hz, H-1), 4.20 (1H, dd, J=4.6 and 11.9 Hz), 4.04 (1H, dd, J=2.3 and 11.9 Hz), 3.89 (1H, m), 3.65 (1H, m), 3.63-3.45 (2H, m), 1.99 (3H, s, Ac), 1.94 (3H, s, Ac), 1.93 (3H, s, Ac), 0.94-0.80 (2H, m, OCH₂CH₂SiMe₃), and -0.08 (9H, s, OCH₂CH₂SiMe₃).

### [Reference Example 2]

### Synthesis of 2-(trimethylsilyl)ethyl 2-N-allyloxycarbonyl-2-amino- 2-deoxy-β-D-glucopyranoside (1).

To a solution of 2-(trimethylsilyl)ethyl 2-N-allyloxycarbonyl-2-amino-2-deoxy-3,4,6-tri-O-acetyl-β-D -glucopyranoside (46) (49.0 g, 0.10 mmol) in methanol (150 ml) was added 28% sodium methoxide solution (in methanol, 6.0 g, 0.02 mmol) at room temperature, and the mixture was stirred for 1 hour. After confirming the completion of reaction, the reaction mixture was concentrated. The resulting residue was dissolved in dichloromethane, washed with water, crystallized using heptane, and the crystals were collected by filtration to give objective Compound 1 (28.6 g, Yield: 79%) as white solid. mp. 134-138°C.
¹H-NMR (270MHz, CDCl₃): δ = 5.90 (1H, m), 5.75 (1H, d, J=8.3 Hz, -NHCO₂-), 5.30 (1H, dd, J=1.3 and 17.2 Hz), 5.18 (1H, dd, J=1.3 and 10.2 Hz), 5.05 (1H, s, OH), 4.72 (1H, s, OH), 4.55 (1H, d, J=5.6 Hz, H-1), 4.48 (1H, m), 3.96 (1H, m), 3.85 (2H, m), 3.80-3.23 (6H, m), 2.41 (1H, s, OH), 0.99-0.85 (2H, m, OCH₂CH₂SiMe₃), 0.00 (9H, s, OCH₂CH₂SiMe₃).

The structural formulae of Compounds (46) to (48) in Reference Examples 1 and 2 are as follows:

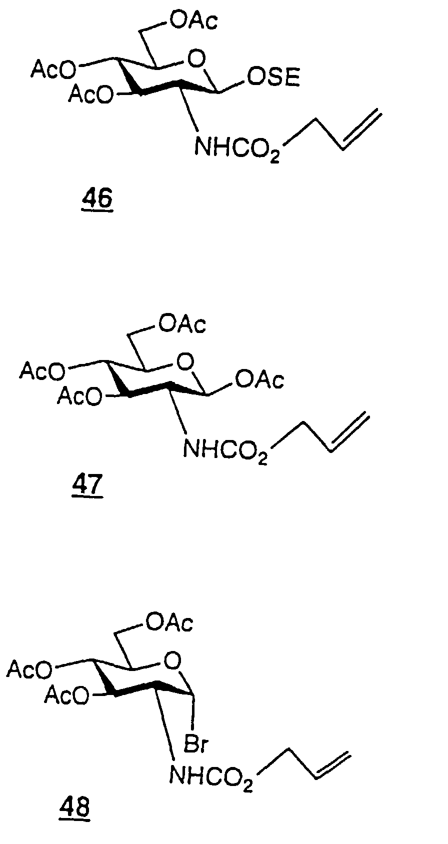

### [Reference Example 3]

1,12-Dodecanediol monobenzoyl ester used for synthesizing glycosidated Product 22 was synthesized as follows.

Commercially available 1,12-dodecanediol (5.00 g, 24.7 mmol) was dissolved in hot pyridine (20 ml), cooled to room temperature, and benzoyl chloride (3.47 g, 2.47 mmol) was added thereto dropwise. After stirring for 2 days at room temperature, the reaction mixture was diluted with ethyl acetate (200 ml) and washed with saturated aqueous sodium bicarbonate, aqueous 1N-hydrochloric acid and saturated aqueous sodium bicarbonate, successively. The organic layer was dried over anhydrous sodium sulfate, filtered, and the solvent was evaporated under reduced pressure. The resulting residue was purified by column chromatography over silica gel (eluant, hexane: ethyl acetate = 2:1) to give objective Compound 49 (3.79 g, Yield: 50%) as white crystals.
¹H-NMR (270MHz, CDCl₃): δ = 8.06-8.03 (2H, m), 7.59-7.52 (1H, m), 7.47-7.41 (2H, m), 4.32 (2H, t, J=6.6 Hz, CH₂OBz), 3.54 (2H, t, J=6.6 Hz, CH₂OH), 1.79-1.23 (21H, m).

### [Reference Example 4]

3-(3,4,5-Trimethoxyphenyl)-1-propanol (52) used for synthesizing glycosidated Product 23 was synthesized as follows:

### [Reference Example 4-1]

### Synthesis of ethyl 3,4,5-trimethoxycinnamate (50)

To a suspension of sodium hydride (60% assay, 944 mg, 23.65 mmol) in dry tetrahydrofuran (200 ml) under ice cooling was added dropwise a solution of triethyl phosphonoacetate (5.30 g, 23.65 mmol) in dry tetrahydrofuran (100 ml). After stirring under ice cooling for 15 minutes, a solution of commercially available 3,4,5-trimethoxybenzaldehyde (4.21 g, 21.50 mmol) in dry tetrahydrofuran (100 ml) was added dropwise. After stirring for 15 minutes under ice cooling, the reaction was quenched by dropwise addition of water. The reaction mixture was diluted with ethyl acetate (300 ml) and washed with water and brine, successively. The organic layer was dried over anhydrous magnesium sulfate, filtered, and the solvent was evaporated in vacuo. The resulting residue was purified by chromatography over silica gel (eluant; hexane:ethyl acetate = 6:1) to give objective Compound 50 (5.34 g, Yield: 94%).
¹H-NMR (270MHz, CDCl₃): δ = 7.61 (1H, d, J=16.1 Hz), 6.76 (2H, s, Ph-H), 6.35 (1H, d, J=16.1 Hz), 4.27 (1H, q, J=7.2 Hz, OCH₂CH₃), 3.89 (9H, s, OMe x 3), 1.34 (3H, t, J=7.2 Hz, OCH₂CH₃).

### [Reference Example 4-2]

### Synthesis of 3,4,5-trimethoxycinnamyl alcohol (51)

To a solution of ethyl 3,4,5-trimethoxycinnamate (50) (3.00 g, 11.3 mmol) in dry tetrahydrofuran (70 ml) cooling with dry ice-carbon tetrachloride to -20°C was added dropwise a solution of diisobutylaluminium hydride in hexane (0.93M, 2.67 ml, 24.8 mmol). After stirring for 15 minutes under cooling, the reaction was quenched by dropwise addition of methanol. To the reaction mixture was added dropwise aqueous 4N-hydrochloric acid. The mixture was diluted further with water and extracted with ethyl acetate. The organic layer was washed with water and brine, successively, dried over anhydrous magnesium sulfate, filtered, and the solvent was evaporated in vacuo to give a residue containing objective Compound 51. This was used in the next reaction without further purification.
¹H-NMR (270MHz, CDCl₃): δ = 6.61 (2H, s, Ph-H),6.54 (1H, dt, J=15.8 and 1.3 Hz), 6.29 (1H, dt, J=15.8 and 5.6 Hz), 4.33 (2H, dd, J=5.6 and 1.3 Hz), 3.87 (6H, s, OMe x 2), 3.82 (3H, s, OMe), 1.68 (1H, br., OH).

### [Reference Example 4-3]

### Synthesis of 3-(3,4,5-trimethoxyphenyl)-1-propanol (52)

To a solution of 3,4,5-trimethoxycinnamyl alcohol (51) obtained in Reference Example 4-2 (theoretical amount: 2.62 g) in methanol (70 ml) was added 10% Pd-C (wet, 150 mg). The reaction vessel was filled with hydrogen gas and hydrogenation was carried out at room temperature under atmospheric pressure. After 1 hour, the reaction mixture was filtered through a pad of Celite, the solid was washed with methanol, and the filtrate was concentrated in vacuo. The resulting residue was purified by column chromatography over silica gel (eluant; hexane:ethyl acetate = 1:1) to give objective Compound 52 (2.55 g, Yield: 67% from Compound 50 in 2 steps).
¹H-NMR (270MHz, CDCl₃): δ = 6.43 (2H, s, Ph-H), 3.85 (6H, s, OMe x2), 3.83 (3H, s, OMe), 3.70 (2H, t, J=6.3 Hz, CH₂OH), 2.66 (2H, m), 1.89 (2H, m), 1.54 (1H, br., OH).

### [Reference Example 5]

3-(4,5-Dimethoxy-3-nonyloxyphenyl)-1-propanol (56) used for synthesizing glycosidated Product 24 was synthesised as follows.

### [Reference Example 5-1]

### Synthesis of 5-nonyloxyveratraldehyde (53)

To a solution of commercially available 5-hydroxyveratraldehyde (1.50 g, 8.23 mmol), triphenylphosphine (2.81 g, 10.70 mmol) and 1-nonyl alcohol (1.79 g, 12.4 mmol) in dry tetrahydrofuran (200 ml) under ice cooling was added dropwise diethyl azodicarboxylate (1.87 g, 10.70 mmol). After stirring for 30 minutes at room temperature, triphenylphosphine (0.43 g, 1.64 mmol) and 1-nonyl alcohol (0.59 g, 4.09mmol) were added, ice-cooled, and diethyl azodicarboxylate (0.28 g, 1.61mmol) was dropwise added. After stirring for 30 minutes at room temperature, additional triphenylphosphine (0.43 g, 1.64 mmol) and diethyl azodicarboxylate (0.28 g, 1.61 mmol) were added under ice cooling, and the mixture was stirred at room temperature for 30 minutes. The reaction mixture was concentrated in vacuo and the resulting residue was purified by column chromatography over silica gel (eluant; hexane:ethyl acetate = 20:1) to give objective Compound 53 (2.59 g, quantitative).
¹H-NMR (270MHz, CDCl₃): δ = 9.86 (1H, s, CHO), 7.12 (1H, s, Ph-H), 7.11 (1H, s, Ph-H), 4.07 (2H, t, J=6.6 Hz, OCH₂CH₂), 3.94 (3H, s, OMe), 3.93 (3H, s, OMe), 1.86 (2H, m, OCH₂CH₂), 1.53-1.24 (12H, m), 0.88 (3H, t, J= 6.9 Hz, CH₃).

### [Reference Example 5-2]

### Synthesis of 3-(4,5-dimethoxy-3-nonyloxyphenyl)-1-propanol (56)

In a manner similar to Reference Example 4, objective Compound 56 was prepared using 5-nonyloxyveratrum aldehyde (53), through Compounds 54 and 55.

NMR data of Compounds 54, 55 and 56 are given below.

### Ethyl 4,5-dimethoxy-3-nonyloxycinnamate (54)

¹H-NMR (270MHz, CDCl₃): δ = 7.59 (1H, d, J=16.1 Hz), 6.75 (1H, s, Ph-H), 6.74 (1H, s, Ph-H), 6.34 (1H, d, J=16.1 Hz), 4.27 (1H, q, J=7.2 Hz, OCH₂CH₃), 4.01 (1H, t, J=6.6 Hz, OCH₂CH₂), 3.88 (6H, s, OMe x 2), 1.84 (2H, m), 1.55-1.20 (12H, m), 1.34 (3H, t, J=7.2 Hz, OCH₂CH₃), 0.88 (3H, t, J=6.6 Hz, CH₃).

### 4,5-Dimethoxy-3-nonyloxycinnamyl alcohol (55)

¹H-NMR (270MHz, CDCl₃): δ = 6.60 (2H, s, Ph-H), 6.53 (1H, d, J=15.8 Hz), 6.27 (1H, dt, J=15.8 and 5.6 Hz), 4.32 (2H, d, J=5.6 Hz, CH₂OH), 4.00 (2H, t, J=6.6 Hz, OCH₂CH₂), 3.86 (3H, s, OMe), 3.85 (3H, s, OMe), 1.81 (2H, m), 1.66-1.27 (13H, m), 0.88 (3H, t, J=6.6 Hz, CH₃).

### 3-(4,5-Dimethoxy-3-nonyloxyphenyl)-1-propanol (56)

¹H-NMR (270MHz, CDCl₃): δ = 6.41 (1H, s, Ph-H), 6.40 (1H, s, Ph-H), 3.98 (2H, t, J=6.6 Hz, OCH₂CH₂), 3.84 (3H, s, OMe), 3.82 (3H, s, OMe), 3.69 (2H, t, J=6.3 Hz, CH₂OH), 1.94-1.75 (4H, m), 1.50-1.23 (13H, m), 0.88 (3H, t, J=6.6Hz, CH₃).

### [Reference Example 6]

3-(3,5-Dimethoxy-4-nonyloxyphenyl)-1-propanol (60) used for preparing glycosidated Product 25 was synthesized using commercially available syringaldehyde, in a manner similar to that of Reference Example 5, through Compounds 57, 58 and 59.

NMR data of Compounds 57, 58, 59 and 60 are given below.

### 4-O-Nonylsyringaldehyde (57)

¹H-NMR (270MHz, CDCl₃): δ = 9.87 (1H, s, CHO), 7.13 (2H, s, Ph-H), 4.07 (2H, t, J=6.6 Hz, OCH₂CH₂), 3.92 (6H, s, OMe x 2), 1.74 (2H, m, OCH₂CH₂), 1.57-1.27 (12H, m), 0.88 (3H, t, J=6.9 Hz, CH₃).

### Ethyl 3,5-dimethoxy-4-nonyloxycinnamate (58)

¹H-NMR (270MHz, CDCl₃): δ = 7.60 (1H, d, J=16.1 Hz), 6.75 (2H, s, Ph-H), 6.34 (1H, d, J=16.1 Hz), 4.26 (1H, q, J=7.2 Hz, OCH₂CH₃), 3.99 (1H, t, J=6.6 Hz, OCH₂CH₂), 3.87 (6H, s, OMe x 2), 1.74 (2H, m), 1.50-1.25 (12H, m), 1.34 (3H, t, J=7.2 Hz, OCH₂CH₃), 0.88 (3H, t, J=6.6 Hz, CH₃).

### 3,5-Dimethoxy-4-nonyloxycinnamyl alcohol (59)

¹H-NMR (270MHz, CDCl₃): δ = 6.60 (2H, s, Ph-H), 6.53 (1H, d, J=15.8 Hz), 6.27 (1H, dt, J=15.8 and 5.6 Hz), 4.31 (2H, d, J=5.6 Hz, CH₂OH), 3.95 (2H, t, J=6.9 Hz, OCH₂CH₂), 3.85 (6H, s, OMe x 2), 1.73 (2H, m), 1.66-1.27 (13H, m), 0.88 (3H, t, J=6.6 Hz, CH₃).

### 3-(3,5-Dimethoxy-4-nonyloxyphenyl)-1-propanol (60)

¹H-NMR (270MHz, CDCl₃): δ = 6.41 (2H, s, Ph-H), 3.92 (2H, t, J=6.9 Hz, OCH₂CH₂), 3.83 (6H, s, OMe x 2), 3.70 (2H, t, J=6.3 Hz, CH₂OH), 1.91 (2H, m), 1.73 (2H, m), 1.50-1.21 (13H, m), 0.88 (3H, t, J=6.6 Hz, CH₃).

The structural formulae of Compounds (49) to (60) in Reference Examples 3 to 6 are as follows:

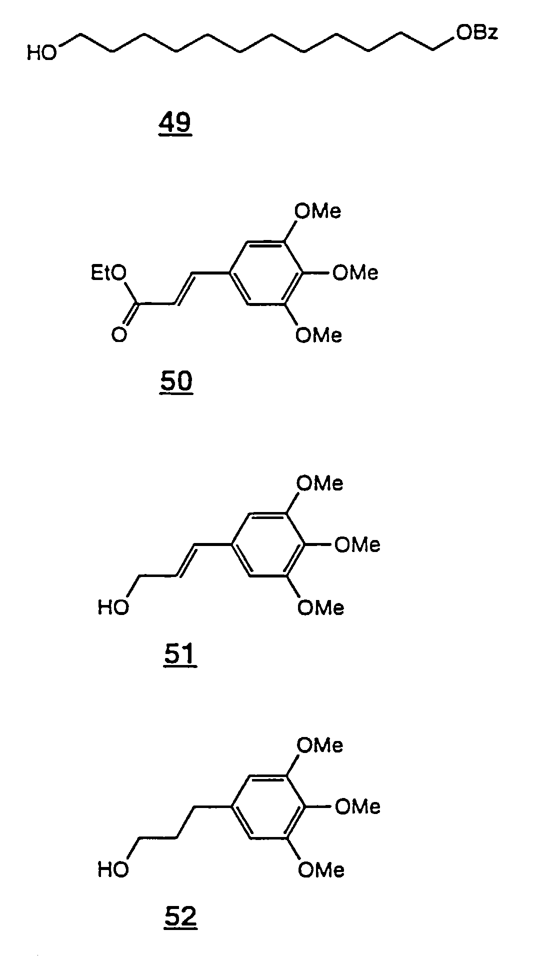

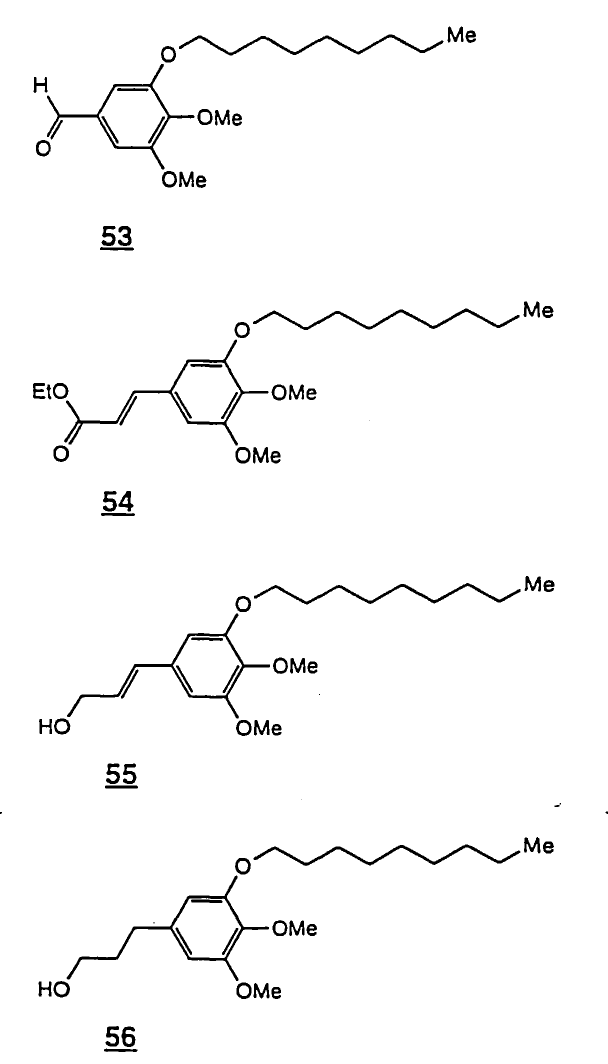

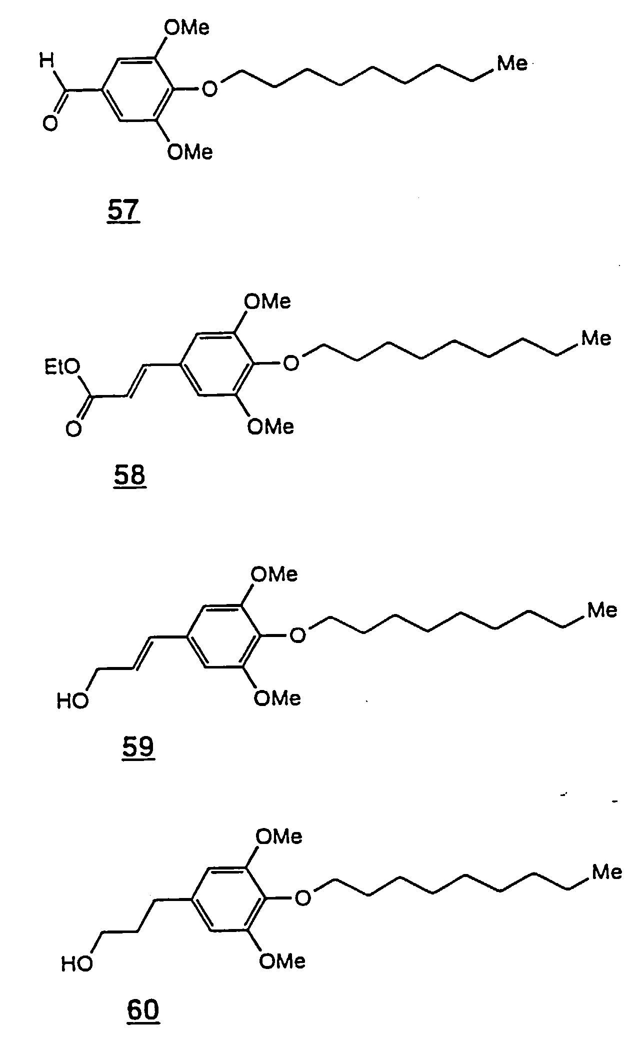

### [Reference Example 7]

3-(4-Trifluoromethylphenyl)-1-propanol (63) used for synthesizing glycosidated Product 27 was synthesised utilizing commercially available 4-trifluoromethyl benzaldehyde in a manner similar to that of Example 4, through Compounds 61 and 62.

NMR data of Compounds 61, 62 and 63 are as shown below.

### Ethyl 4-trifluoromethylcinnamate (61)

¹H-NMR (270MHz, CDCl₃): δ = 7.70 (1H, d, J=16.2 Hz), 7.63 (4H, br.s, Ph-H), 6.51 (1H, d, J=16.2 Hz), 4.28 (2H, q, J=7.1 Hz, OCH₂CH₃),and 1.35 (3H, t, J=7.1 Hz, CH₃).

### 4-(Trifluoromethyl)cinnamyl alcohol (62)

¹H-NMR (270MHz, CDCl₃): δ = 7.57 (2H, d, J=8.2 Hz, Ph-H), 7.48 (2H, d, J=8.2 Hz, Ph-H), 6.67 (1H, d, J=16.0 Hz), 6.46 (1H, dt, J=16.0 and 5.3 Hz), 4.37 (2H, d, J=5.3 Hz, CH₂OH), and 1.66 (1H, s, OH).

### 3-(4-Trifluoromethylphenyl)-1-propanol (63)

¹H-NMR (270MHz, CDCl₃): δ = 7.53 (2H, d, J=7.9 Hz, Ph-H), 7.30 (2H, d, J=7.9 Hz, Ph-H), 3.66 (2H, t, J=6.5 Hz, CH₂OH), 2.76 (2H, t, J=7.8 Hz), 2.74 (1H, br.s, OH), and 1.89 (2H, m).

### [Reference Example 8]

8-[1,1,1-Tri(acetoxymethyl)methyl] aminocarbonyl-1-octanol (69) used for synthesizing glycosidated Product 28 was synthesized by the following method.

### [Reference Example 8-1]

### Synthesis of methyl 9-hydroxynonanoate (65).

To commercially available azelaic acid monomethyl ester (2.02 g, 10 mmol) was added thionyl chloride (2.0 ml), and the mixture was refluxed for 5 hours under nitrogen atmosphere. The reaction mixture was cooled to room temperature, and excess thionyl chloride was evaporated to give a residue containing acid chloride 64. This residue was dissolved in ether (10 ml) and added dropwise into a suspension of sodium borohydride-alumina complex (5.0 g) in ether (15 ml). After stirring for 12 hours at room temperature, the reaction mixture was filtered and was washed with ether. The filtrate was concentrated and resulting residue was purified by column chromatography over silica gel to give objective Compound 65 (1.59 g, Yield: 90%) as colorless oil. [see, Synthesis, 1978: 891].
¹H-NMR (270MHz, CDCl₃): δ = 3.67 (3H, s, CO₂Me), 3.62 (2H, t, J=6.6 Hz, CH₂OH), 2.31 (2H, t, J=7.4 Hz, CH₂CO), 2.22 (1H, br.s, OH), 1.70-1.50 (4H, m, CH₂ x 2), and 1.31 (8H, m).

### [Reference Example 8-2]

### Synthesis of methyl 9-(2-tetrahydropyranyloxy) nonanoate (66).

To a solution of methyl 9-hydroxynonate (65) (852 mg, 4.52 mmol) in dichloromethane (35 ml) were added dihydropyran (1.22 ml, 3.0 eq.) and pyridinium p-toluenesulfonate (PPTS, 352 mg, 0.3 eq.), and the mixture was stirred overnight at room temperature. The reaction mixture was mixed with water and extracted 3 times with dichloromethane. The organic layers were combined, washed with saturated aqueous sodium bicarbonate and brine, successively, dried over anhydrous sodium sulfate, filtered, and the solvent was evaporated in vacuo to give objective Compound 66 (1.37 g, quantitative) as colorless oil.
¹H-NMR (270MHz, CDCl₃): δ = 4.58 (1H, m, H-2 of THP), 3.88 (1H, m), 3.73 (1H, m), 3.67 (3H, s, OMe), 3.51 (1H, m), 3.38 (1H, m), 2.30 (2H, t, J=7.4 Hz, CH₂CO), 1.88-1.46 (10H, m), and 1.31 (8H, m).

### [Reference Example 8-3]

### Synthesis of 1-(2-tetrahydropyranyloxy)-8-[[1,1,1-tri(hydroxymethyl)methyl]aminocarbonyl]octane (67).

To a solution of methyl 9-(2-tetrahydropyranyl oxy)nonanoate (66) obtained by Reference Example 8-2 (4.52 mmol) in dimethyl sulfoxide (35 ml) were added tris(hydroxymethyl)aminomethane (TRIS, 1194 mg, 2.0 eq.) and potassium carbonate (1494 mg, 2.2 eq.), and the mixture was stirred at room temperature for 7.5 hours. The reaction mixture was diluted with water and extracted 3 times with ethyl acetate. The organic layers were combined, washed with saturated aqueous ammonium chloride and brine, successively, dried over anhydrous sodium sulfate, filtered, and the solvent was evaporated in vacuo to give objective Compound 67 (1.87 g, quantitative) as colorless oil. [see, J. Am. Chem. Soc., 112: 8458 (1990)].
¹H-NMR (270MHz, CDCl₃): δ = 6.53 (1H, s, NH), 5.40 (3H, br.s, OH x 3), 4.55 (1H, m, H-2 of THP), 3.87 (1H, m), 3.72 (1H, m), 3.53 (6H, s, CH₂OH x 3), 3.48 (1H, m), 3.36 (1H, m), 2.20 (2H, t, J=7.9 Hz, CH₂CO),1.85-1.42 (10H, m), and 1.29 (8H, m).

### [Reference Example 8-4]

### Synthesis of 1-(2-tetrahydropyranyloxy)-8-[[1,1,1-tri(acetoxymethyl)methyl]aminocarbonyl]octane (68).

To a solution of 1-(2-tetrahydropyranyloxy)-8-[[1,1,1-tri-(hydroxymethyl)methyl]aminocarbonyl]octane (67) obtained in Reference Example 8-3 (4.52 mmol) in pyridine (5.0 ml) was added acetic anhydride (5.0 ml, 14 eq.), and the mixture was stirred at room temperature for 2 hours. The reaction mixture was mixed with ice water and extracted 3 times with ethyl acetate. The organic layers were combined, washed with 1N-hydrochloric acid, saturated aqueous sodium bicarbonate and brine, successively, dried over anhydrous sodium sulfate, filtered, and the solvent was evaporated in vacuo to give objective Compound 68 (1.98 g, Yield: 90% from Compound 65 in 3 steps) as colorless oil.
¹H-NMR (270MHz, CDCl₃): δ = 5.92 (1, s, NH), 4.58 (1H, m, H-2 of THP), 4.44 (6H, s, CH₂OAc x 3), 3.87 (1H, m), 3.72 (1H, m), 3.50 (1H, m), 3.37 (1H, m), 2.15 (2H, t, J=7.4 Hz, CH₂CO), 2.09 (9H, s, OAc x 3), 1.85-1.42 (10H, m), and 1.31 (8H, m).

### [Reference Example 8-5]

### Synthesis of 8-[1,1,1-tri(acetoxymethyl)methyl] aminocarbonyl-1-octanol (69).

To a solution of 1-(2-tetrahydropyranyloxy)-8-[[1,1,1-tri- (acetoxymethyl)methyl]aminocarbonyl]octane (68) (1.72 g, 3.54 mmol) in methanol (20 ml) was added pyridinium p-toluenesulfonate (PPTS, 268 mg, 0.3 eq.), and the mixture was stirred overnight at room temperature. The reaction mixture was mixed with water and extracted 3 times with ethyl acetate. The organic layers were combined, washed with brine, dried over anhydrous sodium sulfate, filtered, and the solvent was evaporated in vacuo. The resulting residue was purified by column chromatography over silica gel (75 g) (eluant; chloroform: methanol = 100:3) to give objective Compound 69 (1.27 g, Yield: 89%) as colorless oil.
¹H-NMR (270MHz, CDCl₃): δ = 5.92 (1, s, NH), 4.44 (6H, s, CH₂OAc x 3), 3.63 (2H, t, J=6.6 Hz, CH₂OH), 2.16 (2H, t, J=7.6 Hz, CH₂CO), 2.09(9H, s, OAc x 3), 1.59 (4H, m), and 1.32 (8H, m).

### [Reference Example 9]

2-(3,4,5-Trimethoxyphenyl)-1-ethanol (71) utilized for synthesizing glycosidated Product 30 was synthesized as follows.

### [Reference Example 9-1]

### Synthesis of ethyl 3,4,5-trimethoxyphenylacetate (70).

To a solution of commercially available 3,4,5-trimethoxyphenylacetic acid (97% assay, 5.15 g, 22.1 mmol) in dry methanol (150 ml) was added dropwise trimethylsilyl chloride (7.0 ml, 2.5 eq.). After stirring overnight at room temperature, the reaction mixture was concentrated in vacuo. The resulting residue was purified by column chromatography over silica gel (200 g, eluant, hexane:ethyl acetate = 2:1) to give objective Compound 70 (5.27 g, Yield: 99%) as colorless oil. [See Synthesis, 1983, 201].
¹H-NMR (270MHz, CDCl₃): δ = 6.49 (2H, s, Ph-H), 3.85 (6H, s, OMe x 2), 3.82 (3H, s, OMe), 3.71 (3H, s, CO₂Me), and 3.56 (2H, s, CH₂).

### [Reference Example 9-2]

### Synthesis of 2-(3,4,5-trimethoxyphenyl)-1-ethanol (71).

To a solution of methyl 3,4,5-trimethoxyphenyl acetate (70) (5.36 g, 22.3 mmol) in dry tetrahydrofuran (150 ml) cooled to -40°C or lower with dry ice-acetonitrile was added dropwise a solution of diisobutylaluminium hydride in hexane (0.93M, 53.2 ml, 2.2 eq.). After stirring for 30 minutes under cooling, methanol was added dropwise to quench the reaction. The reaction mixture was mixed with 2N-hydrochloric acid (50 ml) and ethyl acetate (300 ml), stirred, and layers were separated. The organic layer was washed with brine, dried over anhydrous sodium sulfate, filtered, and the solvent was evaporated in vacuo. The resulting residue was purified by column chromatography over silica gel (150 g) (eluant; hexane:ethyl acetate = 1:1) to give objective Compound 71 (4.65 g, Yield: 98%) as colorless oil.
¹H-NMR (270MHz, CDCl₃): δ = 6.44 (2H, s, Ph-H), 3.85 (6H, s, OMe x 2), 3.82 (3H, s, OMe), 3.90-3.80 (2H, m, CH₂), 2.81 (2H, t, J=6.4 Hz, CH₂), and 1.53 (1H, br.s, OH).

The structural formulae of Compounds (61) to (71) in Reference Examples 7 to 9 are as follows.

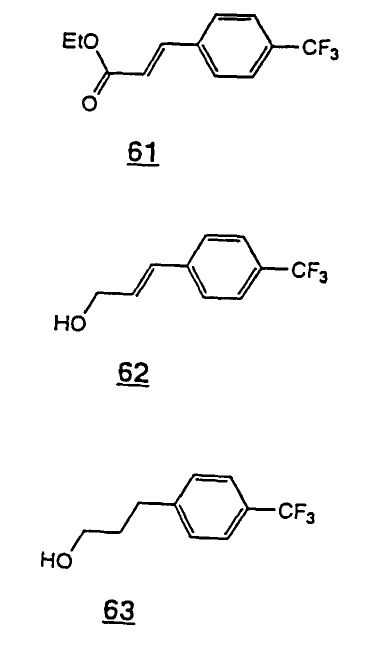

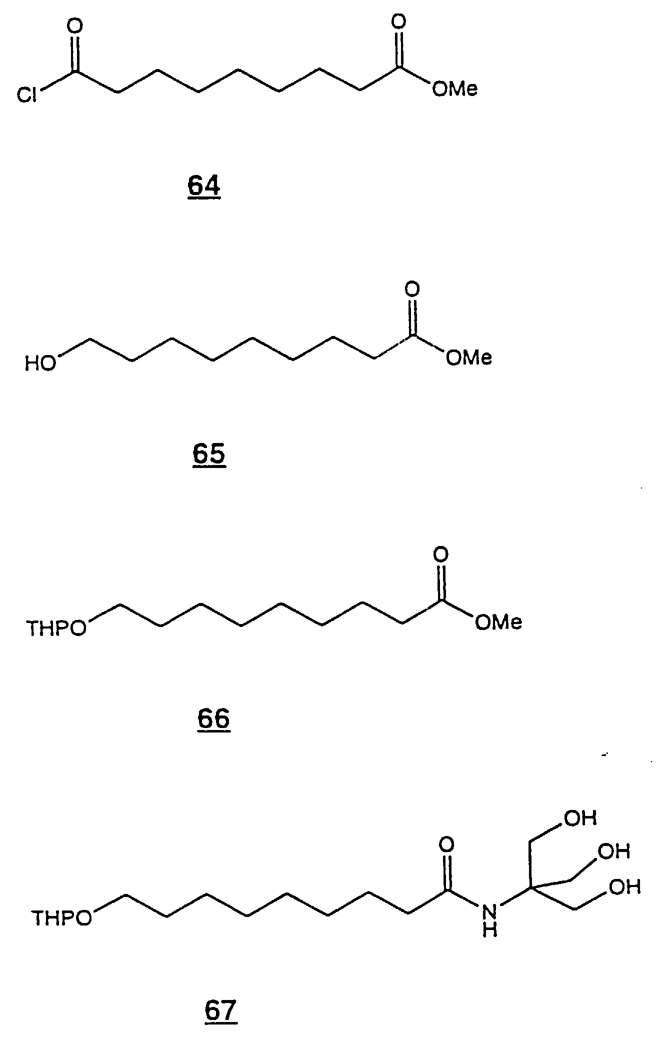

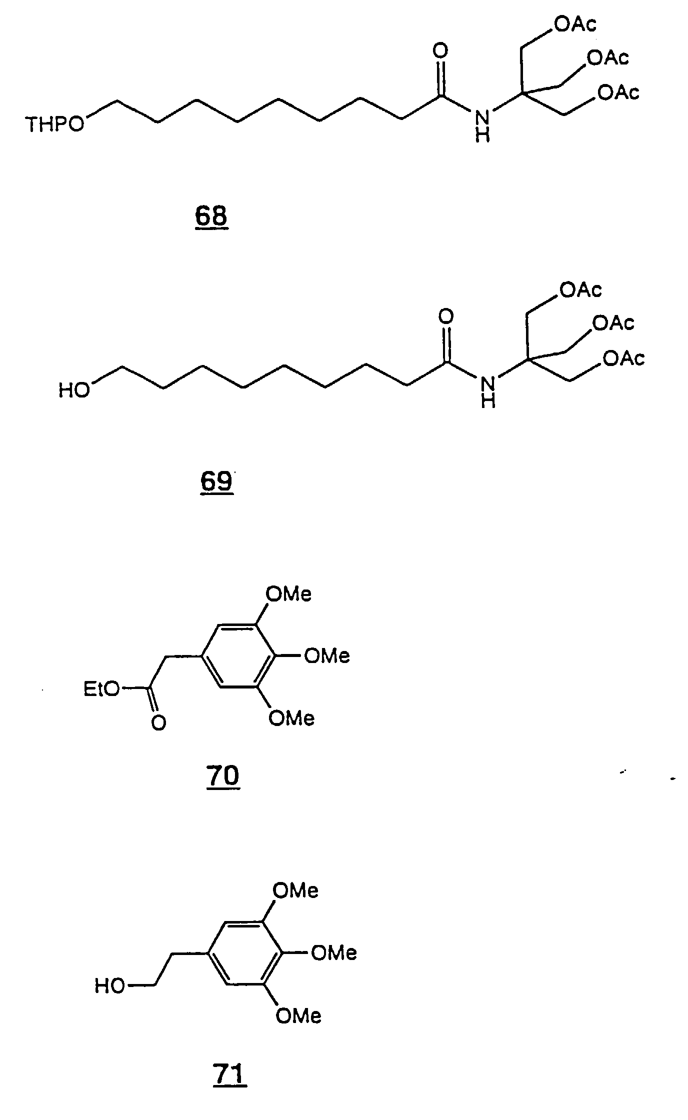

### Example 3

The structural formulae of Compounds (72) to (78) in Example 3 are as follows.

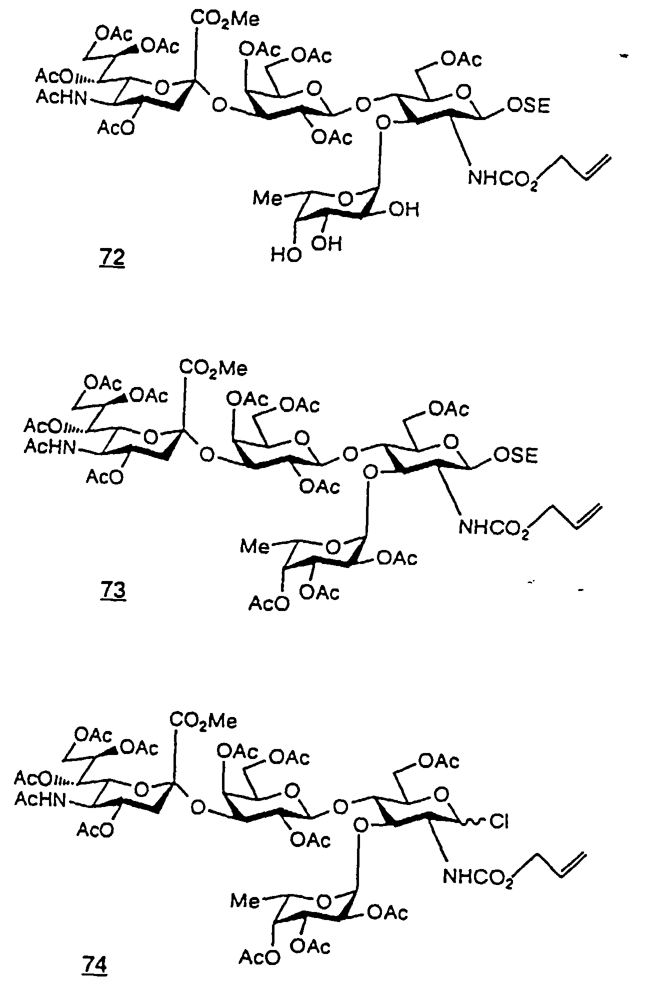

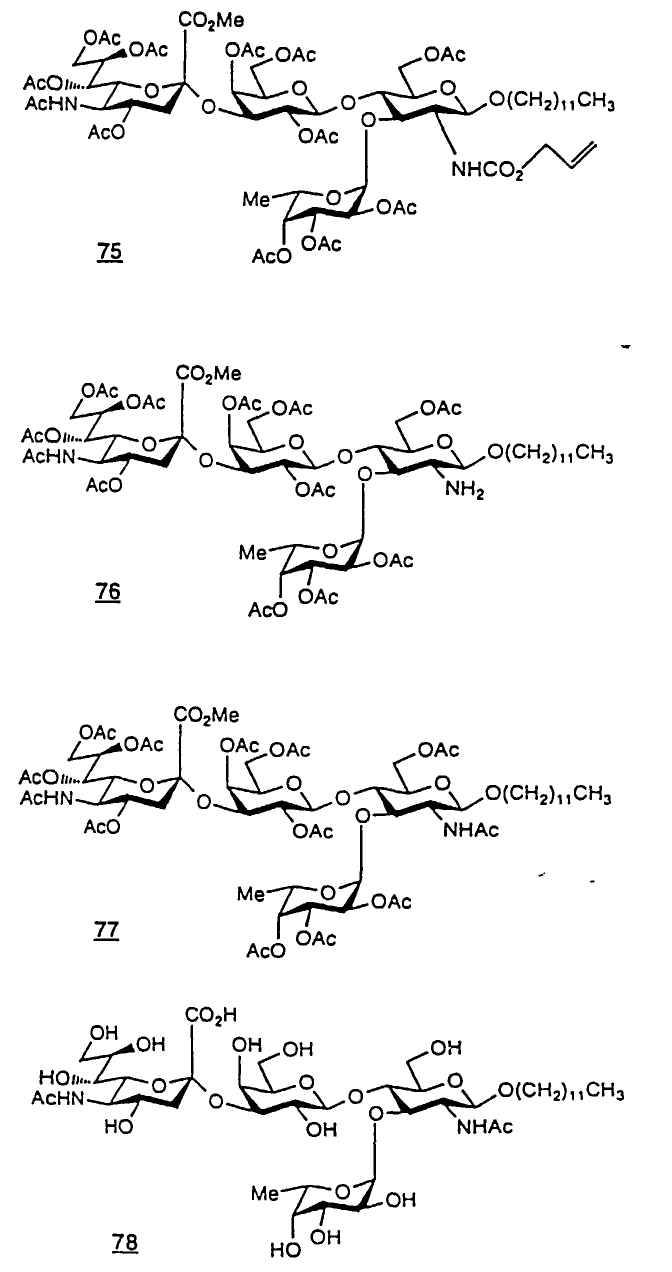

### [Example 3-1]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fucopyranosyl-(1→ 3)-O]-6-O-acetyl-2-N-allyloxycarbonyl-2-amino-2-deoxy-β-D-glucopyranoside) (72).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fucopyranosyl-(1→ 3)-O]-6-O-acetyl-2-amino-2-deoxy-β-D-glucopyranoside) (10) (2.00 g, 1.63 mmol) in dichloromethane (50 ml) were added sodium bicarbonate (683 mg, 8.13 mmol) and allyl chloroformate (491 mg, 4.07 mmol) at room temperature, and the mixture was stirred for 7 hours. After confirming the completion of reaction, methanol (25 ml) and pyridine (10 ml) were added, and the mixture was stirred for 15 minutes. The reaction mixture was concentrated to give a residue containing objective Compound 72 (theoretical amount: 2.14 g). This was used in the next reaction without further purification.

### [Example 3-2]

### Synthesis of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-6-O-acetyl-2-N-allyloxycarbonyl-2-amino-2-deoxy-β-D-glucopyranoside) (73).

To a solution of the residue obtained in Example 3-1 containing 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[α-L-fuco-pyranosyl-(1 →3)-O]-6-O-acetyl-2-N-allyloxycarbonyl-2-amino-2-deoxy-β-D-glucopyranoside (72) (theoretical amount: 2.14 g) in pyridine (25 ml) were added acetic anhydride (20 ml) and dimethylaminopyridine (100 mg) under ice cooling, and the mixture was stirred at room temperature for 12 hours. After confirming the completion of reaction, methanol (25 ml) was added dropwise to the mixture under ice cooling, and the mixture was stirred at room temperature for 30 minutes. The reaction mixture was concentrated, and the resulting residue was diluted with ethyl acetate and washed with aqueous copper(II) sulfate and brine, successively. The organic layer was dried over sodium sulfate, filtered, and the filtrate was concentrated. The residue was purified by silica gel chromatography to give objective Compound 73 as a pale yellow amorphous (1.41 g, Yield: 60% from Compound 10 in 2 steps).
¹H NMR (CDCl₃) δ = 5.91 (1H, m, H-2 of Alloc), 5.50-3.80 (30H, m), 3.86 (3H, s, CO₂Me), 3.70-3.45 (4H, m), 2.59 (1H, dd, J=4.2 and 12.5 Hz, H-3e of NeuAc), 2.20 (3H, s, OAc), 2.16 (3H, s, OAc), 2.14 (3H, s, OAc),2.13(3H, s, OAc), 2.09 (3H, s, OAc), 2.08 (3H, s, OAc), 2.07 (3H, s, OAc), 2.06 (3H, s, OAc), 2.05 (3H, s, OAc), 2.00 (3H, s, OAc), 1.95 (3H,s, OAc), 1.85 (3H, s, NAc), 1.69 (1H, t, J=12.5 Hz, H-3a of NeuAc), 1.20 (3H, d, J=6.6 Hz, Me of Fuc), 0.91 (2H, m, OCH₂CH₂Si), and -0.01 (9H, s, SiMe₂).

### [Example 3-3]

### Synthesis of [methyl (5-acetamido-3,5-dideoxy-4,7,8,9-tetra-O-acetyl-α-D-glycero-D-galacto-2-nonulopyrano sylonate)]-(2→3)-O-(2,4,6-tri-O-acetyl-β-D-galactopyranosyl )-(1→4)-O-[2,3,4-tri-O-acetyl-α-L-fucopyranosyl-(1→3)-O]-(6 -O-acetyl-2-N-allyloxycarbonyl-2-amino-2-deoxy-β-D-glucopyr anosyl)chloride (74).

To a solution of 2-(trimethylsilyl)ethyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-N-allyloxycarbonyl-2 -amino-2-deoxy-β-D-glucopyranoside) (73) (1.40 g, 0.973 mmol) in chloroform (25 ml) were added dichloromethyl methyl ether(440 µl, 4.86 mmol) and zinc chloride (27 mg, 0.198 mmol) at room temperature and the mixture was stirred for 9 hours with occasional addition of additional zinc chloride (30 mg after 2 hours, 30 mg after 6 hours) and dichloromethyl methyl ether (440 µl after 4 hours, 440 µl after 6 hours). After confirming the completion of reaction, the reaction mixture was concentrated to give a residue containing objective Compound 74 (theoretical amount: 1.32 g). This was used in the next reaction without further purification.

### [Example 3-4]

### Synthesis of dodecyl [methyl (5-acetamido-3,5-dideoxy-4,7,8,9-tetra-O-acetyl-α-D-glycero-D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-N-allyloxycarbonyl-2-amino-2-deoxy-β-D-glucopyranoside) (75).

To a solution of the residue obtained in Example 3-3 containing [methyl (5-acetamido-3,5-dideoxy-4,7,8,9-tetra-O-acetyl-α-D-glycero-D-galacto-2-nonulopyrano sylonate)]-(2→3)-O-(2,4,6-tri-O-acetyl-β-D-galactopyranosyl )-(1→4)-O-[2,3,4-tri-O-acetyl-α-L-fucopyranosyl-(1→3)-O]-(6-O-acetyl-2-N-allyloxycarbonyl-2-amino-2-deoxy-β -D-gluco-pyranosyl)chloride (74) (theoretical amount: 1.32 g) in dichloromethane (10 ml) were added Molecular Sieves 4A (660 mg) and tin(II) trifluoromethanesulfonate (607 mg, 1.46 mmol). To the resultant mixture was added dropwise a solution of 1-dodecanol (363 mg, 1.95 mmol) and tetramethylurea (170 mg, 1.46 mmol) in dichloromethane (50 ml), and the mixture was stirred for 3 hours. After confirming the completion of reaction, the reaction mixture was filtered through a pad of Celite and the filtrate was washed with saturated sodium bicarbonate and brine, successively. The organic layer was dried over magnesium sulfate, filtered, and the filtrate was concentrated. The resulting residue was purified by column chromatography over silica gel to give pale yellow amorphous objective Compound 75 (777 mg, Yield: 53% from Compound 73 in 2 steps).
¹H NMR (CDCl₃) δ = 5.91 (1H, m, H-2 of Alloc), 5.60-3.75 (30H, m), 3.86 (3H, s, CO₂Me), 3.62 (1H, dd, J=2.6 and 13.5 Hz), 3.70-3.30 (3H, m), 2.59 (1H, dd, J=3.6 and 12.2 Hz, H-3e of NeuAc), 2.25 (3H, s, OAc), 2.20 (3H, s, OAc), 2.16 (3H, s, OAc), 2.14 (3H, s, OAc), 2.13 (3H, s, OAc), 2.09 (3H, s, OAc), 2.07 (3H, s, OAc), 2.06 (3H, s, OAc), 2.05 (3H,s, OAc), 2.00 (3H, s, OAc), 1.95 (3H, s, OAc) , 1.85 (3H, s, NAc), 1.68 (1H, t, J=12.2 Hz, H-3a of NeuAc), 1.65-1.40 (2H, m, OCH₂CH₂(CH₂)₉CH₃), 1.35-1.15 (18H, OCH₂CH₂(CH₂)₉CH₃), 1.20 (3H, d, J=6.6 Hz, Me of Fuc), and 0.88 (3H, t, J=6.9 Hz, O(CH₂)₁₁CH₃).

### [Example 3-5]

### Synthesis of dodecyl [methyl (5-acetamido-3,5-dideoxy-4,7,8,9-tetra-O-acetyl-α-D-glycero-D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-amino-2-deoxy-β-D-glucopyranoside) (76).

To a solution of dodecyl [methyl (5-acetamido-3,5-dideoxy-4,7,8,9-tetra-O-acetyl-α-D-glycero-D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-N-allyloxycarbonyl-2-amino-2-deoxy-β-D-glucopyranoside) (75) (762 mg, 0.51 mmol) in tetrahydrofuran (10 ml) were added tetrakis(triphenylphosphine) palladium (500 mg) and polymethylhydrosiloxane (69 µl) at room temperature, and the mixture was stirred for 3.5 hours. After confirming the completion of reaction, the reaction mixture was diluted with dichloromethane and washed with water. The organic layer was dried over magnesium sulfate, filtered, and the filtrate was concentrated. The resulting residue was purified by column chromatography over silica gel to give objective Compound 76 as a pale yellow amorphous (573 mg, Yield: 80%).
¹H-NMR (270MHz, CDCl₃) δ = 5.55-3.00 (31H, m), 3.86 (3H, s, CO₂Me), 2.60 (1H, dd, J=4.0 and 12.5 Hz, H-3e of NeuAc), 2.21 (3H, s, OAc), 2.15(3H, s, OAc), 2.13 (3H, s, OAc), 2.12 (3H, s, OAc), 2.09 (3H, s, OAc), 2.07 (3H, s, OAc), 2.05 (3H, s, OAc), 2.04 (3H, s, OAc), 2.02 (3H, s, OAc), 2.00 (3H, s, OAc), 1.93 (3H, s, OAc), 1.85 (3H, s, NAc), 1.69 (1H,t, J=12.5 Hz, H-3a of NeuAc), 1.60-1.35 (2 H, m, OCH₂CH₂(CH₂)₉CH₃), 1.30-1.05 (18H, OCH₂CH₂(CH₂)₉CH₃), 1.19 (3H, d, J=6.6 Hz, Me of Fuc), and 0.88 (3H, t, J=6.9 Hz, O(CH₂)₁₁CH₃).

### [Example 3-6]

### Synthesis of dodecyl [methyl (5-acetamido-3,5-dideoxy-4,7,8,9-tetra-O-acetyl-α-D-glycero-D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(2-acetamido-6-O-acetyl-2-deoxy-β-D-glucopyranoside) (77).

To a solution of dodecyl [methyl (5-acetamido-3,5-dideoxy-4,7,8,9-tetra-O-acetyl-α-D-glycero -D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(6-O-acetyl-2-amino-2-deoxy-β-D-glucopyranoside) (76) (20.0 mg, 0.014 mmol) in dichloromethane (2.0 ml) were added pyridine (0.5 ml) and acetic anhydride (0.1 ml) at room temperature, and the mixture was stirred for 24 hours. After confirming the completion of reaction, methanol (0.1 ml) was added to the reaction mixture under ice cooling, and the mixture was stirred at room temperature for 30 minutes. The reaction mixture was concentrated, diluted with ethyl acetate, and washed with aqueous copper(II) sulfate and water successively. The organic layer was dried over sodium sulfate, filtered, and the filtrate was concentrated. The resulting residue was purified by column chromatography over silica gel to give objective Compound 77 as a pale yellow amorphous (14.9 mg, Yield: 73%).
¹H NMR (CDCl₃) δ = 5.69 (1H, d, J=7.5 Hz), 5.55-3.30 (29H, m), 3.83 (3H, s, CO₂Me), 2.56 (1H, dd, J=4.3 and 12.5 Hz, H-3e of NeuAc), 2.19 (3H, s, OAc), 2.14 (3H, s, OAc), 2.12 (3H, s, OAc), 2.11 (3H, s, OAc), 2.09 (3H, s, OAc), 2.06 (6H, s, OAc x 2), 2.04 (6H, s, O Ac x 2), 1.98 (3H, s, OAc), 1.93 (3H, s, OAc and NAc), 1.84 (3H, s, NAc), 1.67 (1H, t, J=12.5 Hz, H-3a of NeuAc), 1.51 (2H, m, OCH₂CH₂(CH₂)₉Me), 1.35-1.00 (18H, OCH₂CH₂(CH₂)₉Me), 1.17 (3H, d, J=6.3 Hz, Me of Fuc), and 0.86 (3H, t, J=6.4 Hz, O(CH₂)₁₁Me).

### [Example 3-7]

### Synthesis of dodecyl (5-acetamido-3,5-dideoxy-α-D-glycero- D-galacto-2-nonulopyranosylonic acid)-(2→3)-O-(β-D-galactopyranosyl)-(1→4)-O-[α-L-fucopyranosyl-(1→3)-O ]-(2-acetamido-2-deoxy-β-D-glucopyranoside) (78).

To a solution of dodecyl [methyl (5-acetamido-3,5-dideoxy-4,7,8,9-tetra-O-acetyl-α-D-glycero-D-galacto-2-nonulopyranosylonate)]-(2→3)-O-(2,4,6-tri-O-acetyl-β-D-galactopyranosyl)-(1→4)-O-[2,3,4-tri-O-acetyl-α-L-fucopyranosyl-(1→3)-O]-(2-acetamido-6-O-acetyl-2-deoxy-β-D-glucopyranoside) (77) (302 mg, 0.206 mmol) in methanol (30 ml) at room temperature was added 28% sodium methoxide solution (in methanol, 1.5 ml), and the mixture was stirred for 3 days. After confirming the consumption of starting material, water (15 ml) was added and the mixture was stirred at room temperature for 24 hours. After confirming the completion of reaction, the reaction mixture was neutralized with acidic ion exchange resin (DOWEX 50W-X8) and filtered. The filtrate was concentrated, the residue was purified by column chromatography over polyacrylamide gel, and fractions were lyophilized to give objective Compound 78 (88 mg, Yield: 43%) as white powder. Rf=0.57 (developed with a 12:10:3 mixture of chloroform, methanol and 15mM aqueous calcium chloride).
¹H NMR (D₂O) δ = 5.01 (1H, d, J=4.0 Hz, H-1 of Fuc), 4.50-4.40 (2H, m, H-1 of Gal and H-1 of GlcN), 4.00-3.30 (25H, m), 2.68 (1H, dd, J=4.3 and 12.5 Hz, H-3e of NeuAc), 1.95 (6H, s, NAc x 2), 1.71 (1H, t, J=12.5 Hz, H-3a of NeuAc), 1.45 (2H, m, OCH₂CH₂(CH₂)₉Me), 1.25-1.10 (18H, m, OCH₂CH₂(CH₂)₉Me), 1.08 (3H, d, J=6.3 Hz, Me of Fuc), and 0.77 (3H, t, J=6.6 Hz, O(CH₂)₁₁Me).
MS m/z (FAB+): Calcd. for C₄₃H₇₆O₂₃N₂Si: 988. Found: 989 (M+H⁺); 1011 (M+Na⁺).

### Example 4

The structural formulae of Compounds (79) to (88) in Example 4 are as follows.

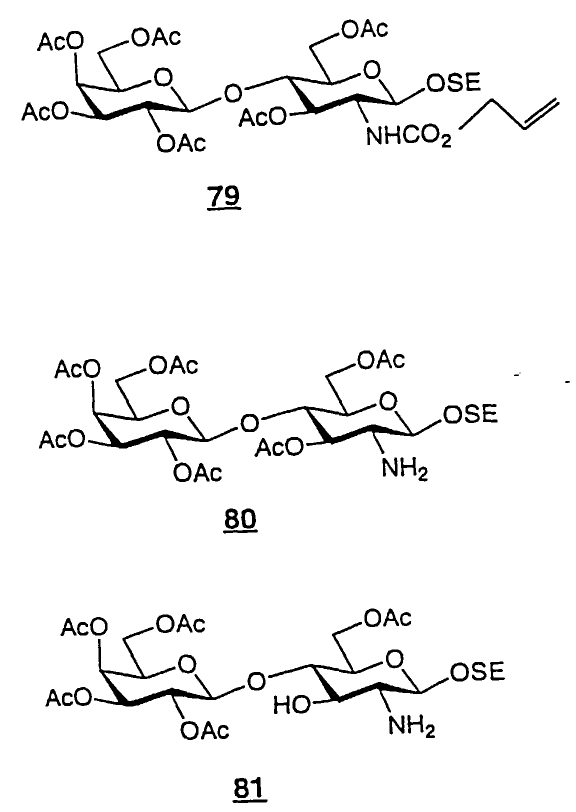

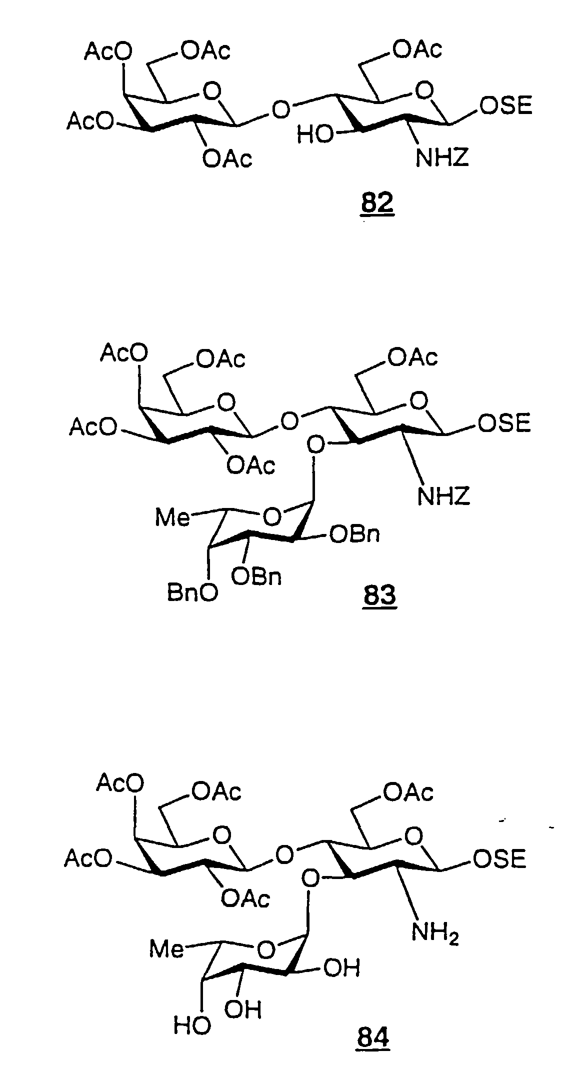

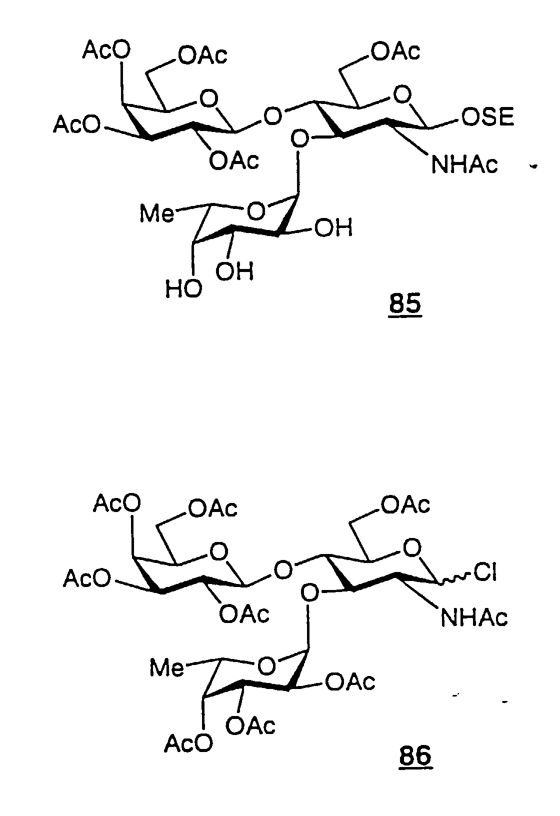

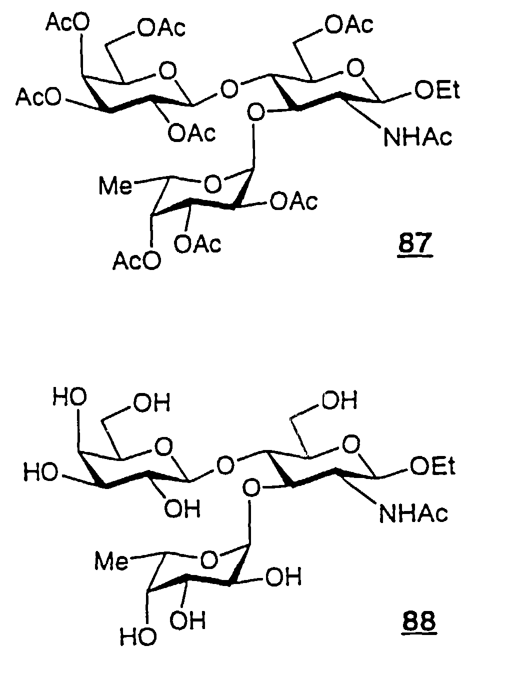

### [Example 4-1]

### Synthesis of 2-(trimethylsilyl)ethyl (2,3,4,6-tetra-O-acetyl-β-D-galactopyranosyl)-(1→4)-O-(2-N-allyloxycarbonyl-2-amino-2-deoxy-3,6-di-O-acetyl-β-D-gluco pyranoside) (79).

To a solution of 2-(trimethylsilyl)ethyl β-D-galactopyranosyl-(1→4)-O-(2-N-allyloxycarbonyl-2-amino-2-de oxy-β-D- glucopyranoside) (2) (613.7 mg, 1.17 mmol) in pyridine (5.0 ml) were added acetic anhydride (3.0 ml) and dimethylaminopyridine (50 mg) at room temperature, and the mixture was stirred for 12 hours. After confirming the completion of reaction, methanol (5.0 ml) was added dropwise to the ice-cooled mixture, and the mixture was stirred at room temperature for 30 minutes. The reaction mixture was concentrated, and the residue was diluted with ethyl acetate and washed with saturated aqueous copper(II) sulfate and brine, successively. The organic layer was dried over sodium sulfate and filtered, and the filtrate was concentrated. The resulting residue was purified by column chromatography over silica gel to give objective Compound 79 as a pale yellow amorphous (531.2 mg, Yield: 59%).
¹H NMR(CDCl₃) δ = 5.90 (1H, m, H-2 of Alloc), 5.40-5.10 (5H, m), 4.95 (1H, dd, J=3.3 and 10.3 Hz), 4.79 (1H, br.m), 4.60-4.40 (5H, m), 4.20-3.50 (9H, m), 2.14 (3H, s, OAc), 2.10 (3H, s, OAc), 2.06 (3H, s, OAc), 2.05 (3H, s, OAc), 2.04 (3H, s, OAc), 1.96 (3H, s, OAc), 1.00-0.85(2H, m, OCH₂CH₂SiMe₃), and -0.01 (9H, s, OCH₂CH₂SiMe₃).

### [Example 4-2]

### Synthesis of 2-(trimethylsilyl)ethyl (2,3,4,6-tetra-O-acetyl-β-D-galactopyranosyl)-(1→4)-O-(2-amino-2-deoxy-3,6-di-O-acetyl- β-D-glucopyranoside) (80).

To a solution of 2-(trimethylsilyl)ethyl (2,3,4,6-tetra-O-acetyl-β-D-galactopyranosyl)-(1→4)-O-(2-N-allyloxycarbonyl-2-amino-2-deoxy-3,6-di-O-acetyl-β-D-glucop yranoside) (79) (4.00 g, 5.14 mmol) in tetrahydrofuran (80 ml) were added polymethylhydrosiloxane (0.50 ml) and tetrakis(triphenylphosphine) palladium (TPAL) (297 mg, 0.257 mmol) under protection from light at room temperature, and the mixture was stirred. After 24 hours, additional TPAL (149 mg, 0.129 mmol) was added, and the mixture was stirred for 17.5 hours. After confirming the completion of the reaction, the reaction mixture was diluted with ethyl acetate and washed with brine. The organic layer was dried over sodium sulfate and filtered, and the filtrate was concentrated to give a residue containing objective Compound 80 (theoretical amount: 3.57 g). This was used in the next reaction without further purification.
¹H NMR(CDCl₃) δ = 5.40-4.90 (4H, m), 4.55-3.50 (14H, m), 2.14 (3H, s, OAc), 2.10 (3H, s, OAc), 2.09 (3H, s, OAc), 2.04 (3H, s, OAc), 2.02 (3H, s, OAc), 1.95 (3H, s, OAc), 1.00-0.85 (2H, m, OCH₂CH₂SiMe₃), and 0.01 (9H, s, OCH₂CH₂SiMe₃).

### [Example 4-3]

### Synthesis of 2-(trimethylsilyl)ethyl (2,3,4,6-tetra-O-acetyl-β-D-galactopyranosyl)-(1→4)-O-(6-O-acetyl-2-amino-2-deoxy- β-D-glucopyranoside) (81).

A solution of the residue produced in Example 4-2 containing 2-(trimethylsilyl)ethyl (2,3,4,6-tetra-O-acetyl-β-D-galactopyranosyl)-(1→4)-O-(2-amino-2-deoxy-3,6- di-O-acetyl-β-D-glucopyranoside) (80) (theoretical amount: 3.57 g) in methanol (180 ml) was stirred at room temperature for 3 days. After confirming the completion of reaction, the reaction mixture was concentrated to give a residue containing objective Compound 81 (theoretical amount: 3.35 g).

### [Example 4-4]

### Synthesis of 2-(trimethylsilyl)ethyl (2,3,4,6-tetra-O-acetyl-β-D-galactopyranosyl)-(1→4)-O-(6-O-acetyl-2-amino-2-N-benzyloxycarbonyl-2-deoxy-β-D-glucopyranoside) (82).

To a solution of the residue produced in Example 4-3 containing 2-(trimethylsilyl)ethyl (2,3,4,6-tetra-O-acetyl-β-D-galacto-pyranosyl)-(1→4)-O-(6-O -acetyl-2-amino-2-deoxy-β-D-glucopyranoside) (81) (theoretical amount: 3.57 g) in dichloromethane under ice cooling was added sodium bicarbonate (1.30 g, 15.4 mmol) and then benzyloxycarbonyl chloride (1.20 ml, 8.22 mmol) was added dropwise, and the mixture was stirred at room temperature. After 5 hours, additional sodium bicarbonate (0.60 g, 7.10 mmol) and benzyloxycarbonyl chloride (0.60 ml, 4.11 mmol) were added, and the mixture was stirred for 12 hours. After confirming the completion of reaction, the reaction mixture was diluted with dichloromethane and washed with water, saturated aqueous sodium bicarbonate and brine, successively. The organic layer was dried over sodium sulfate, filtered, and the filtrate was concentrated. The residue was purified by column chromatography over silica gel to give objective Compound 82 as a pale yellow amorphous (3.04 g, Yield: 75% from Compound 79 in 3 steps).
¹H NMR(CDCl₃) δ = 7.40-7.25 (5H, m, Ph-H), 5.37 (1H, d, J=3.6 Hz) , 5.30-3.70 (15H, m), 3.65-3.35 (3H, m), 3.28 (1H, m), 2.09 (3H, s, OAc), 2.06 (3H, s, OAc), 2.04 (3H, s, OAc), 2.03 (3H, s, OAc), 2 .00 (3H, s, OAc), 1.00-0.85 (2H, m, OCH₂CH₂SiMe₃), and 0.01 (9H, s, OCH₂CH₂SiMe₃).

### [Example 4-5]

### Synthesis of 2-(trimethylsilyl)ethyl (2,3,4,6-tetra-O-acetyl-β-D-galactopyranosyl)-(1→4)-O-[2,3,4-tri-O-benzyl-α-L-fucopyranosyl-(1→3)-O]-(6-O-acetyl-2-amino-2-N-benzyloxycarbonyl-2-deoxy- β-D-glucopyranoside) (83).

To a solution of 2-(trimethylsilyl)ethyl (2,3,4,6-tetra-O-acetyl-β-D-galactopyranosyl)-(1→4)-O-(6-O-acetyl-2-amino-2-N-benzyloxycarbonyl-2-deoxy-β-D-glucopyra noside) (82) (3.01 g, 3.83 mmol)in dichloroethane (15 ml) at room temperature were added Molecular Sieves 4A (1.50 g), tetramethylurea (2.75 ml, 23.0 mmol) and 2,3,4-tri-O-benzyl-L-fucopyranosyl fluoride (10.02 g, 23.0 mmol) (8), and the mixture was stirred for 2 hours. The reaction vessel was protected from light, cooled to -20°C, and then tin(II) chloride (2.90 g, 15.3 mmol) and silver perchlorate (3.20 g, 15.3 mmol) were added. The reaction mixture was warmed to room temperature over 60 minutes and stirred for 15 hours. After confirming the completion of reaction, the reaction mixture was diluted with ethyl acetate, filtered through a pad of Celite, and the filtrate was washed with water. The organic layer was dried over sodium sulfate, the filtrate was concentrated, and the resulting residue was purified by column chromatography over silica gel to give objective Compound 83 as a pale yellow amorphous (4.60 g).
¹H NMR(CDCl₃) δ = 7.45-7.15 (20H, m, Ph-H), 5.40-3.35 (3H, m), 2.09 (3H, s, OAc), 2.05 (3H, s, OAc), 2.01 (3H, s, OAc), 2.00 (6H, s, OAc x 2), 1.99 (3H, s, OAc), 1.14 (3H, d, J=6.6 Hz, Me of Fuc), 0.95-0.75 (2H, m, OCH₂CH₂SiMe₃), and -0.03(9H, s, OCH₂CH₂SiMe₃).

### [Example 4-6]

### Synthesis of 2-(trimethylsilyl)ethyl (2,3,4,6-tetra-O-acetyl-β-D-galactopyranosyl)-(1→4)-O-[α-L-fucopyranosyl-(1→3)-O]-(6-O-acetyl-2-aimino-2-deoxy-β-D-glucopyranoside) (84).

To a solution of 2-(trimethylsilyl)ethyl (2,3,4,6-tetra-O-acetyl-β-D-galactopyranosyl)-(1→4)-O-[2,3, 4-tri-O-benzyl-α-L-fucopyranosyl-(1→3)-O]-(6-O-acetyl-2-amino-2-N-benzyloxycarbonyl-2- deoxy-β-D-glucopyranoside) (83) (503 mg, 0.42 mmol) in ethanol (25 ml) were added ammonium formate (1.0 g) and 10% Pd-C (wet, 1.0 g), and the mixture was heated to reflux. After 3 hours, the same amounts of additional ammonium formate and 10% Pd-C were added, and the mixture was refluxed for further 4 hours. After confirming the completion of reaction, the reaction mixture was filtered through a pad of Celite and the filtrate was concentrated to give objective Compound 84 as a colorless amorphous (250 mg, Yield: 75% from Compound 82 in 2 steps).

### [Example 4-7]

### Synthesis of 2-(trimethylsilyl)ethyl (2,3,4,6-tetra-O-acetyl-β-D-galactopyranosyl)-(1→4)-O-[2,3,4-tri-O-acetyl-α-L-fucopyranosyl-(1→3)-O]-(6-O-acetyl-2-deoxy-2-acetamido-β-D-glucopyranoside) (85).

To a solution of 2-(trimethylsilyl)ethyl (2,3,4,6 -tetra-O-acetyl-β-D-galactopyranosyl)-(1→4)-O-[α-L-fucopyra nosyl-(1→3)-O]-(6-O-acetyl-2-amino-2-deoxy-β-D-glucopyrano side) (84) prepared in Example 4-6 (807 mg, 1.01 mmol) in pyridine (20 ml) were added acetic anhydride (10 ml) and dimethylaminopyridine (35 mg), and the mixture was stirred for 3 days. After confirming the completion of reaction, the reaction mixture was concentrated and the residue was diluted with ethyl acetate. The solution was washed with saturated aqueous sodium bicarbonate, and the organic layer was dried over sodium sulfate, filtered and concentrated. The resulting residue was purified by column chromatography over silica gel to give objective Compound 85 as a pale yellow amorphous (724 mg, Yield: 74%).
¹H NMR(CDCl₃) δ = 5.52 (1H, d, J=8.3 Hz, NH), 5.42-5.36 (3H, m), 5.19 (1H, dd, J=3.3 and 10.9 Hz), 5.13-4.95 (3H, m), 4.85 (1H, m), 4.67 (1H, d, J=7.3 Hz), 4.61-4.44 (3H, m), 4.31-4.10 (3H, m), 3.9 1-3.78 (3H, m), 3.57-3.44 (3H, m), 2.19 (3H, s, OAc), 2.14 (3H, s, OAc), 2.13 (3H, s, OAc), 2.10 (3H, s, OAc), 2.08 (3H, s, OAc), 2.06 (3H, s, OAc), 2.04 (3H, s, OAc), 1.97 (6H, s, OA and NAc), 1.21 (3H, d, J=6.6 Hz, Me of Fcu), 0.96-0.84 (2H, m, OCH₂CH₂SiMe₃), and -0.01 (9H, s, OCH₂CH₂SiMe₃).

### [Example 4-8]

### Synthesis of (2,3,4,6-tetra-O-acetyl-β-D-galactopyranosyl)-(1→4)-O-[2,3,4-tri-O-acetyl-α-L-fucopyra nosyl-(1→3)-O]-(6-O-acetyl-2-deoxy-2-acetamido-β-D-gluco pyranosyl) chloride (86).

To a solution of 2-(trimethylsilyl)ethyl (2,3,4,6 -tetra-O-acetyl-β-D-galactopyranosyl)-(1→4)-O-[2,3,4-tri-O-acetyl-α-L-fucopyranosyl-(1→3)-O]-(6-O-acetyl-2-deoxy-2-acetamido-β-D- glucopyranoside) (85) (687 mg, 0.71 mmol) in chloroform (70 ml) were added dichloromethyl methyl ether (0.32 ml, 3.54 mmol) and zinc chloride(58 mg, 0.43 mmol) at room temperature, and the mixture was stirred for 5 hours. After confirming the completion of reaction, the reaction mixture was concentrated to give a residue containing objective Compound 86 (theoretical amount: 628 mg). This was used in the next reaction without further purification.

### [Example 4-9]

### Synthesis of ethyl (2,3,4,6-tetra-O-acetyl-β-D-galactopyranosyl)-(1→4)-O-[2,3,4-tri-O-acetyl-α-L-fucop yranosyl-(1→3)-O]-(6-O-acetyl-2-deoxy-2-acetamido-β-D-gluco pyranoside) (87).

To a solution of the residue obtained in Example 4-8 containing (2,3,4,6-tetra-O-acetyl-β-D-galacto pyranosyl)-(1→4)-O-[2,3,4-tri-O-acetyl-α-L-fucopyranosyl-(1 →3)-O]-(6-O-acetyl-2-deoxy-2-acetamido-β-D-glucopyranosyl) chloride (86) (theoretical amount: 628 mg) in dichloromethane were added Molecular Sieves 4A (1.44 g) and tin(II) trifluoromethanesulfonate (887 mg, 2.13 mmol). To this mixture was added dropwise at room temperature a solution of ethanol (0.20 ml, 3.14 mmol) and tetramethylurea (0.26 ml, 2.17 mmol) in dichloromethane (10 ml). After 8 hours, the same amounts of tin(II) trifluoromethanesulfonate, ethanol and tetramethylurea were added, and the mixture was stirred for 60 hours. After confirming the completion of reaction, the reaction mixture was mixed with saturated aqueous sodium bicarbonate (10ml), filtered through a pad of Celite, and the filtrate was washed with saturated aqueous sodium bicarbonate. The organic layer was dried over sodium sulfate, filtered, and the filtrate was concentrated to give residue, which was purified by column chromatography over silica gel to give objective Compound 87 as a pale yellow amorphous (219 mg, Yield: 35% from Compound 85 in 2 steps).
¹H NMR(CDCl₃) δ = 5.63 (1H, d, J=8.6 Hz, NH), 5.43-5.34 (3H, m), 5.21(1H, dd, J=3.3 and 10.9 Hz), 5.14-4.96 (3H, m), 4.84 (1H, m), 4.68 (1H, d, J=7.6 Hz), 4.60 (1H, dd, J=2.6 and 11.8 Hz), 4.53-4.46 (2H, m), 4.29 (1H, dd, J=7.6 and 11.6 Hz), 4.18-4.12 (2H, m), 3.90-3.79 (3H, m), 3.65-3.46 (3H, m), 2.20 (3H, s, OAc), 2.16 (3H, s, OAc), 2.14 (3H, s, OAc), 2.11 (3H, s, OAc), 2.08 (3H, s, OAc), 2.07 (3H, s, OAc), 1.98 (6H, s, OAc x 2), 1.97 (3H, s, NAc), 1.21 (3 H, d, J=6.6 Hz, Me of Fuc), and 1.17 (3H, t, J=7.3 Hz, OCH₂CH₃).

### [Example 4-10]

### Synthesis of ethyl(β-D-galactopyranosyl)-(1→4)-O-[α-L-fucopyranosyl-(1→3)-O]-(2-deoxy-2-acetamido-β-D-glucop yranoside) (88).

To a solution of ethyl (2,3,4,6-tetra-O-acetyl-β-D-galactopyranosyl)-(1→4)-O-[2,3,4-tri-O-acetyl-α-L-fucopyranosyl-(1→3)-O]-(6-O-acetyl-2-deoxy-2-acetamido-β-D-glucopyranoside) (87) prepared in Example 4-9 (205 mg, 0.229 mmol) in methanol (6 ml) at room temperature was added 28% sodium methoxide solution (in methanol, 62 µl), and the mixture was stirred for 12 hours. After confirming the completion of reaction, the reaction mixture was neutralized with acidic ion exchange resin (DOWEX 50W-X8), filtered, and the filtrate was concentrated. The resulting residue was purified by column chromatography over polyacrylamide gel, and lyophilized to give the objective Compound 88 (123 mg, Yield: 96%) as white powder.
¹H-NMR(270MHz, D₂O): δ = 4.99 (1H, d, J=4.0 Hz), 4.45 (1H, d, J=7.6 Hz), 4.34 (1H, d, J=7.6 Hz), 3.91-3.35 (18H, m), 1.92 (3H, s, NHAc), 1.06 (3H, d, J=7.3 Hz, Me of Fuc), 1.05 (3H, t, J=7.3 Hz, OCH₂CH₃).

### [Experiment 1]

To each well of a 96 well plate was added a PBS solution containing 0.1% gelatine at 50 µl/well, and after 15 minutes at room temperature, the solution was removed with a Pasteur pipette. On the coated 96 well plate, human umbilical vein endothelial cells (HUVEC, 5th generation) were cultured until confluent. The culture medium was removed with a Pasteur pipette and the MEM medium containing 45 U/ml of IL-1β was added at 100 µl/well, and incubated for 4 hours. On the same plate, wells containing the MEM medium without IL-1β were prepared and similarly incubated for 4 hours.

Blood was collected from a healthy human using heparin as an anticoagulant. In a 50 ml-tube was placed 15 ml of neutrophil·monocyte fraction, and 25 ml of whole blood was gently layered. The brake function was released and centrifuged at 1700 rpm for 30 minutes at room temperature, and the neutrophil layer was collected and transferred into a 50 ml tube containing 30 ml of HBSS solution. The brake function was recovered and centrifuged at 3000 rpm for 3 minutes at room temperature, the supernatant was removed, and 30 ml of HBSS was added for resuspension. This procedure was repeated twice. The neutrophils were counted using hemocytometer, adjusted to 5×10⁶ cells/ml with the HBSS solution, charged in a 15 ml tube, mixed with anti-CD18 antibody at final concentration of 10 µg/ml, and incubated for 1 hour with occasional gentle swirling.

MEM medium was removed from the HUVEC plate with a Pasteur pipette, a compound of the present invention diluted with MEM medium at 50 µl/well was added, and incubated for 20 minutes.

The neutrophils treated with anti-CD18 antibody were added to the HUVEC plate at 25 µl/well, and incubated for 5 minutes.

The plate was taken out of the CO₂ incubator, the solution in the plate was discarded by inverting the plate, and the plate was washed gently 4-times with the MEM medium (containing 2% FCS) at 200 µl/well to remove nonadhesive neutrophils. An 8-channel multipipetter was used for this washing procedure and the washing solution was added to wells along the upper wall and discarded by inverting the plate.

After discarding the wash solution, a citrate solution containing 0.1% NP-40 at room temperature was added at 50 µl/well, and kept at room temperature for 5 to 10 minutes. The substrate solution (4 mg OPDA, 4 µl 30% H₂O₂/4 ml citrate solution) at room temperature was added at 50 µl/well, protected from light with aluminum foil, allowed to develop color for 5 to 20 minutes, and the reaction was terminated by adding 4N-H₂SO₄ at 50µl/well. The substrate solution was used within 30 minutes after prepartion.

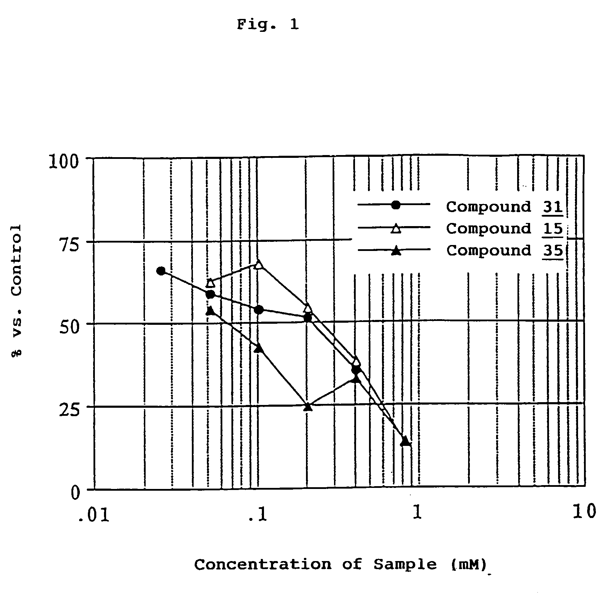

The OD490 of each well was determined, and the OD490 value of wells containing MEM medium without IL-1β was subtracted from that of each well. The value found in wells containing none of the compounds of the present invention was set to be 100% and the growth inhibiting activity of the compound of the invention was assessed as shown in Figure 1.

The materials for the experiment were obtained from the following sources:
HUVEC : Dainippon Seiyaku. MEM (Eagle Minimum Essential Medium) : Nissui Seiyaku. anti-CD18 antibody : CAMFOLIO Corp. L130, neutrophil·monocyte fraction : ICN flow Corp. PBS (Dulbecco's Phosphate Buffered Saline) : GIBCO Corp. HBSS solution : HBSS + 10mM-HEPES. HBSS (Hanks Balanced Salt Solution) : GIBCO Corp. OPDA (Ortho-phenylenediamine) : Sigma Corp.

Unless otherwise specified, MEM medium contained 10% FCS.

A compound of the invention was adjusted to 10mM with DPBS, and the pH was adjusted to 7.0-7.4 with 1N-NaOH.

The citrate solution was prepared by dissolving 2.33 g of citric acid and 9.20 g of Na₂HPO₄·12H₂O in 500 ml of desalted water.

Compounds (15), (31) and (35) of the invention inhibited the adhesion of HUVEC to neutrophils by 50% or more at a concentration of 0.05-0.5mM.

### [Experiment 2]

The DPBS solution containing rsE-selectin (3 µg/ml) was added to a 96 well plate at 50 µl/well, while wells filled with DPBS without rsE-selectin (non-specific adhesion control) were prepared on the same plate, and the plate was kept at room temperature for 3 hours. Then wells were washed 3 times with a DPBS/BSA solution at 200 µl/well, and DPBS/BSA solution was added at 200 µl/well, and the plate was kept at room temperature for 1 hour. After removing DPBS/BSA solution, a medium containing NWB or NWB plus a compound of the invention was added at 40 µl/well.

Frozen HL-60 cells were thawed promptly at 37°C, and washed with NWB cooled to 4°C 3 times by centrifugation at 1500rpm × 5 min. The cells were adjusted with NWB to 1×10⁷ cells/ml, added to the plate at 20 µl/well, and the plate was kept at room temperature for 15 min.

The plate was set on a plate washer to remove nonadhesive HL-60 cells. The plate washer was set at slow mode, cycle 3 (washing 3 times), soak 0 sec., 12 rows (along vertical direction), and volume of NWB for 1 washing 200 µl/well at room temperature.

A citrate solution containing 0.1% NP-40 at room temperature was added at 50 µl/well, and the plate was kept at room temperature for 5 minutes. The substrate solution (4 mg OPDA, 4µl 30% H₂O₂/4 ml citrate) was added at 50 µl/well, protected from light with aluminum foil, allowed to develop color for 5 to 20 minutes, and the reaction was terminated by adding 4N-H₂SO₄ at 50µl/well. The substrate solution was used within 30 minutes after preparation.

The OD490 of each well was determined, and the OD490 value of the well for nonspecific adhesion was subtracted from the value of each well. The value found in a well containing only the medium was used as a control (100%) and the amount of adhesion in wells containing Compounds (15), (31), (33)-(40) or (41) of the invention was calculated by percent in terms of the values of the control. By drawing inhibition curves, 50% inhibition concentration (IC₅₀) was determined. The results are shown in Table 1.

**TABLE 1**

| Compound | IC₅₀ (mM) |
|---|---|
| 15 | 0.11 |
| 31 | 0.11 |
| 33 | 0.21 |
| 34 | 0.11 |
| 35 | 0.05 |
| 36 | 0.11 |
| 37 | 0.11 |
| 38 | 0.15 |
| 39 | 0.15 |
| 40 | 0.07 |
| 41 | 0.11 |

The materials for the experiment were obtained from the following sources. rsE-selectin: producible by the method described in J. C. Poulson, J. Am. Chem. Soc., 117: 66-79 (1995). The sample of rsE-selectin used in the experiments was kindly supplied by the author. HL-60 : ATCC Corp. DPBS (Dulbecco Phosphate Buffered Saline) : GIBCO Corp. BSA (Bovine Serum Albumin) : Sigma Corp. HBSS (Hanks Balanced Salt Solution) : GIBCO Corp. OPDA (Ortho-phenylenediamine) : Sigma Corp. RPMI 1640 medium : GIBCO Corp. Bovine Fetal Serum : GIBCO Corp. Plate : Immulon. 2. (flat bottomed) : Dynatech Laboratories. Plate washer ; Microplate Washer : Molecular Devices Corp.

A compound of the present invention was adjusted to 100mM with DPBS, and pH was adjusted to 7.1-7.4.

HL-60 was cultured in RPMI 1640 medium supplemented with 10% Bovine Fetal Serum. Using a cell freezing solution consisting of 80% RPMI 1640 medium + 10% Bovine Fetal Serum + 10% DMSO, HL-60 was frozen at 1.5 × 10⁷ cells per a tube according to a conventional method, and stocked at -80°C. This was used under sterile condition.

Composition of DPBS/BSA was DPBS + 1% BSA.

Composition of NWB was HBSS + 10mM-HEPES + 0.2%glucose + 1%BSA+ 1mM-CaCl₂. These two solutions are prepared under sterile condition.

The citrate solution was prepared by dissolving 2.33 g of citric acid and 9.20 g of Na₂HPO₄·12H₂O in 500 ml of desalted water.

Compound (15), (31), (33)-(40) or (41) of the invention inhibited 50% of the adhesion of rsE-selectin to HL-60 at concentrations listed in Table 1.

### BRIEF EXPLANATION OF FIGURE

Figure 1 shows the adhesion inhibiting activity of Compounds (15), (31), and (35) of the present invention in several concentrations. The adhesion inhibiting activity is shown by percentage (%) as 100% for wells containing no sample (control).

## Claims

1. A Lewis X derivative represented by the general formula: 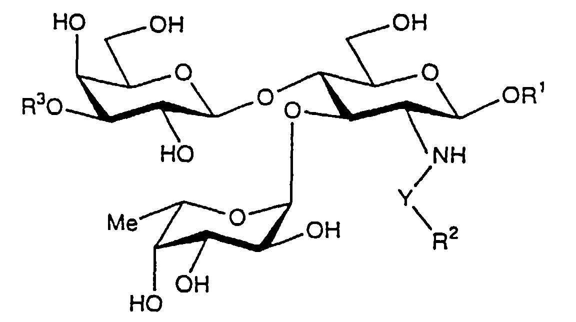 [wherein R¹ is C₁-C₁₈ alkyl, aryl, or aryl C₁-C₁₂ alkyl having at least one or more of substituents X as defined below.
When R¹ has two or more of substituents X, they may be different from each other.
Substituent X is selected from a group consisting of halogen, trifluoromethyl, hydroxy, C₁-C₁₈ alkoxy, aryloxy, aryl C₁-C₆ alkyloxy, amino, aryl C₁-C₆ alkylamino, mono(C₁-C₁₈ alkyl)amino, di(C₁-C₁₈ alkyl)amino, (C₁-C₁₈ alkyl)(aryl C₁-C₆ alkyl)amino, C₁-C₁₈ alkanoylamino, aroylamino, mono(C₁-C₁₈ alkyl)carbamoyl, di(C₁-C₁₈ alkyl)carbamoyl, aryl C₁-C₆ alkylcarbamoyl, (C₁-C₁₈ alkyl)(aryl C₁-C₆ alkyl)carbamoyl, arylcarbamoyl, C₁-C₁₈ alkanoyl, aroyl, C₁-C₁₈ alkylthio, arylthio, C₁-C₁₈ alkylsulfonyl, arylsulfonyl, cyano, and nitro.
The substituent X as defined above may be further substituted on its alkyl chain or aryl ring once or twice by the substituent as defined above.
Y is C(O), SO₂, C(O)NH, C(O)O or C(O)S;
R² is aryl, substituted aryl, or aryl C₁-C₆ alkyl; and
R³ is hydrogen or a group represented by the general formula: 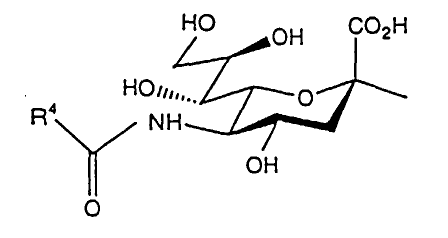 [wherein R⁴ is methyl or hydroxymethyl]], or a salt thereof.

2. A Lewis X derivative as claimed in Claim 1, wherein R¹ is C₁-C₁₈ alkyl having at least one or more of substituents X, or a salt thereof.

3. A Lewis X derivative as claimed in Claim 1, wherein R¹ is phenyl having at least one or more of substituents X, or a salt thereof.

4. A Lewis X derivative as claimed in Claim 1, wherein R¹ is phenyl C₁-C₁₂ alkyl having at least one or more of substituents X, or a salt thereof.

5. A Lewis X derivative as claimed in any one of Claims 1 to 4, wherein the substituent X is hydroxyl, or a salt thereof.

6. A Lewis X derivative as claimed in any one of Claims 1 to 4, wherein the substituent X is C₁-C₁₈ alkoxy, or a salt thereof.

7. A Lewis X derivative as claimed in any one of Claims 1 to 4, wherein the substituent X is aryloxy, or a salt thereof.

8. A Lewis X derivative as claimed in any one of Claims 1 to 7, wherein Y is C(O), or a salt thereof.

9. A Lewis X derivative as claimed in any one of Claims 1 to 8, wherein R² is aryl, or a salt thereof.

10. A Lewis X derivative as claimed in Claim 9, wherein R² is phenyl or naphthyl, or a salt thereof.

11. A Lewis X derivative represented by the general formula: 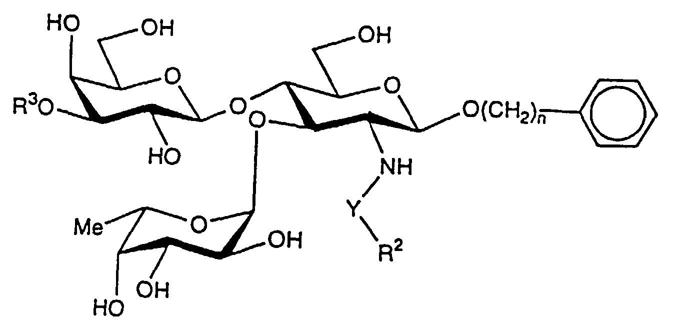 [wherein Y is C(O), SO₂, C(O)NH, C(O)O or C(O)S;
R² is aryl, substituted aryl or aryl C₁-C₆ alkyl;
R³ is hydrogen or a group represented by the general formula: 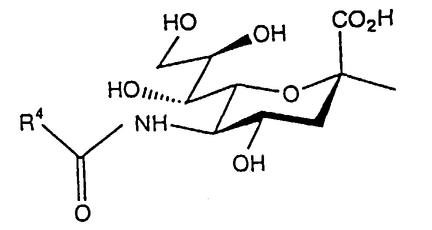 [wherein R⁴ is methyl or hydroxymethyl]; and
n is an integer of 2 to 6], or a salt thereof.

12. A compound represented by the general formula: 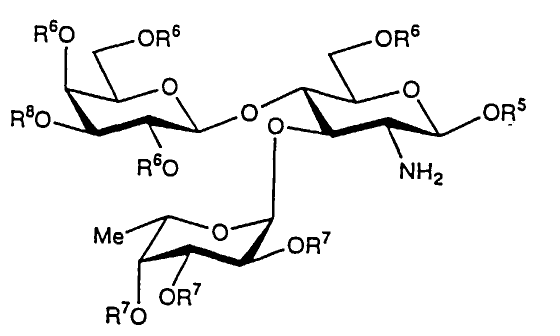 [wherein R⁵ is 2-tri(C₁-C₄ alkyl/phenyl)silylethyl;
R⁶ and R⁷ are each hydrogen, C₁-C₆ alkanoyl or aroyl; and
R⁸ is hydrogen, C₁-C₆ alkanoyl, aroyl or a group represented by the general formula: 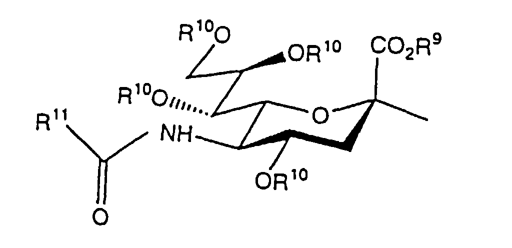 [wherein R⁹ is hydrogen or C₁-C₆ alkyl;
R¹⁰ is hydrogen, C₁-C₆ alkanoyl or aroyl; and
R¹¹ is methyl, hydroxymethyl, C₁-C₆ alkanoyloxymethyl or aroyloxymethyl]], or a salt thereof.

13. A compound represented by the general formula: 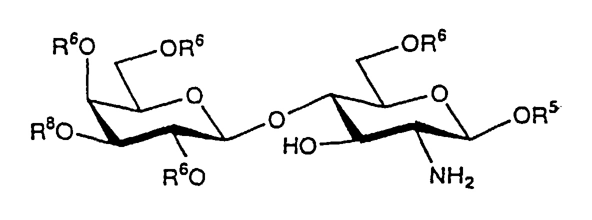 [wherein R⁵ is 2-tri(C₁-C₄ alkyl/phenyl)silylethyl;
R⁶ is hydrogen, C₁-C₆ alkanoyl or aroyl; and
R⁸ is hydrogen, C₁-C₆ alkanoyl, aroyl or a group represented by the general formula: 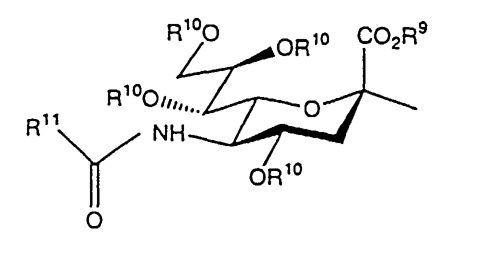 [wherein R⁹ is hydrogen or C₁-C₆ alkyl;
R¹⁰ is hydrogen, C₁-C₆ alkanoyl or aroyl ; and
R¹¹ is methyl, hydroxymethyl, C₁-C₆ alkanoyloxy methyl or aroyloxymethyl]], or a salt thereof.

14. A process for preparing a Lewis X derivative as defined hereinafter, which process comprises appropriately modifying the amino group of a compound as claimed in Claim 12 to give a compound represented by the general formula: 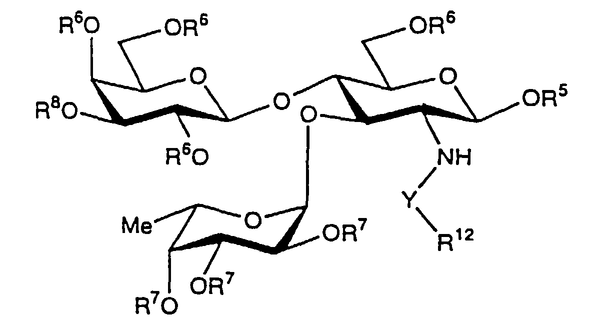 [wherein R⁵ is 2-tri(C₁-C₄ alkyl/phenyl)silylethyl;
R⁶ and R⁷ are each hydrogen, C₁-C₆ alkanoyl or aroyl;
R⁸ is hydrogen, C₁-C₆ alkanoyl, aroyl or a group represented by the general formula: 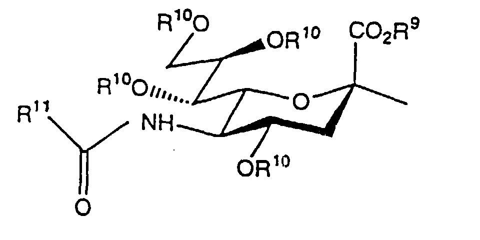 [wherein R⁹ is hydrogen or C₁-C₆ alkyl;
R¹⁰ is hydrogen, C₁-C₆ alkanoyl or aroyl; and
R¹¹ is methyl, hydroxymethyl, C₁-C₆ alkanoyloxymethyl or aroyloxymethyl]
Y is C(O), SO₂, C(O)NH, C(O)O or C(O)S; and
R¹² is C₁-C₆ alkyl, aryl, substituted aryl, or aryl C₁-C₆ alkyl], then subjecting the resultant compound to O-acylation (together with esterification, when R⁹ is hydrogen) (this O-acylation is not necessary, if none of R⁶, R⁷ and R⁸ is hydrogen and R⁸ has no unprotected hydroxy) to give a compound represented by the general formula: 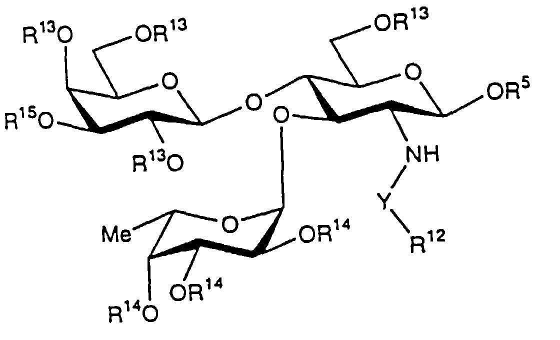 [wherein R⁵, Y and R¹² are as defined above;
R¹³ and R¹⁴ are each C₁-C₆ alkanoyl or aroyl; and
R¹⁵ is C₁-C₆ alkanoyl, aroyl or a group represented by the general formula: 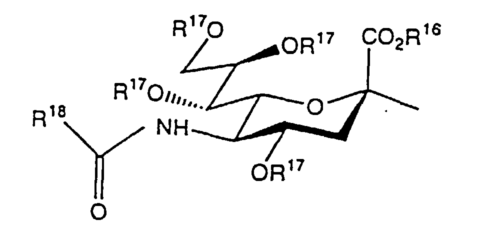 [wherein R¹⁶ is C₁-C₆ alkyl;
R¹⁷ is C₁-C₆ alkanoyl or aroyl; and
R¹⁸ is methyl, C₁-C₆ alkanoyloxymethyl or aroyloxymethyl]], then converting 2-tri(C₁-C₄ alkyl/phenyl) silylethyloxy group at the reducing terminal to a suitable leaving group to give a compound represented by the general formula: 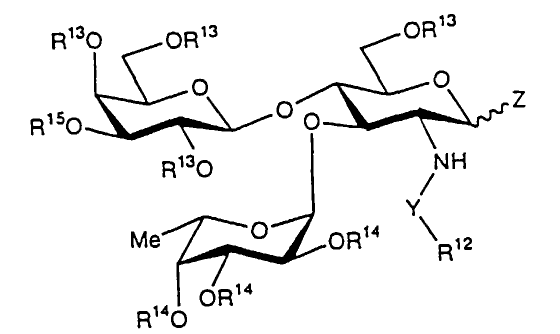 [wherein R¹³, R¹⁵, Y, R¹⁴ and R¹² are as defined above; and Z is a leaving group], then glycosylating the resultant compound with a compound represented by the general formula:
R¹⁹OH
[wherein R¹⁹ is unsubstituted C₁-C₁₈ alkyl, aryl or aryl C₁-C₁₂ alkyl, or substituted C₁-C₁₈ alkyl, aryl or aryl C₁-C₁₂ alkyl] to give a compound represented by the general formula: 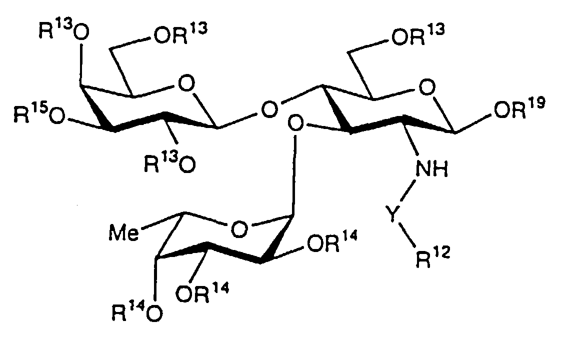 [wherein R¹³, R¹⁵, Y, R¹⁴, R¹² and R¹⁹ are as defined above], and finally hydrolyzing the resultant compound to give the Lewis X derivative represented by the general formula: 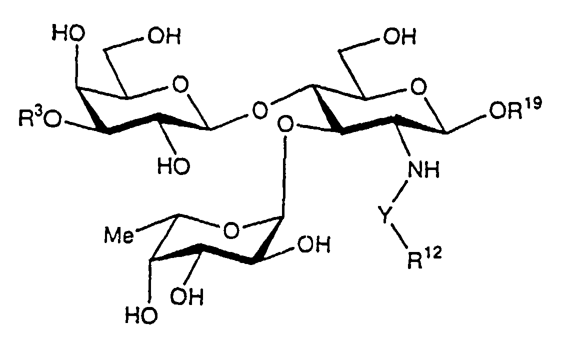 [wherein Y, R¹² and R¹⁹ are as defined above; and
R³ is hydrogen or a group represented by the general formula: 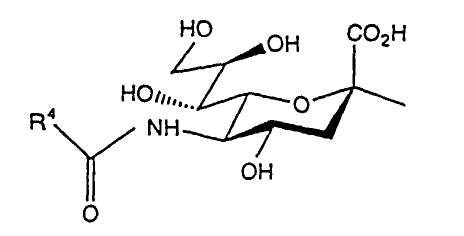 [wherein R⁴ is methyl or hydroxymethyl]].

15. A process for preparing a Lewis X derivative as defined hereinafter, which process comprises appropriately protecting the N-atom of the compound as claimed in Claim 12 to give a compound represented by the general formula: 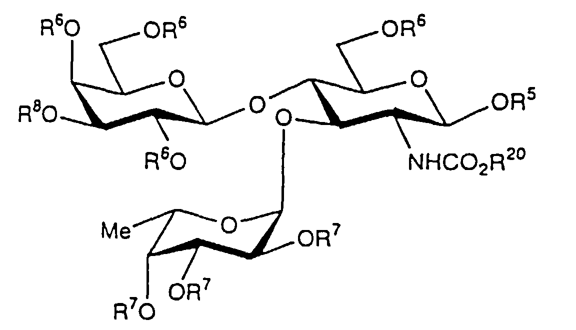 [wherein R⁵ is 2-tri(C₁-C₄ alkyl/phenyl)silylethyl;
R⁶ and R⁷ are each hydrogen, C₁-C₆ alkanoyl or aroyl;
R⁸ is hydrogen, C₁-C₆ alkanoyl, aroyl or a group represented by the general formula: 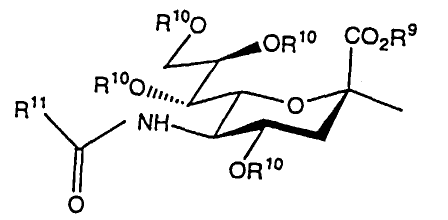 [wherein R⁹ is hydrogen or C₁-C₆ alkyl;
R¹⁰ is hydrogen, C₁-C₆ alkanoyl or aroyl; and
R¹¹ is methyl, hydroxymethyl, C₁-C₆ alkanoyloxymethyl or aroyloxymethyl] and
R²⁰ is allyl, t-butyl or benzyl], then subjecting the resultant compound to O-acylation (together with esterification, when R⁹ is hydrogen) (if none of R⁶, R⁷ and R⁸ is hydrogen and R⁸ has no unprotected hydroxy, this O-acylation is not necessary) to give a compound represented by the general formula: 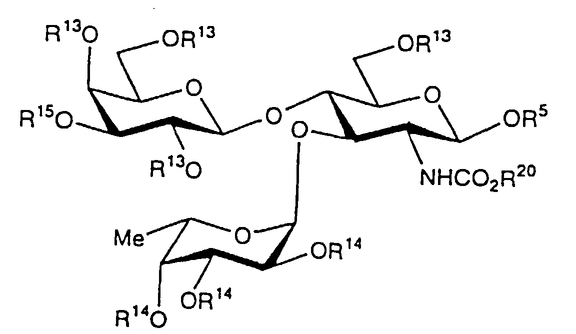 [wherein R⁵ and R²⁰ are as defined above;
R¹³ and R¹⁴ are each C₁-C₆ alkanoyl or aroyl; and
R¹⁵ is C₁-C₆ alkanoyl, aroyl or a group represented by the general formula: 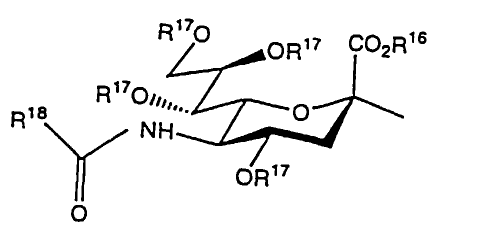 [wherein R¹⁶ is C₁-C₆ alkyl;
R¹⁷ is C₁-C₆ alkanoyl or aroyl; and
R¹⁸ is methyl, C₁-C₆ alkanoyloxymethyl or aroyloxymethyl]], then converting 2-tri(C₁-C₄ alkyl/phenyl) silylethyloxy group on the reducing terminal to an appropriate leaving group to give a compound represented by the general formula: 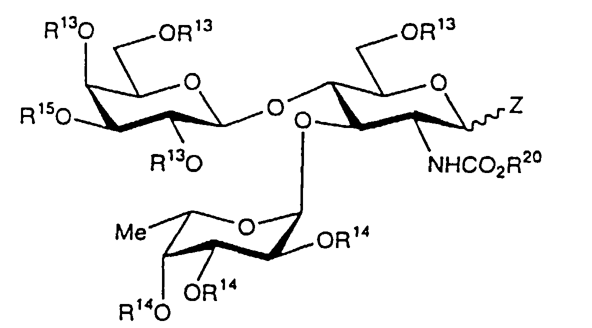 [wherein R¹³, R¹⁵, R¹⁴ and R²⁰ are as defined above; and
Z is a leaving group], then glycosylating the resultant compound with a compound represented by the general formula:
R¹⁹OH
[wherein R¹⁹ is unsubstituted C₁-C₁₈ alkyl, aryl or aryl C₁-C₁₂ alkyl, or substituted C₁-C₁₈ alkyl, aryl or aryl C₁-C₁₂ alkyl] to give a compound represented by the general formula: 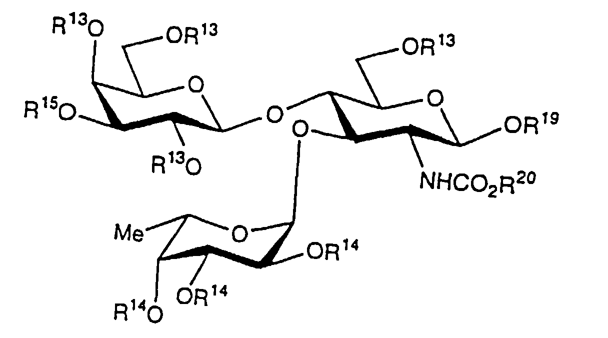 [wherein R¹³, R¹⁵, R¹⁴, R¹⁹ and R²⁰ are as defined above], then removing the protecting group on N-atom to give a compound represented by the general formula: 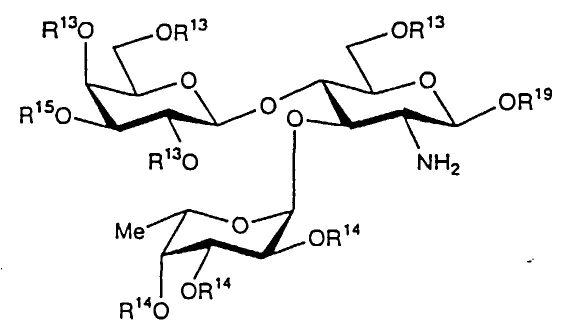 [wherein R¹³, R¹⁵, R¹⁴ and R¹⁹ are as defined above], then appropriately modifying the amino group of the product to give a compound represented by the general formula: 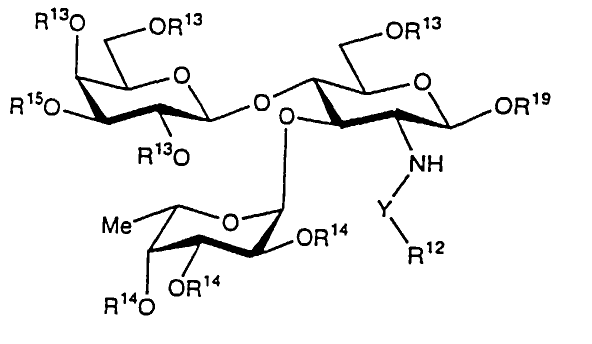 [wherein R¹³, R¹⁵, R¹⁴ and R¹⁹ are as defined above;
Y is C(O), SO₂, C(O)NH, C(O)O or C(O)S; and
R¹² is C₁-C₆ alkyl, aryl, substituted aryl, or aryl C₁-C₆ alkyl], and finally hydrolyzing the resultant compound to give the Lewis X derivative represented by the general formula: 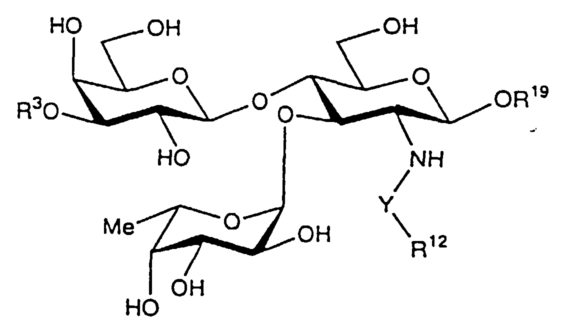 [wherein Y, R¹² and R¹⁹ are as defined above; and
R³ is hydrogen or a group represented by the general formula: 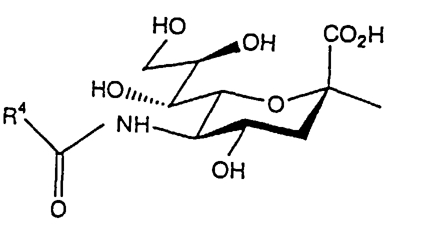 [wherein R⁴ is methyl or hydroxymethyl]].

16. A process for preparing the compound as claimed in Claim 12 which comprises protecting the N-atom of the compound represented by the general formula: 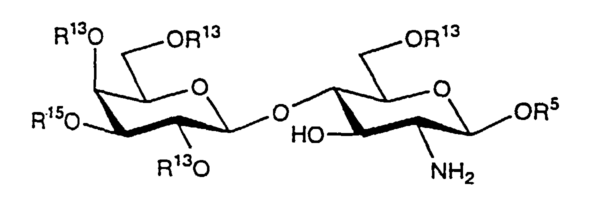 [wherein R⁵ is 2-tri(C₁-C₄ alkyl/phenyl)silylethyl;
R¹³ is C₁-C₆ alkanoyl or aroyl; and
R¹⁵ is C₁-C₆ alkanoyl, aroyl or a group represented by the general formula: 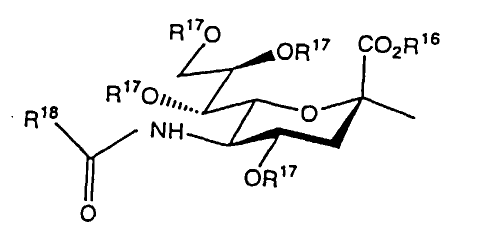 [wherein R¹⁶ is C₁-C₆ alkyl;
R¹⁷ is C₁-C₆ alkanoyl or aroyl; and
R¹⁸ is methyl, C₁-C₆ alkanoyloxymethyl or aroyloxymethyl]], to give a compound represented by the general formula: 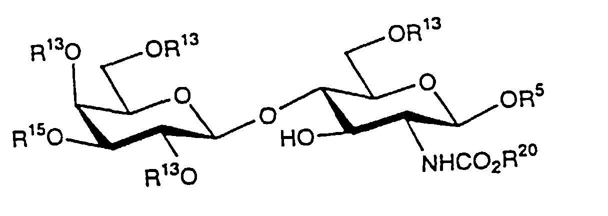 [wherein R⁵, R¹³ and R¹⁵ are as defined above; and
R²⁰ is allyl, t-butyl or benzyl], then subjecting the resultant compound to glycosylation with an L-fucopyranosyl derivative represented by the general formula: 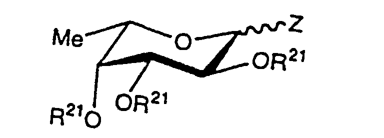 [wherein R²¹ is C₁-C₆ alkanoyl, aroyl, benzyl, or substituted benzyl; and Z is a leaving group] to give a compound represented by the general formula: 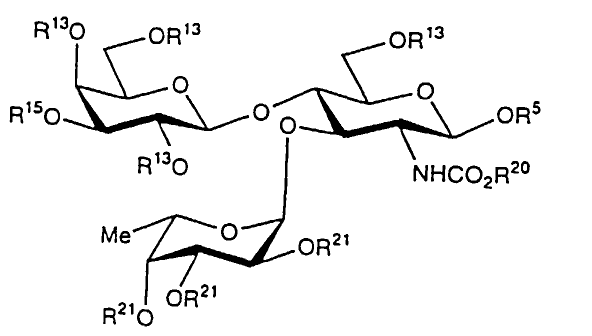 [wherein R⁵, R¹³, R²⁰, R¹⁵ and R²¹ are as defined above], then subjecting the resultant compound to deprotection of a protective group, if necessary (if R²¹ is benzyl or substituted benzyl, this deprotection is indispensable), to give a compound represented by the general formula: 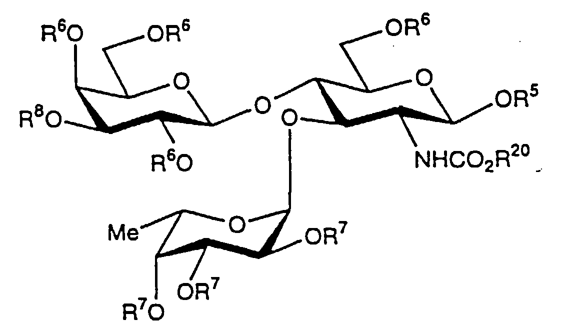 [wherein R⁵ and R²⁰ are as defined above,
R⁶ and R⁷ are each hydrogen, C₁-C₆ alkanoyl or aroyl; and
R⁸ is hydrogen, C₁-C₆ alkanoyl, aroyl or a group represented by the general formula: 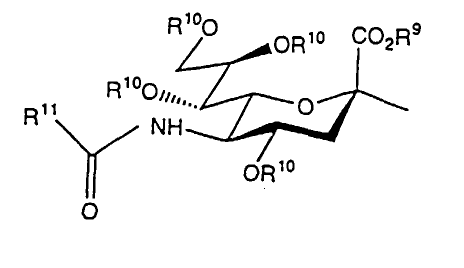 [wherein R⁹ is hydrogen or C₁-C₆ alkyl;
R¹⁰ is hydrogen, C₁-C₆ alkanoyl or aroyl; and
R¹¹ is methyl, hydroxymethyl, C₁-C₆ alkanoyloxymethyl or aroyloxymethyl]], and finally subjecting the resultant compound to N-deprotection.

17. A process for preparing a compound as claimed in Claim 12 which comprises reacting a compound represented by the general formula: 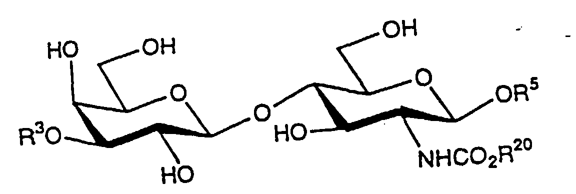 [wherein R⁵ is 2-tri(C₁-C₄ alkyl/phenyl)silylethyl;
R²⁰ is allyl, t-butyl or benzyl;
R³ is hydrogen or a group represented by the general formula: 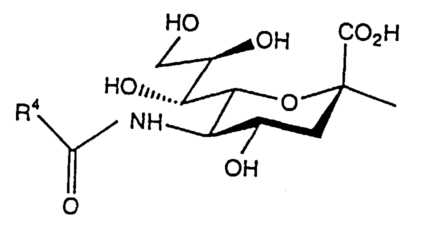 [wherein R⁴ is methyl or hydroxymethyl]] with GDP-fucose using fucosyl transferase to give a compound represented by the general formula: 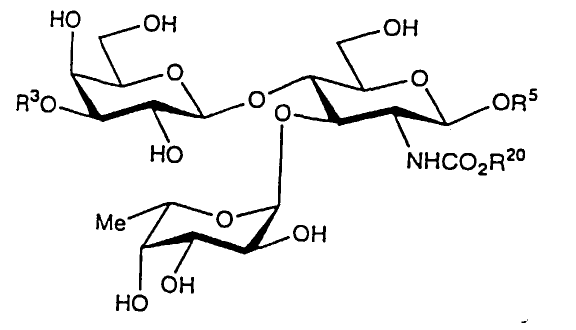 [wherein R⁵, R³, and R²⁰ are as defined above], then subjecting the resultant compound, if necessary, to O-acylation and carboxy-esterification, and finally subjecting the resultant compound to N-deprotection.

18. A process for preparing a compound as claimed in Claim 13 which comprises reacting a glucosamine derivative represented by the general formula: 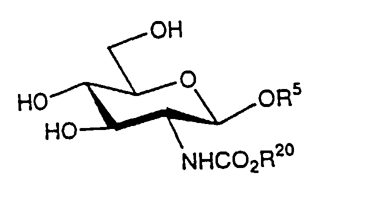 [wherein R⁵ is 2-tri(C₁-C₄ alkyl/phenyl)silylethyl; and
R²⁰ is allyl, t-butyl or benzyl] with UDP-galactose using galactosyl transferase and then, if necessary with CMP-N-acetylneuraminic acid using sialyl transferase, to give a compound represented by the general formula: 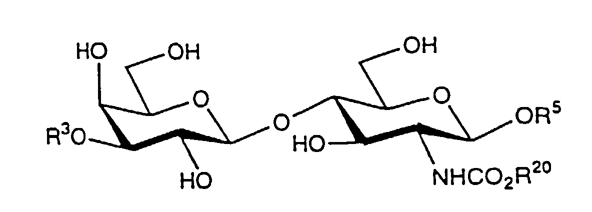 [wherein R⁵ and R²⁰ are as defined above; and
R³ is hydrogen or a group represented by the general formula: 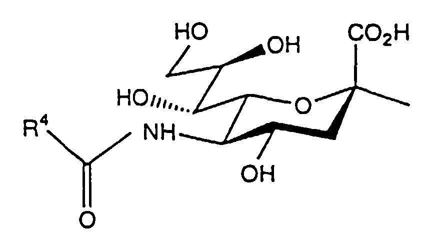 [wherein R⁴ is methyl or hydroxymethyl]],
then subjecting the resultant compound, if necessary, to O-acylation (together with esterification, if necessary), to give a compound represented by the general formula: 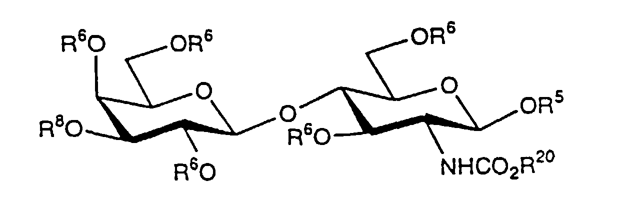 [wherein R⁵ and R²⁰ are as defined above;
R⁶ is hydrogen, C₁-C₆ alkanoyl or aroyl; and
R⁸ is hydrogen, C₁-C₆ alkanoyl, aroyl or a group represented by the general formula: 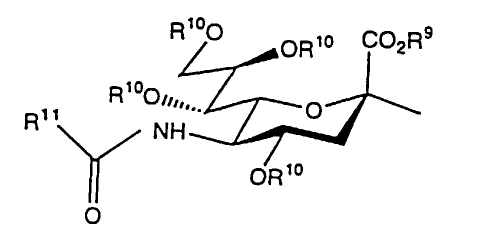 [wherein R⁹ is hydrogen or C₁-C₆ alkyl;
R¹⁰ is hydrogen, C₁-C₆ alkanoyl or aroyl; and
R¹¹ is hydrogen, methyl, C₁-C₆ alkanoyloxymethyl or aroyloxymethyl]],
then subjecting the resultant compound to N-deprotection to give a compound represented by the general formula: 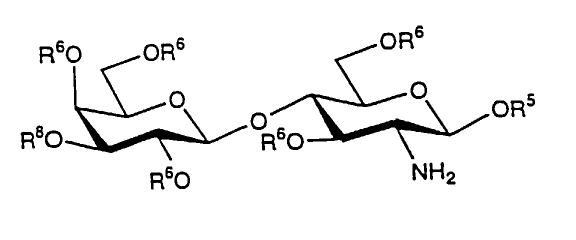 [wherein R⁵, R⁶ and R⁸ are as defined above], and finally subjecting the resultant compound to a regioselective deacylation reaction.
